(19)

**Europäisches Patentamt**

**European Patent Office**

**Office européen des brevets**

(11)   **EP 4 644 413 A1**

(12)   # EUROPEAN PATENT APPLICATION

(43) Date of publication:
    **05.11.2025 Bulletin 2025/45**

(21) Application number: **24174150.3**

(22) Date of filing: **03.05.2024**

(51) International Patent Classification (IPC):
    ***C07K 16/00*** *(2006.01)*    ***C07K 16/28*** *(2006.01)*

(52) Cooperative Patent Classification (CPC):
    **C07K 16/2887; C07K 16/00; C07K 16/2893;**
    C07K 2317/41; C07K 2317/526; C07K 2317/71;
    C07K 2317/72; C07K 2317/732; C07K 2317/734;
    C07K 2317/92; C07K 2317/94

(84) Designated Contracting States:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Designated Extension States:
    **BA**
    Designated Validation States:
    **GE KH MA MD TN**

(71) Applicant: **Genmab B.V.**
    **3584 CT Utrecht (NL)**

(72) Inventors:
    • **LLOYD, Katy Ann**
     **NL-3584 CT Utrecht (NL)**
    • **XUE, Xiaoguang**
     **NL-3584 CT Utrecht (NL)**

    • **LECONET, Wilhem**
     **NL-3584 CT Utrecht (NL)**
    • **DE JONG, Rob**
     **NL-3584 CT Utrecht (NL)**
    • **LABRIJN, Aran Frank**
     **NL-3584 CT Utrecht (NL)**

(74) Representative: **Genmab A/S**
    **Carl Jacobsens Vej 30**
    **2500 Valby (DK)**

Remarks:
    The complete document including Reference
    Table(s) and the Sequence Listing(s) can be
    downloaded from the EPO website

(54)   **BINDING AGENTS HAVING ALTERED FC-MEDIATED EFFECTOR FUNCTIONS**

(57)    The present invention relates to CH3 domain-containing binding agents, such as antibodies, that have altered Fc-mediated effector functions resulting from one or more amino acid modifications in the Fc domain. The invention further relates to polynucleotides encoding such binding agents, cells capable of producing such binding agents and the use of the binding agents of the invention for the treatment of diseases.

## Description

### Field of the invention

The present invention relates to CH3 domain-containing binding agents, such as antibodies, that have altered Fc-mediated effector functions resulting from one or more amino acid modifications in the Fc domain. The invention further relates to polynucleotides encoding such binding agents, cells capable of producing such binding agents and the use of the binding agents of the invention for the treatment of diseases. Moreover, the invention relates to methods for altering one or more effector functions of a binding agent, wherein the methods comprise the introduction of particular amino acids at particular positions in the binding agent.

### Background of the invention

Antibodies conventionally consist of four polypeptide chains, two light (L) chains and two heavy (H) chains, that form a complex through non-covalent and covalent interactions. Each L chain associates with one H chain through non-covalent interactions between the variable (V) domains (VL and VH, respectively) and constant (C) domains (CL and CH1, respectively), and are stabilized by a covalent inter-chain disulfide bridge between the C domains. The two H chains associate with each other through non-covalent interactions between the two CH3 domains and are stabilized by covalent inter-chain disulfide bridges in the hinge region of the molecule. The CH3 domains are held together mainly through hydrophobic interactions at the center of the interface and symmetric electrostatic interactions surrounding the hydrophobic core (Ha et al, Front. Immunol. (2016), 7: 394).

Antibodies are highly effective molecules which can have effects on foreign entities or target cells via various mechanisms. Binding of an antibody to a target antigen on a cell surface can drive a direct antagonistic or agonistic effect on the target cell. Antibodies can also exert effects on target cells through Fc-mediated effector functions such as complement-dependent cytotoxicity (CDC), complement-dependent cellular phagocytosis (CDCP), antibody-dependent cell-mediated cytotoxicity (ADCC) and antibody-dependent cell-mediated phagocytosis (ADCP). Such Fc-mediated effector functions may contribute to the therapeutic efficacy of antibody treatments.

CDC and CDCP are initiated by binding of C1q to the Fc regions of antibodies bound to surface antigen on a target cell. C1q is a multimeric protein consisting of six globular binding heads attached to a stalk. The individual globular binding heads have low affinity for IgG; and C1q must gain avidity by binding multiple IgG molecules on a cell surface to trigger the classical complement pathway. IgG hexamerization upon target binding on the cell surface has been shown to support avid C1q binding (Diebolder (2014) Science 343:1260). The hexamerization is mediated through intermolecular non-covalent Fc-Fc interactions, and Fc-Fc interactions can be enhanced by point mutations in the CH3 domain, including E345R and E430G. WO2013/004842 discloses antibodies or polypeptides comprising variant Fc regions having one or more amino acid modifications resulting in modified effector functions such as CDC. WO2014/108198 discloses polypeptides such as antibodies comprising variant Fc regions having one or more amino acid modifications resulting in increased CDC. WO2016/164480 discloses antigen binding complexes having agonistic activity.

ADCC and ADCP are initiated by binding of the Fc region to Fc receptor (FcR)-bearing cells. In humans, FcgRI, FcgRIIa (H/R) and FcgRIIIa (F/V) are activatory receptors and FcgRIIb is an inhibitory receptor. Previous efforts have been made to identify antibody Fc-variants with an enhanced effector function or other modified properties. Such studies have focused on, e.g., exchanging segments between IgG isotypes to generate chimeric IgG molecules (Natsume et al., 2008 Cancer Res 68(10), 3863-72), or amino acid substitutions in the hinge region (Dall'Acqua et al., 2006 J Immunol 177, 1129-1138), or in or near the C1q-binding site in the CH2 domain, centered around residues D270, K322, P329, and P331 (Idusogie et al., 2001 J Immunol 166, 2571-2575; Michaelsen et al., 2009 Scand J Immunol 70, 553-564 and WO 99/51642). For example, Moore et al. (2010 mAbs 2(2), 181-189) describes testing various combinations of S267E, H268F, S324T, S239D, I332E, G236A and I332E for enhanced effector functions via CDC or ADCC. Other Fc mutations affecting binding to Fc- receptors (WO 2006/105062, WO 2000/42072, U.S. Patent 6,737,056 and U.S. Patent 7,083,784) or physical properties of the antibodies (WO 2007/005612 A1) have also been suggested.

Most efforts for enhancing Fc-mediated effector functions have concentrated on altering glycosylation patterns, introducing mutations in the CH2 regions known to interact with Fc gamma receptors (FcgRs) and complement components, or through amino acid modifications to improve Fc-Fc interactions. By concentrating on a narrow set of modifications, existing solutions may overlook alternative features of the Fc region that could be leveraged for therapeutic benefit. The CH3 domain, for instance, has been less explored in the context of modulating antibody effector functions. There remains a need for novel antibodies combining different strategies, such as Fc-Fc interaction or enhanced C1q / FcgR/ FcRn binding, with novel approaches like CH3-CH3 interface modifications to modulate Fc-mediated effector functions to improve therapeutic efficacy.

**Summary of the invention**

It is an object of the present invention to provide for novel approaches for modulation of Fc region-mediated effector functions via modifications in the CH3-CH3 interface. Accordingly, it is an object of the invention to provide a binding agent, such as an antibody, comprising a mutated CH3 domain of a human IgG and a target-binding region, such as an antigen-binding region, which binding agent has increased capacity for FcgammaRIIa-H activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for FcgammaRIIb activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for FcgammaRIIIa-V activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for FcgammaRIIa-H activation, but wild-type-like capacity for CDC activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for FcgammaRIIb activation, but wild-type-like capacity for CDC activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for FcgammaRIIIa-V activation, but wild-type-like capacity for CDC activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for CDC activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for FcgammaRIIa-H activation and an increased capacity for CDC activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for FcgammaRIIb activation and an increased capacity for CDC activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for FcgammaRIIIa-V activation and an increased capacity for CDC activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for CDC activation and a wild-type-like or reduced capacity for FcgammaRIIa-H activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for CDC activation and a wild-type-like or reduced capacity for FcgammaRIIb activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased capacity for CDC activation and a wild-type-like or reduced capacity for FcgammaRIIIa-V activation, when compared to a parent binding agent which is identical but without the CH3 mutation(s).

It is another object of the present invention to provide for a binding agent, such as an antibody, comprising a mutated CH3 domain of a human IgG and a target-binding region, such as an antigen-binding region, which binding agent has increased FcRn binding, when compared to a parent binding agent which is identical but without the CH3 mutation(s).

Additionally, the binding agents containing mutations CH3 domains display similar stability profiles and no increased immunogenicity risk compared to their counterparts without mutations. Furthermore, only limited effects on glycosylation or the ability to bind to target, were observed for antibodies comprising these CH3 domains. When injected in mice, antibodies comprising these CH3 domains generally displayed similar clearance compared to their counterparts without the mutations. The mutated CH3 domains are compatible with additional Fc engineering strategies that address other functional properties (e.g. increased half-life, etc).

Thus, in a first aspect, the present invention provides a binding agent comprising at least CH3 domain of a human IgG and a target-binding region, wherein said CH3 domain comprises, one or more mutations in the CH3 domain, such as at least one of the following amino acid residues:

a) R347 or K347;
b) V350;
c) K351, Y351, D351 or E351;
d) K356;
e) Q357 or K357;
f) E360 or D360;
g) K364 or W364;
h) W366, L366, K366 or V366;
i) D368;
j) S370, E370 or A370;
k) D392, L392 or E392;
l) W394;
m) K399, V399 or M399;
n) A405 or T405;
o) A407 or V407;
p) D409, W409 or V409; or
q) E439,

wherein the numbering is according to the EU index.

In further aspects, the invention relates to polynucleotide constructs and host cells that can be used for producing a binding agent of the invention and to pharmaceutical compositions comprising the binding agent, the polynucleotide or the host cell according to the invention and a pharmaceutically-acceptable carrier and/or excipient.

Furthermore, the invention relates to the binding agent, polynucleotide(s) or host cell according to the present invention for use as a medicament and to a method for the treatment of a disease or disorder, comprising administration, to a subject in need thereof, of a binding agent, polynucleotide(s) or host cell according to the invention.

In a further aspect, the invention relates to a method for altering one or more effector functions of a parent binding agent comprising a CH3 domain of a human IgG and a target-binding region, or for producing a binding agent having one or more altered effector function, which method comprises introducing one or more of the following amino acids into the parent binding agent:

a) R347 or K347;
b) V350;
c) K351, Y351, D351 or E351;
d) K356;
e) Q357 or K357;
f) E360 or D360;
g) K364 or W364;
h) W366, L366, K366 or V366;
i) D368;
j) S370, E370 or A370;
k) D392, L392 or E392;
l) W394;
m) K399, V399 or M399;
n) A405 or T405;
o) A407 or V407;
p) D409, W409 or V409; or
q) E439,

wherein the numbering is according to the EU index and wherein the binding agent has an altered effector function relative to a comparator binding agent which is identical except that it has the wild-type amino acid residue(s) at the specified position(s).

**Figure legends**

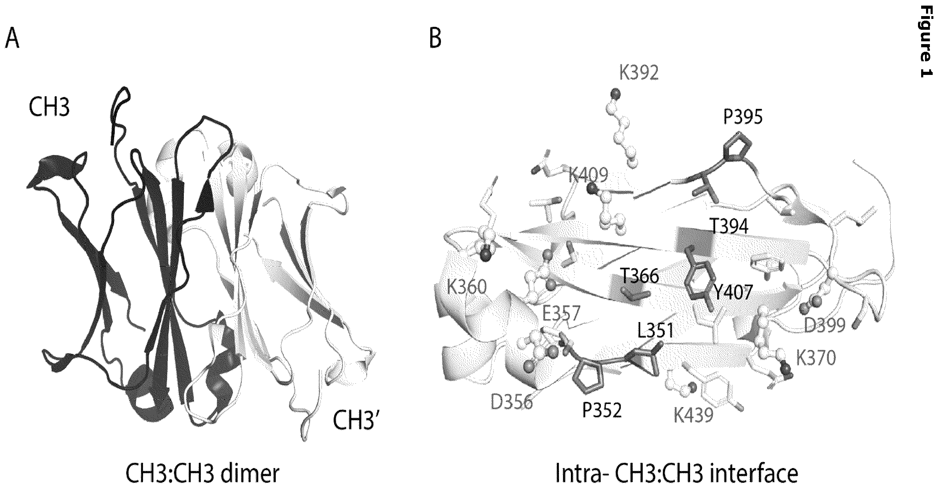

**Figure 1 -** Representation of the CH3:CH3 dimer interface of human IgG1. (A) IgG1 CH3:CH3 dimer. (B) Intra-CH3 interface.

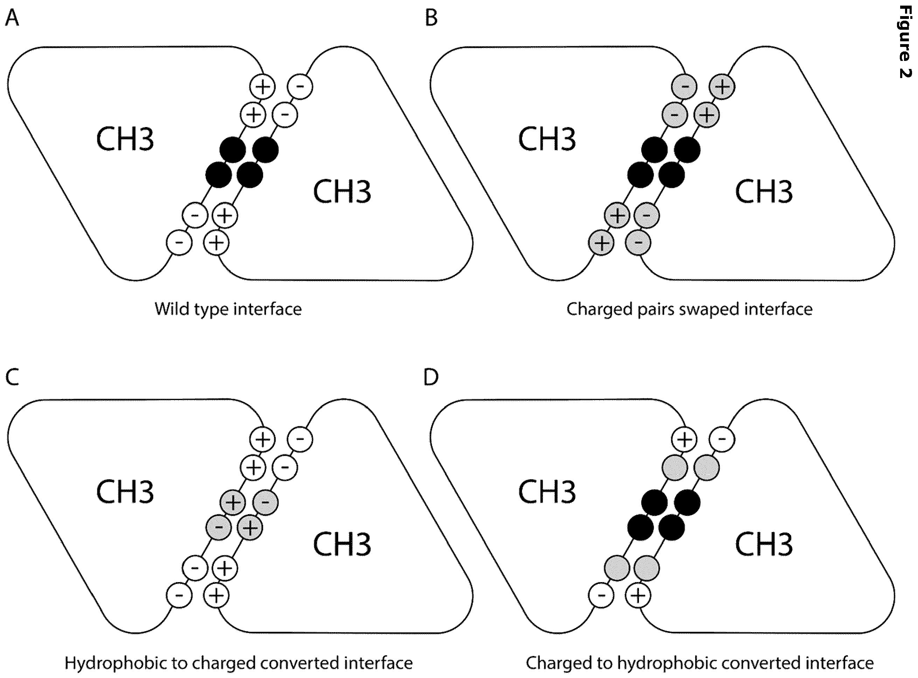

**Figure 2** - Schematic representation of the CH3 interface design strategy. (A) WT interface. (B) Charged pairs swapped interface. (C) Hydrophobic patches swapped to charged pairs. (D) Charged pairs swapped to hydrophobic patches converted interface.

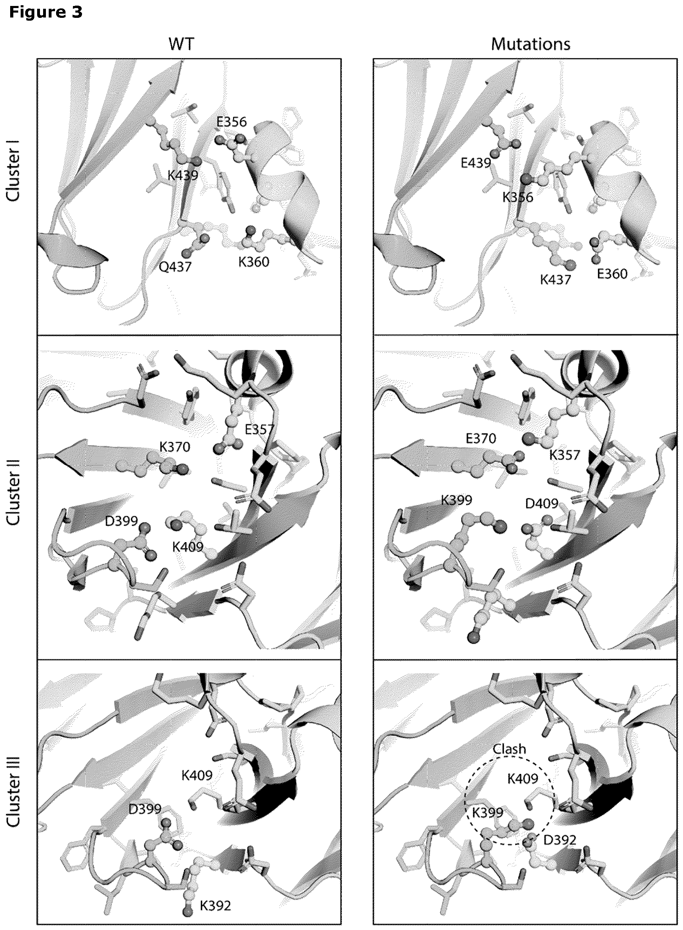

**Figure 3** - Design strategy: charged pairs swap. Left column: WT CH3:CH3 interface with highlighted residues involved in clusters I (top), II (middle) and III (bottom). Right column: introduced mutations in IgG1 CH3:CH3 interface residues involved in clusters I (top), II (middle) and III (bottom).

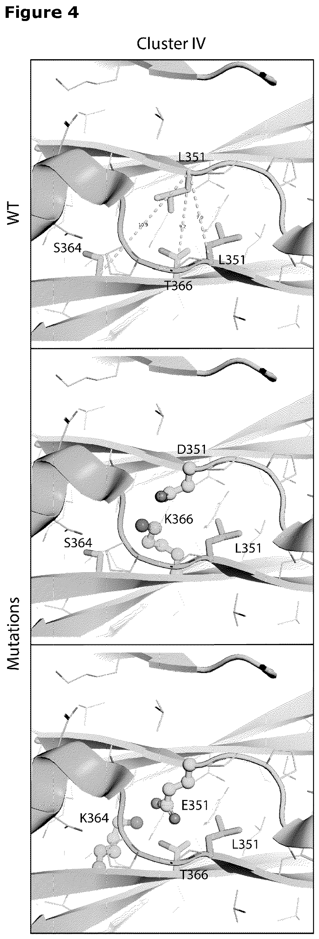

**Figure 4** - Design strategy: hydrophobic patches converted to charged pairs. Cluster IV interface wild-type residues (top) and mutations (middle and bottom).

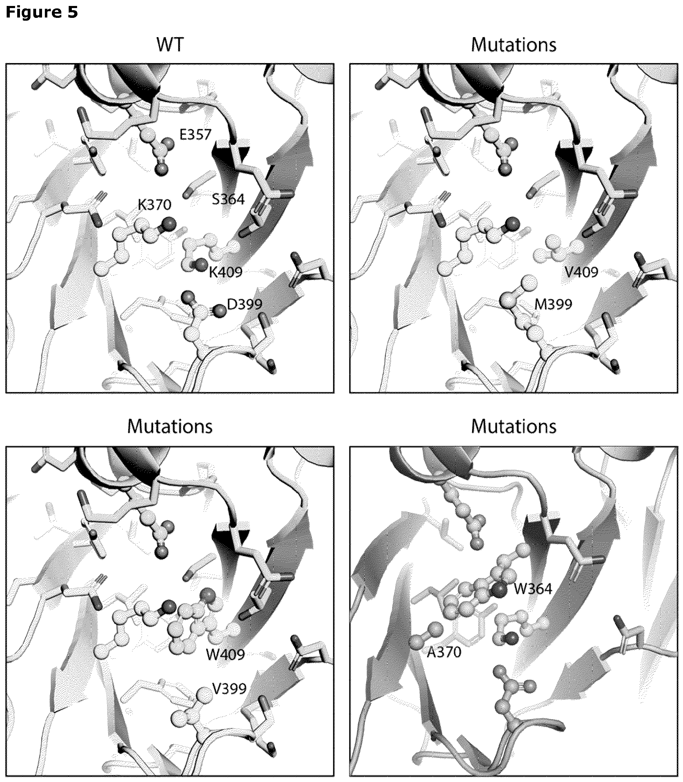

**Figure 5** - Design strategy: conversion of charged pairs to hydrophobic patches. Left top figure: wild-type IgG1 CH3:CH3 interface residues. Right top and bottom figures: mutations introduced in IgG1 CH3:CH3 interface.

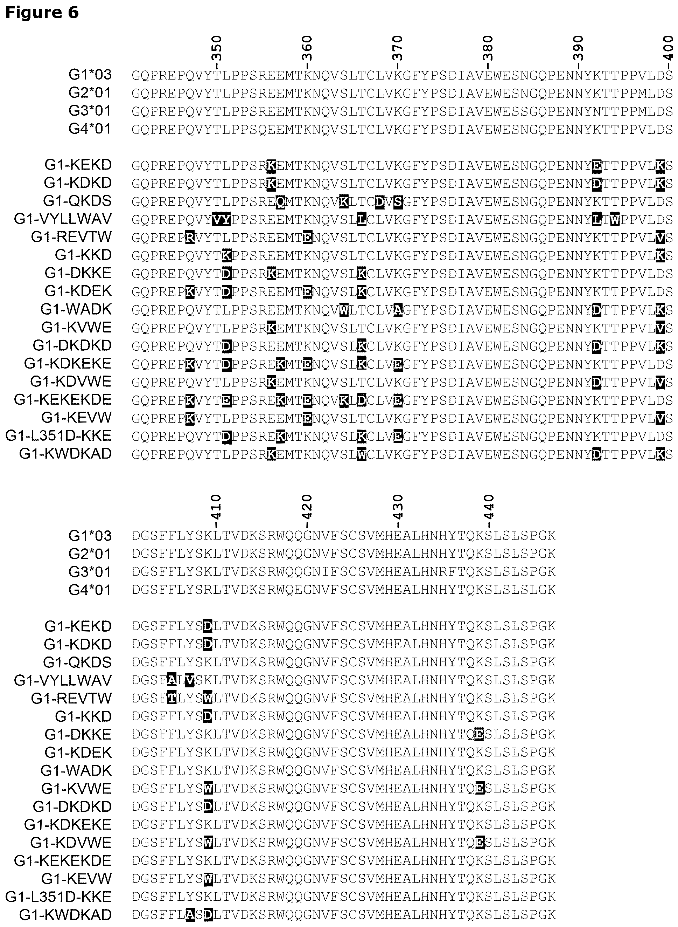

**Figure 6** - Alignment of the CH3 domains of IgG1 containing CH3 interface mutations and human IgG subclass sequences. The highlighted residues indicate positions within the CH3:CH3 interface that were mutated. Amino acid numbering according to Eu numbering.

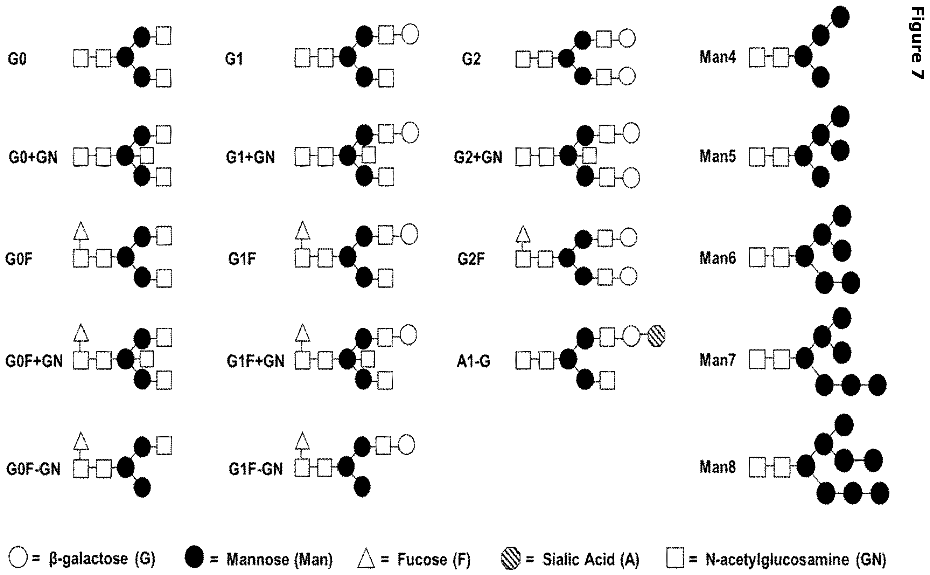

**Figure 7** - Schematic representations of glycan species detected on IgG antibody variants with or without CH3 interface mutations.

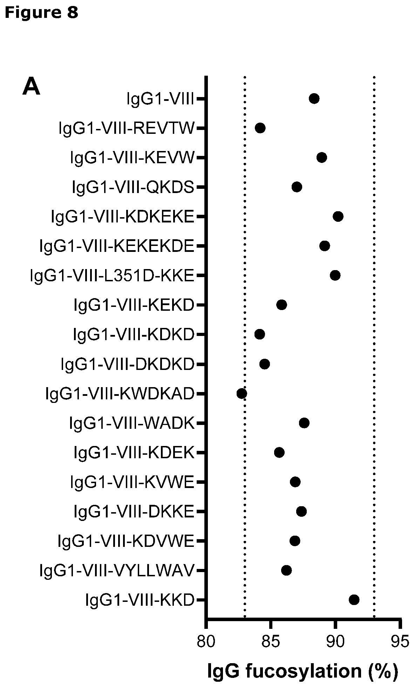

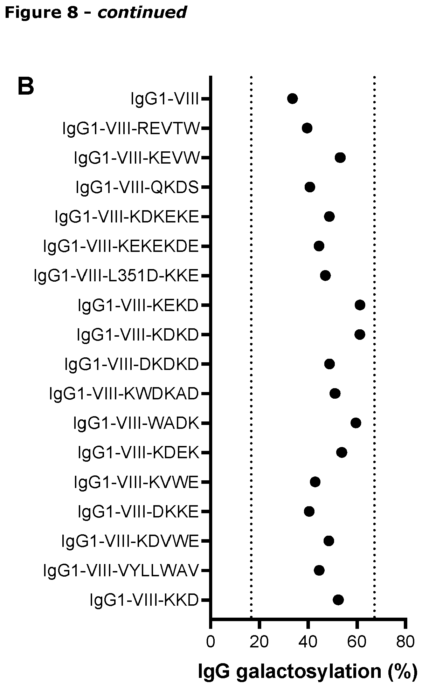

Figure 8 - (A) Relative abundance (%) of fucosylated N-glycans on the heavy chain of IgG1-VIII with or without CH3 interface mutations, as assessed by liquid chromatography-mass spectrometry (LC-MS). Dotted vertical lines represent differences of 5% lower or higher than the mean value of IgG1-VIII-WT. (B) Relative abundance (%) of galactosylated N-glycans on the heavy chain of IgG1-VIII with or without CH3 interface mutations, as assessed by liquid chromatography-mass spectrometry (LC-MS). Dotted vertical lines represent differences of 2-fold lower or higher than the mean value of IgG1-VIII-WT.

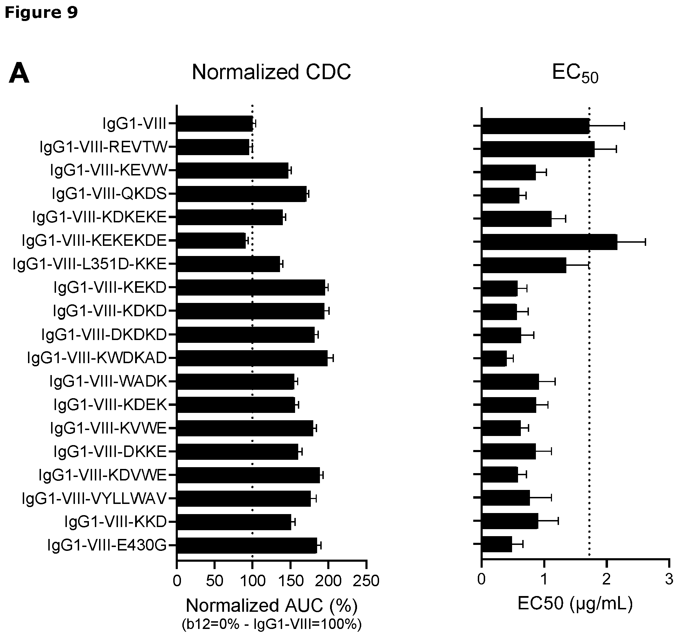

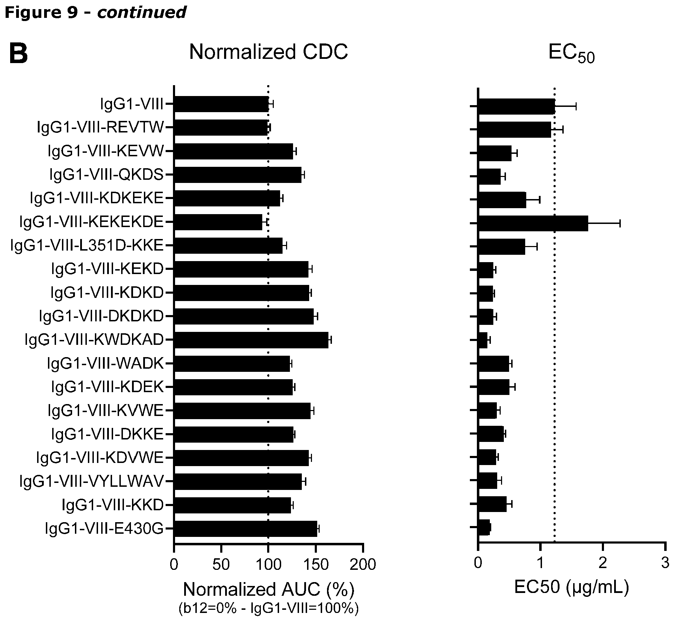

**Figure 9** - CDC activity in Raji cells (A) and Daudi cells (B) by antibody variants of IgG1-VIII with or without CH3 interface mutations. CDC activity is presented as the area under the dose-response curve (AUC; left panel), normalized to the AUC calculated for non-binding control antibody IgG1-b12 (set to 0%) and wild-type IgG1-VIII (set to 100%). Calculated EC50 values are also shown (right panel). Data shown are the mean ± SD of three independent experiments.

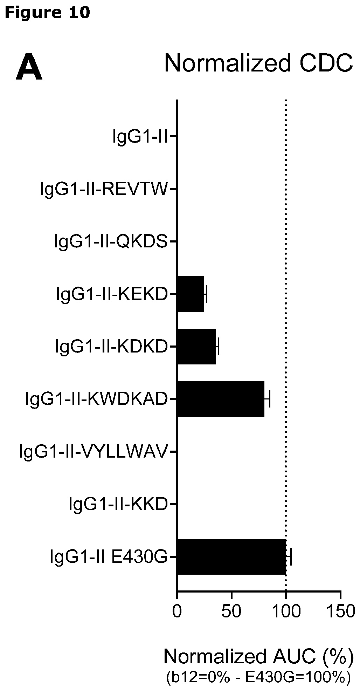

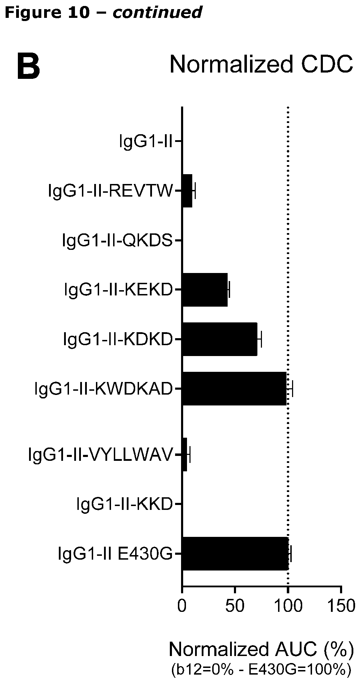

**Figure 10-** CDC activity in Raji cells (A) and Daudi cells (B) by antibody variants of IgG1-II with or without CH3 interface mutations. CDC activity is presented as the area under the dose-response curve (AUC), normalized to the AUC calculated for non-binding control antibody IgG1-b12 (set to 0%) and positive control IgG1-VIII-E430G (set to 100%). Data shown are the mean ± SD of three independent experiments.

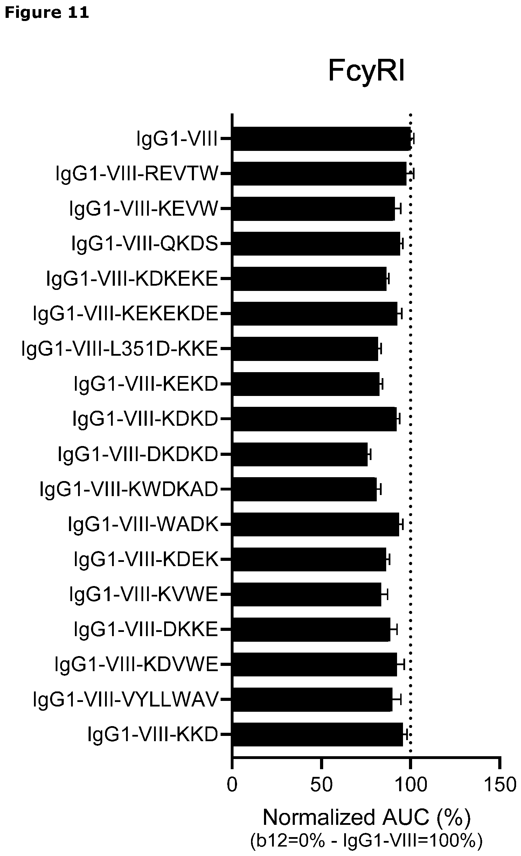

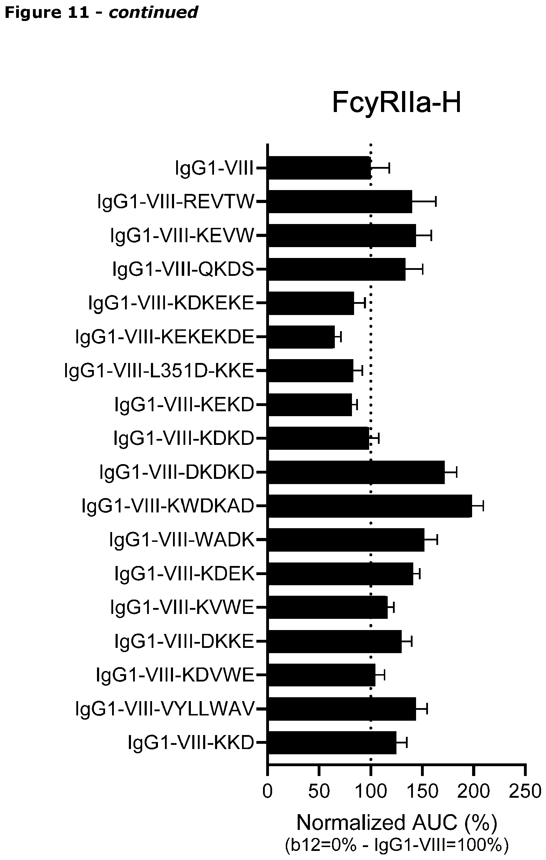

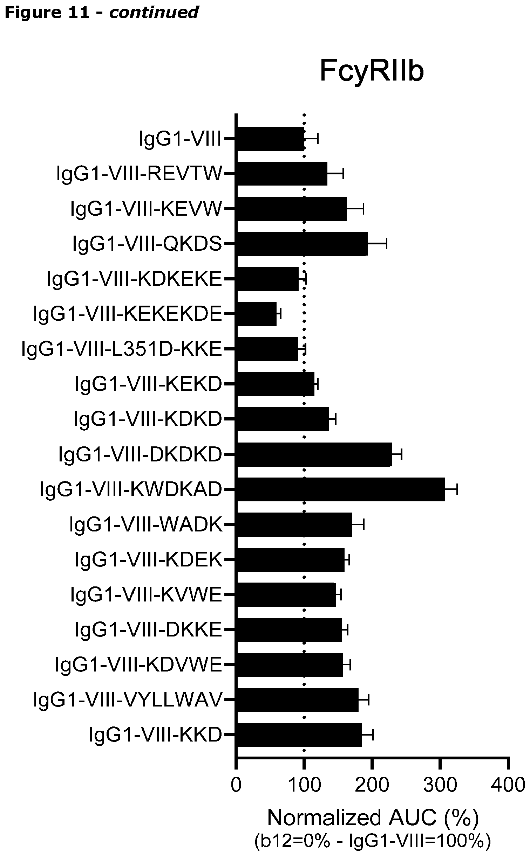

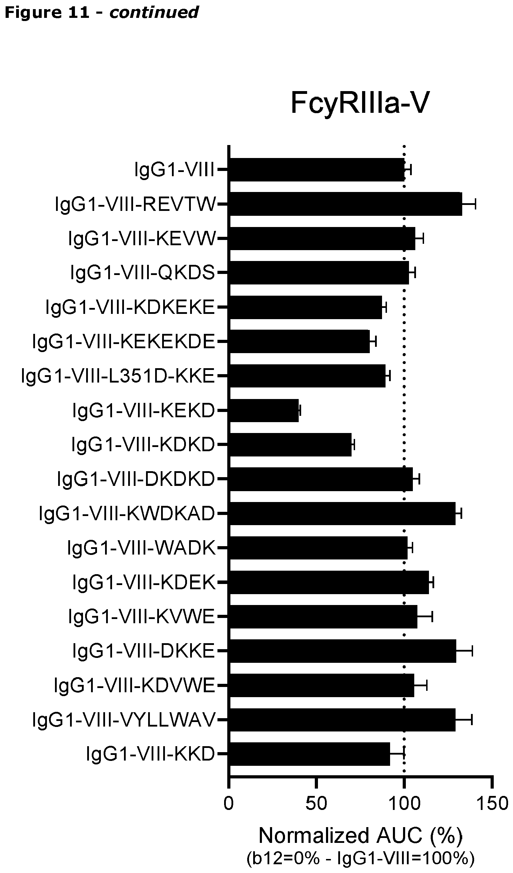

**Figure 11 -** Activation of FcγR by antibody variants of IgG1-VIII with or without CH3 interface mutations. FcγR activation is presented as the area under the dose-response curve (AUC), with the response being the luminescence measured as relative light units (RLU), normalized to the AUC calculated for non-binding control antibody IgG1-b12 (set to 0%) and wild-type IgG1-VIII (set to 100%). Data shown are the mean AUC ± SD of three independent experiments.

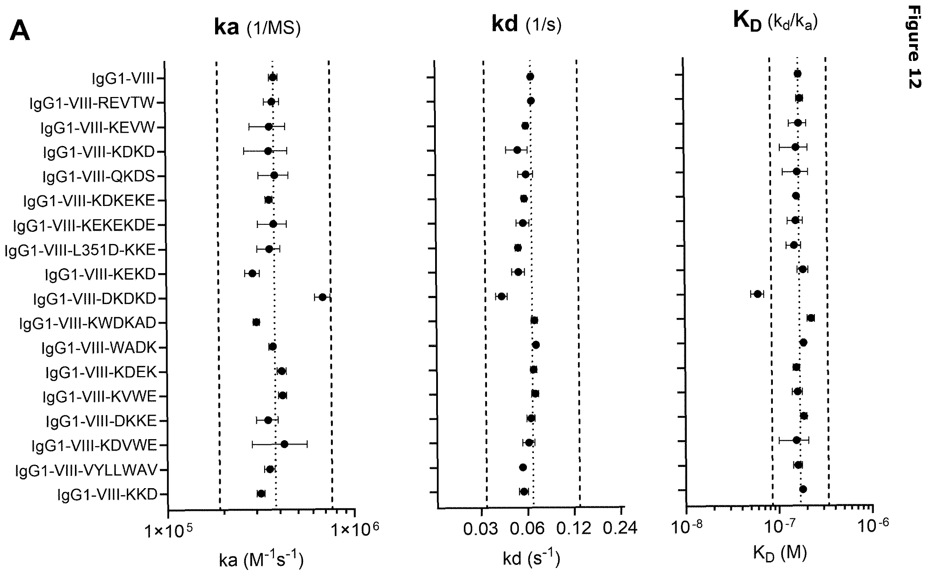

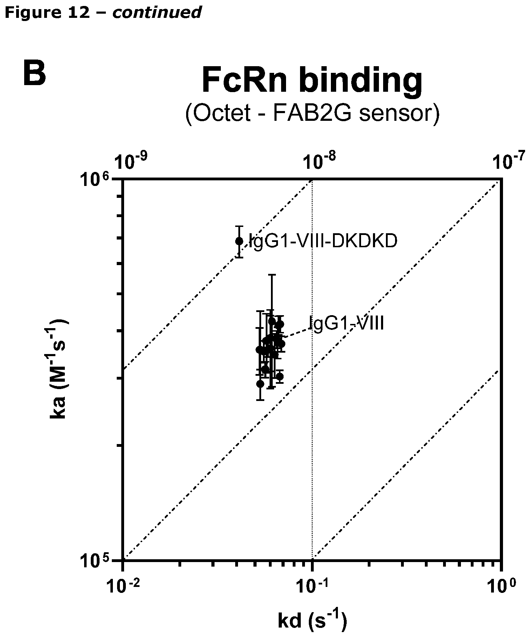

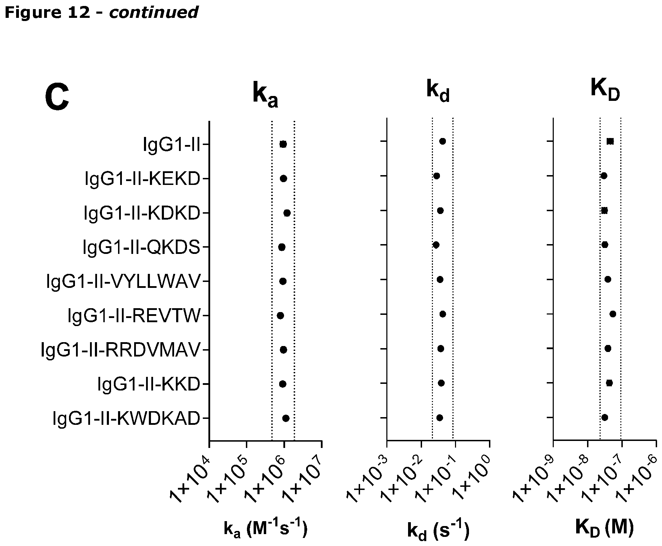

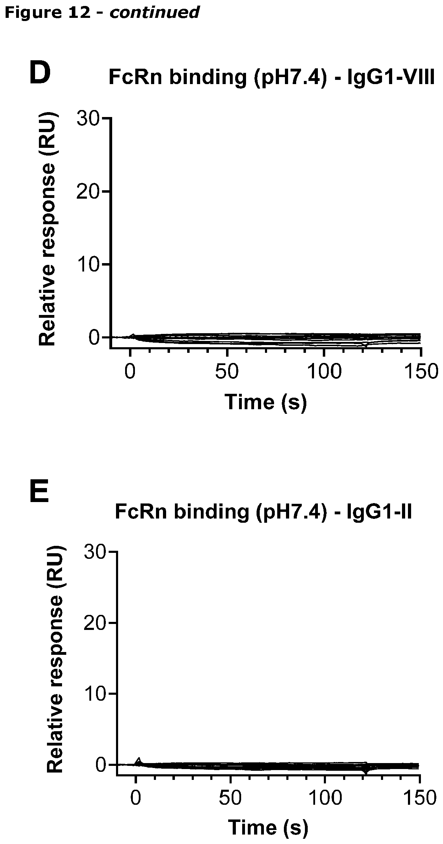

**Figure 12** - The $k_a$, $k_d$ and $K_D$ (from left to right) of the interaction between FcRn and antibody variants of IgG1-VIII with or without CH3 interface mutations (A, B), and between FcRn and antibody variants of IgG1-II with or without CH3 interface mutations (C). For each panel of CH3-interface mutation-containing antibodies, the **thick** dotted vertical lines represent $k_a$, $k_d$, or $K_D$ values that are 2-fold lower or higher than the mean values of the corresponding antibody without CH3 interface mutations, and the thin dotted lines represent IgG1-VIII wild-type binding. Data shown are the mean ± SD of three independent experiments. Binding to, along with dissociation from, FcRn at pH7.4 for all tested antibody variants of IgG1-VIII (D) or IgG1-III (E) with or without CH3 interface mutations. For each of the tested antibodies, the curve corresponding to the highest tested antibody concentration (i.e., 100 nM) is shown in the sensorgram. Data were corrected for background signal using reference sensors that were not incubated with antibody, and curves were fitted using Biacore Insight Evaluation software (Cytiva).

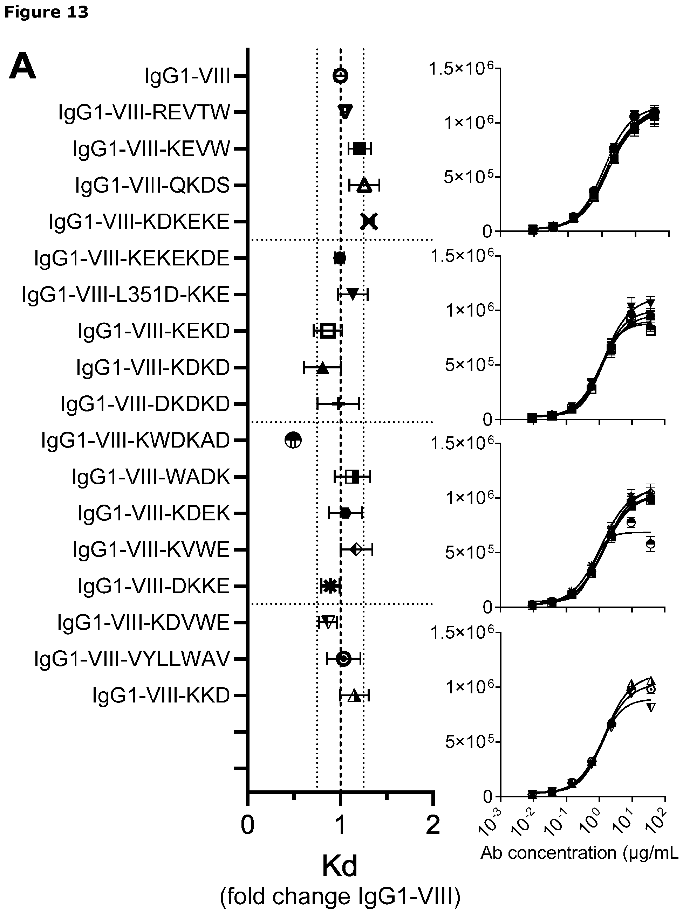

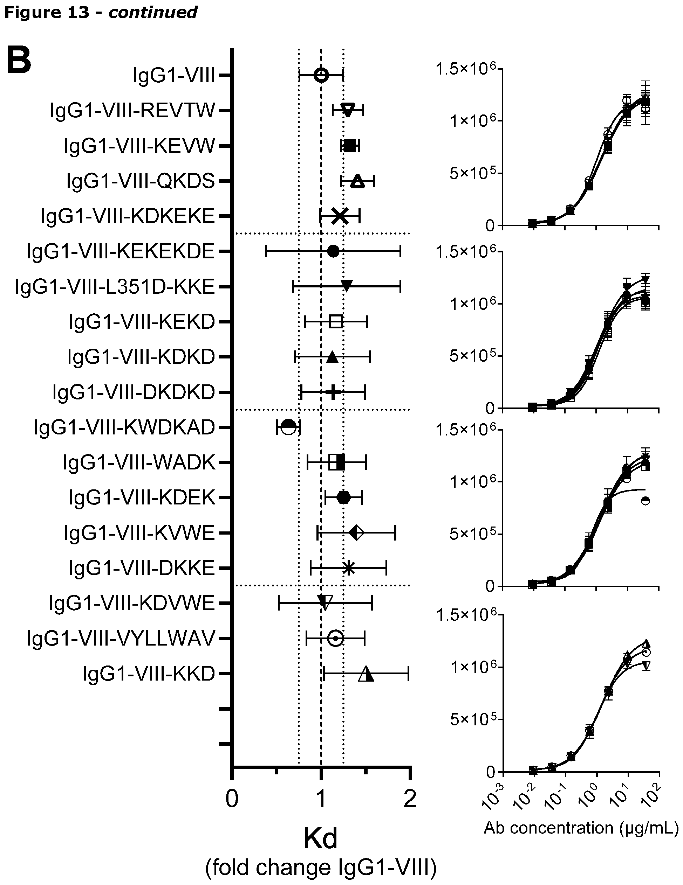

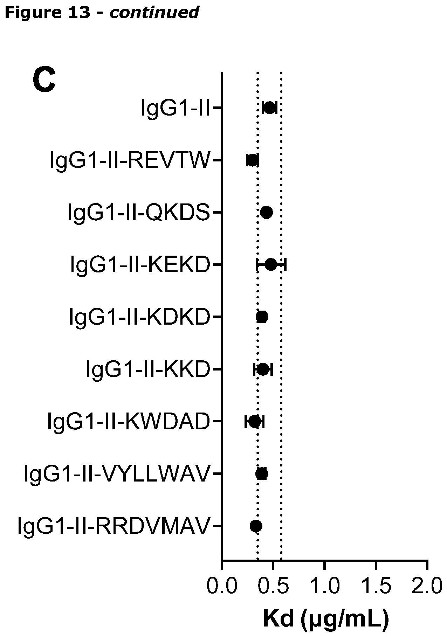

**Figure 13** - The Kd of the interaction between target-expressing Raji cells (A) and Daudi cells (B) and antibody variants of IgG1-VIII with or without CH3 interface mutations, and between target-expressing Raji cells (C) and antibody variants of IgG1-II with or without CH3 interface mutations. For each panel of CH3-interface mutation-containing antibodies, dotted vertical lines represent Kd values that are 25% lower or higher than the mean values of the corresponding antibody without CH3 interface mutations. Data shown are the mean Kd $\pm$ SD of two independent experiments.

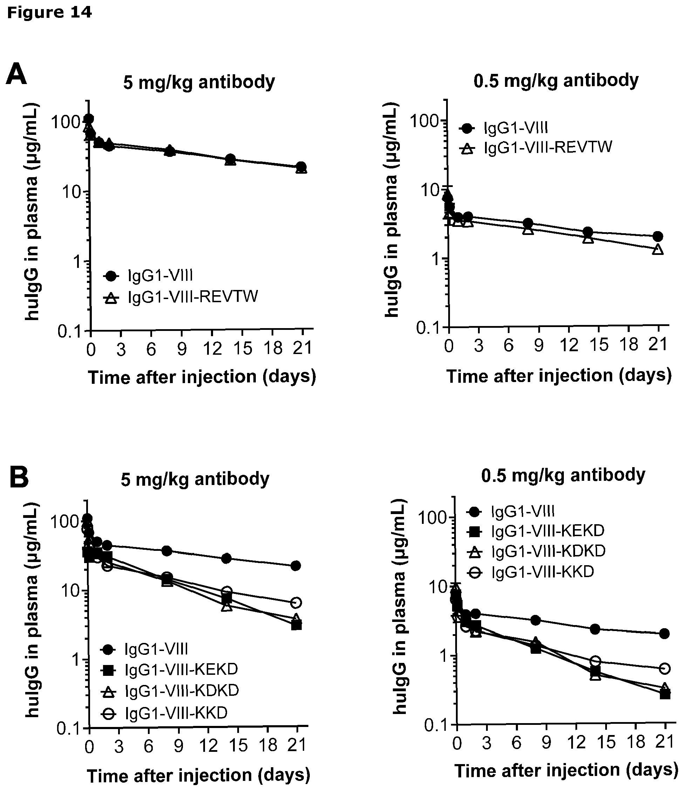

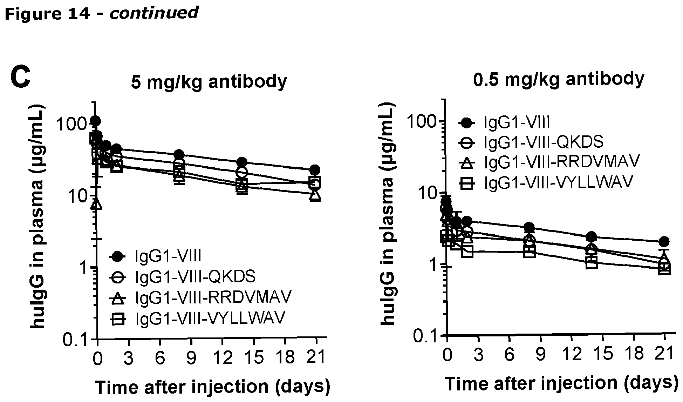

**Figure 14** - Total human IgG (huIgG) levels in mouse plasma measured at various timepoints after in vivo administration of approximately 5.0 mg antibody per kg body weight or 0.5 mg antibody per kg bodyweight (A-C). Shown are the mean $\pm$ SD of three mice per treatment group, and of four mice for the group that received 5 mg/kg IgG1-VIII-VYLLWAV. Mean plasma IgG levels at 10 min after injection of 5 mg/kg IgG1-VIII-WT, IgG1-VIII-KDKD, or IgG1-VIII-RRDVMAV, at 10 min after injection of 0.5 mg/kg IgG1-VIII-REVTW, at 1 day after injection of 5 mg/kg IgG1-VIII-KEKD, at 1 day, 2 days, and 16 days after injection of 5 mg/kg IgG1-VIII-KDKD, and at 21 days after injection of 0.5 mg/kg IgG1-VIII-KDKD were based on two results, either due to an insufficient plasma sample volume of one of the mice, or due to plasma IgG concentrations of one of the mice being below the lower limit of quantification of the ECLIA assay.

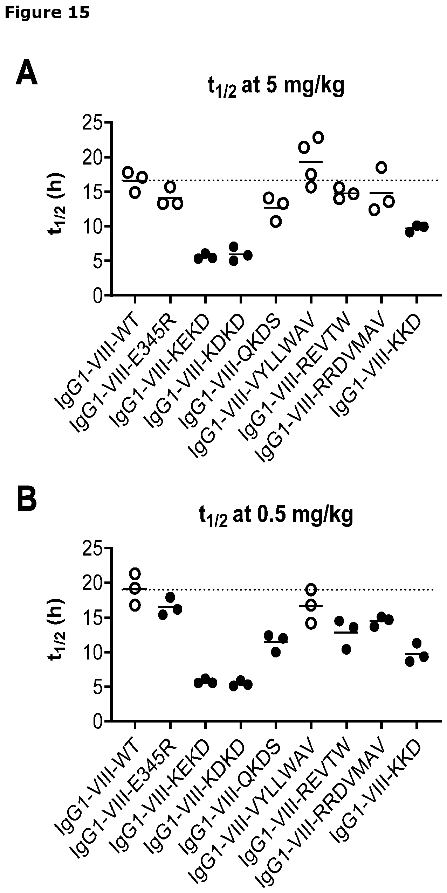

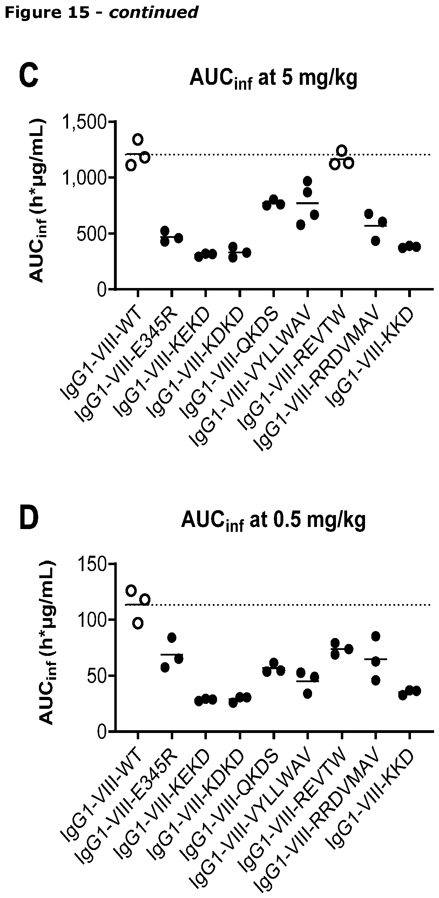

**Figure 15** - The terminal half-life ($t_{1/2}$) of IgG1-VIII antibodies injected in mice at approximately 5 mg/kg (A) or 0.5 mg/kg (B) and the area under the concentration-time curve from time point zero (t=0) to infinity ($AUC_{inf}$) for IgG1-VIII antibodies injected in mice at a dose of 5 mg/kg (C) or 0.5 mg/kg (D). Individual dots represent samples of individual mice and horizontal bars represent the group mean. For all mice indicated with filled black dots, the group mean significantly ($p$-value < 0.05) differs from the mean of the group of mice receiving IgG1-VIII-WT, as evaluated by one-way ANOVA with a post-hoc Dunnett's test to correct for multiple comparisons.

**Detailed description of the invention**

**Definitions**

According to the present invention, a *"binding agent"*, such as an antibody or fragment thereof, is capable of binding to a predetermined target if it has a significant affinity for said predetermined target and binds to said predetermined target in standard assays. "*Affinity*" or "*binding affinity*" is often measured by equilibrium dissociation constant (KD). Preferably, the term "*significant affinity*" refers to the binding to a predetermined target with a dissociation constant (KD) of $10^{-5}$ M or lower, $10^{-6}$ M or lower, $10^{-7}$ M or lower, $10^{-8}$ M or lower, $10^{-9}$ M or lower, $10^{-10}$ M or lower, $10^{-11}$ M or lower, or $10^{-12}$ M or lower.

The term "*immunoglobulin*" (IgG) refers to a class of structurally related glycoproteins consisting of two pairs of polypeptide chains, one pair of light (L) low molecular weight chains and one pair of heavy (H) chains, all four potentially interconnected by disulfide bonds. The structure of immunoglobulins has been well characterized. Briefly, each heavy chain typically is comprised of a heavy chain variable region (abbreviated herein as VH) and a heavy chain constant region. The heavy chain constant region typically is comprised of three domains, CH1, CH2, and CH3 and a so-called "hinge region". The heavy chains are inter-connected via disulfide bonds in the hinge region. Each light chain typically is comprised of a light chain variable region (abbreviated herein as VL) and a light chain constant region. The light chain constant region typically is comprised of one domain (abbreviated herein as CL). The VH and VL regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4 (see also Chothia and Lesk J. Mol. Biol. 196, 901 917 (1987)). Unless otherwise stated or contradicted by context, CDR sequences herein are identified according to IMGT rules using DomainGapAlign (Lefranc MP., Nucleic Acids Research 1999;27:209-212 and Ehrenmann F., Kaas Q. and Lefranc M.-P. Nucleic Acids Res., 38, D301-307 (2010); see also internet http address www.imgt.org/).

Unless otherwise stated or contradicted by context, reference to amino acid positions in the C regions, in the present invention is according to the Eu numbering (EU index) (Edelman et al., Proc Natl Acad Sci U SA. 1969 May;63(1):78-85; Kabat et al., Sequences of proteins of immunological interest. 5th Edition - 1991 NIH Publication No. 91-3242) and in V regions is according to the Kabat numbering (Kabat et al., Sequences of proteins of immunological interest. 5th Edition - 1991 NIH Publication No. 91-3242).

When reference is made to certain amino acid positions (e.g. in the context of IgG1), it will be clear that corresponding positions in similar protein sequences (such as other isotypes or allotypes), can be determined by the skilled person, e.g. by sequence alignment.

The *"Fc-region"* of an immunoglobulin is defined as the fragment of an antibody which would be typically generated after digestion of an antibody with papain (which is known for someone skilled in the art) which includes the two CH2-CH3 domains of an immunoglobulin and a connecting region, e.g. a hinge region. The constant domain of an antibody heavy chain defines the antibody isotype/subclass, e.g. IgG1, IgG2, IgG3, IgG4, IgA1, IgA2, IgM, IgD, or IgE. The Fc-region mediates the effector functions of antibodies with cell surface receptors called Fc receptors and proteins of the complement system.

In the context of the present invention the term *"hinge region"* refers to the hinge region of an immunoglobulin heavy chain. Thus, for example the hinge region of a human IgG1 antibody corresponds to amino acids 216-230 according to the Eu numbering. However, the hinge region may also be any of the other subtypes as described herein.

The term *"CH2 region"* or "*CH2 domain*" as used herein is intended to refer to the CH2 domain of an immunoglobulin heavy chain. Thus, for example the CH2 domain of a human IgG1 antibody corresponds to amino acids 231-340 according to the Eu numbering. However, the CH2 domain may also be any of the other subtypes as described herein. The term "*CH2 region*" or "*CH2 domain*" used interchangeably herein.

The term "*CH3 region*" or "*CH3 domain*" as used herein is intended to refer to the CH3 domain of an immunoglobulin heavy chain. Thus, for example the CH3 domain of a human IgG1 antibody corresponds to amino acids 341-447 according to the Eu numbering. However, the CH3 domain may also be any of the other subtypes as described herein. The term "*CH3 region*" or "*CH3 domain*" used interchangeably herein.

The term *"monomer"* or *"monomer polypeptide",* as used herein, refers to a single polypeptide chain or a single polypeptide molecule, that is capable of pairing with one or more other monomer polypeptides, to form a dimer, oligomer or multimer, due to the presence of at least one domain that promotes the polymerization due to interaction(s) between opposite domains in the one or more other monomers. A monomer polypeptide according to the present invention can for example be a heavy chain of an antibody or immunoglobulin, or a derivative thereof, such as a Fc-fusion protein, or a fragment thereof, such as a fragment comprising a CH3 domain or a fragment comprising an Fc region comprising a CH3 domain, wherein the CH3 domain can promote the polymerization with a CH3 domain from another monomer polypeptide. Antibody light chains are not monomer polypeptides in the context of the present invention.

In the context of the present invention, the term *"dimer"* refers to the product as formed by the polymerization of two monomer polypeptides comprising at least one domain that promotes the polymerization due to interaction(s) between opposite domains. In the context of the present invention, term *"heterodimer"* refers to the polymerization of two monomer polypeptides, which are not identical. In the context of the present invention, term *"homodimer"* refers to the polymerization of two identical monomer polypeptides such as an antibody heavy chain.

The term "*antibody*" (Ab) in the context of the present invention refers to an immunoglobulin molecule, a fragment of an immunoglobulin molecule, or a derivative of either thereof, which has the ability to specifically bind to an antigen. The antibody of the present invention typically comprises an Fc region of an immunoglobulin and an antigen-binding region. An antibody generally contains two CH2-CH3 domains and a connecting region, e.g. a hinge region. The variable regions of the heavy and light chains of the immunoglobulin molecule contain a binding domain that interacts with an antigen (an antigen-binding region). As described above, the Fc regions of the antibodies may mediate the binding of the immunoglobulin to host tissues or factors, including various cells of the immune system (such as effector cells) and components of the complement system such as C1q, the first component in the classical pathway of complement activation. In the context of the present invention, the term antibody includes without limitation VHH-Fc, scFv-Fc and scFab-Fc molecules.

The term "*full-length antibody*" when used herein, refers to an antibody which contains all heavy and light chain constant and variable domains corresponding to those that are normally found in a wild-type antibody of that isotype.

The term "*human antibody*", as used herein, is intended to include antibodies having variable and constant regions derived from human germline immunoglobulin sequences. The human antibodies of the invention may include amino acid residues not encoded by human germline immunoglobulin sequences (e.g. mutations, insertions or deletions introduced by random or site-specific mutagenesis in vitro or by somatic mutation in vivo). However, the term "*human antibody*", as used herein, is not intended to include antibodies in which CDR sequences derived from the germline of another species such as mammalian species, such as a mouse, have been grafted onto human framework sequences.

The terms "*HexaBody® technology*" or "*Hexabody® platform*" as used herein refers to an antibody Fc engineering technology platform developed to promote Fc:Fc interactions between individual antibodies and enhancing hexamer formation, such as by introducing the E430G, E345R or E345K mutation, as described in WO2013004842A2. Such enhanced Fc:Fc interactions may result in improved target-bound antibody clustering on the cell surface, leading to a variety of potential effects, including enhanced C1q binding and enhanced complement-dependent cytotoxicity.

The term *"Fc effector functions,"* as used herein, is intended to refer to functions that are a consequence of binding a polypeptide or antibody to its target, such as an antigen, on a cell membrane wherein the Fc effector function is attributable to the Fc region of the polypeptide or antibody. Examples of Fc effector functions include (i) C1q-binding, (ii) complement activation, (iii) complement-dependent cytotoxicity (CDC), (iv) antibody-dependent cell-mediated cytotoxity (ADCC), (v) Fc-gamma receptor-binding, (vi) antibody-dependent cellular phagocytosis (ADCP), (vii) complement-dependent cellular cytotoxicity (CDCC), (viii) complement-enhanced cytotoxicity, (ix) binding to complement receptor of an opsonized antibody mediated by the antibody, (x) opsonisation, (xi) trogocytosis, (xii) complement dependent cellular phagocytosis (CDCP) and (xiii) a combination of any of (i) to (xii).

As used herein, the term *"complement activation"* refers to the activation of the classical complement pathway, which is initiated by a large macromolecular complex called C1 binding to antibody-antigen complexes on a surface. C1 is a complex, which consists of 6 recognition proteins C1q and a hetero-tetramer of serine proteases, C1r2C1s2. C1 is the first protein complex in the early events of the classical complement cascade that involves a series of cleavage reactions that starts with the cleavage of C4 into C4a and C4b and C2 into C2a and C2b. C4b is deposited and forms together with C2a an enzymatic active convertase called C3 convertase, which cleaves complement component C3 into C3b and C3a, which forms a C5 convertase This C5 convertase splits C5 in C5a and C5b and the last component is deposited on the membrane and that in turn triggers the late events of complement activation in which terminal complement components C5b, C6, C7, C8 and C9 assemble into the membrane attack complex (MAC). The complement cascade results in the creation of pores in the cell membrane which causes lysis of the cell, also known as complement-dependent cytotoxicity (CDC). Complement activation can be evaluated by using C1q efficacy, CDC kinetics CDC assays (as described in WO2013/004842, WO2014/108198) or by the method Cellular deposition of C3b and C4b described in Beurskens et al., J Immunol April 1, 2012 vol. 188 no. 7, 3532-3541.

The term *"complement-dependent cytotoxicity"* (CDC), as used herein, is intended to refer to the process of antibody-mediated complement activation leading to lysis of the cell to which the antibody is bound, which, without being bound by theory is believed to be the result of pores in the membrane that are created by the assembly of the so-called membrane attack complex (MAC). Suitable assays for evaluating CDC are known in the art and include, for example, in vitro assays in which normal human serum is used as a complement source, as described in the Examples herein.

The term *"antibody-dependent cell-mediated cytotoxicity"* ("ADCC") as used herein, is intended to refer to a mechanism of killing of antibody-coated target cells by cells expressing Fc receptors that recognize the constant region of the bound antibody. Suitable assays for evaluating ADCC are known in the art.

The term *"antibody-dependent cellular phagocytosis"* ("ADCP") as used herein is intended to refer to a mechanism of elimination of antibody-coated target cells by internalization by phagocytes. The internalized antibody-coated target cells are contained in a vesicle called a phagosome, which then fuses with one or more lysosomes to form a phagolysosome. Suitable assays for evaluating ADCP are known in the art and include, for example, the in vitro cytotoxicity assay with macrophages as effector cells and video microscopy as described by van Bij et al. in Journal of Hepatology Volume 53, Issue 4, October 2010, Pages 677-685.

As used herein, *"trogocytosis"* refers to a process characterized by the transfer of cell surface molecules from a donor cell to an acceptor cell, such as an effector cell. Typical acceptor cells include T and B cells, monocytes/macrophages, dendritic cells, neutrophils, and NK cells. Trogocytosis-mediated transfer of a cell surface molecule such as, e.g., CD38, from a donor cell to an acceptor cell may also result in the transfer of an antibody-antigen complex from the donor cell to an acceptor cell, i.e., an antibody-antigen complex where an antibody is bound to the cell surface molecule. In particular, a specialized form of trogocytosis may occur when the acceptor cells are Fc-gamma-receptor (Fc$\gamma$R) expressing effector cells; these acceptor cells may take up and internalize donor cell-associated immune complexes composed of specific antibodies bound to target antigens on donor cells, typically after binding of Fc$\gamma$Rs to the Fc regions of the antibodies. Suitable assays for evaluating trogocytosis are known in the art.

By *"Fc gamma receptor"* or *"Fc$\gamma$R"* as used herein is meant any member of the family of proteins that binds the IgG antibody Fc region and is substantially encoded by one of the Fc$\gamma$R genes. In humans this family includes but is not limited to: Fc$\gamma$RI (CD64), including isoforms Fc$\gamma$RIa, Fc$\gamma$RIb, and Fc$\gamma$RIc; Fc$\gamma$RII (CD32), including isoforms Fc$\gamma$RIIa (including allotypes H131 and R131), Fc$\gamma$RIIb (including Fc$\gamma$RIIb-1 and Fc$\gamma$RIIb-2), and Fc$\gamma$RIIc; and Fc$\gamma$RIII (CD16), including isoforms Fc$\gamma$RIIIa (including allotypes V158 and F158) and Fc$\gamma$RIIIb (including allotypes Fc$\gamma$RIIIb-NA1 and Fc$\gamma$RIIIb-NA2), as well as any undiscovered human Fc$\gamma$Rs or Fc$\gamma$R isoforms or allotypes. An Fc$\gamma$R may be from any organism, including but not limited to humans, mice, rats, rabbits, and monkeys. Mouse Fc$\gamma$Rs include but are not limited to Fc$\gamma$RI (CD64), Fc$\gamma$RII (CD32), Fc$\gamma$RII) (CD16), and Fc$\gamma$RII)-2 (CD16-2), as well as any undiscovered mouse Fc$\gamma$Rs or Fc$\gamma$R isoforms or allotypes.

The term *"inert"*, as used herein, refers to the suppressed ability or inability of the Fc domain, through Fc engineering technology, of an antibody to execute Fc-mediated effector functions. Such Fc-mediated effector function can e.g. be measured by determining Fc-mediated CD69 expression on T cells in a co-culture of cells, by binding to Fc$\gamma$ receptors, by binding to C1q, or by induction of Fc-mediated cross-linking of FcRs. Mutations that impart inertness on antibodies have been discussed in e.g., Liu et al., Antibodies, 2020 Nov 17;9(4):64; 29(10):457-66. Examples of such inert mutations include e.g. G236R, G237A, L234A-L235A-P329G [AAG; Schlothauer et al., Protein Eng. Design and Selection 2016; 29(10):457-66], L234F-L235E-D265A [FEA; US10590206B2], L234F-L235E-G236R [FER; WO2022/189667A1] and P329R (as described e.g., in WO2018083126A1).

The term *"HexElect® technology"* or *"HexElect® platform"*, as used herein, refers to an antibody Fc engineering technology and platform developed to inhibit self-oligomerization of each of a pair of antibodies, such as by introducing K439E or S440K, and promote selective hetero-hexamerization of the said antibodies upon binding their cognate antigen on the same cell, as e.g. described in WO2019211472 and Oostindie et al. Logic-gated antibody pairs that selectively act on cells co-expressing two antigens. Nat Biotechnol 40(10):1509-1519 (2022).

The term *"prolonged IgG1 half-life"* refers to the extension of the serum half-life of IgG1 antibodies. Serum half-life of antibodies and their derivatives is dependent on their capacity to bind the Fc neonatal receptor (FcRn) at low pH (e.g., pH 6.0) in endosomes, which can be enhanced by Fc engineering, e.g., by introducing mutations M252Y, S254T, and T256E (YTE) in the IgG Fc domain, as described in e.g., Dall'Acqua, W. F. et al. Increasing the affinity of a human IgG1 for the neonatal Fc receptor: biological consequences. J. Immunol. 169, 5171-5180 (2002).

The term *"stable half antibody"* as used herein refers to an Fc engineering technology platform, such as UniBody, developed to promote weakened CH3:CH3 interactions in hinge-deleted or hinge mutated IgG molecules, such as by deleting the amino acid sequence ESKYGPPCPSCP of the hinge region and introducing F405T and Y407E mutations in human IgG4, as described e.g. in US 2012/0020952 A1. Such engineered IgG molecules may result in stable, effector-function silent monomeric half-antibodies that are monovalent, thus able to bind their target without activation of the target by cross-linking or by inducing effector function.

The term *"chimeric antibody"*, as used herein, refers to an antibody in which both chain types, i.e. heavy chain and light chain, are chimeric as a result of antibody engineering. A chimeric chain is a chain that contains a foreign variable domain (originating from a non-human species, or synthetic or engineered from any species including human) linked to a constant region of human origin.

The term *"isotype"* as used herein, refers to the immunoglobulin class (for instance IgG, IgD, IgA1, IgGA2, IgE, or IgM. IgGs may by further classified as subclasses IgG1, IgG2, IgG3 or IgG4. The term "subclass" as used herein, refers to the IgG1, IgG2, IgG3 and IgG4 subclasses of the IgG isotype. The IgG1 subclass may be further classified according to allotypes thereof such as IgG1m(za) and IgG1m(f) that is encoded by heavy chain constant region genes. Further, each heavy chain isotype can be combined with either a kappa or lambda light chain.

The term *"mixed isotype"* or *"mixed subclass"* used herein refers to the Fc region of an immunoglobulin generated by combining structural features of one isotype with the analogous region from another isotype thereby generating a hybrid isotype. A mixed isotype may comprise an Fc region having a sequence comprised of two or more isotypes selected from the following IgG1, IgG2, IgG3, IgG4, IgD, IgA1, IgGA2, IgE, or IgM thereby generating combinations such as e.g. IgG1/IgG3, IgG1/IgG4, IgG2/IgG3, IgG2/IgG4, or IgG1/IgA.

The term *"domain crossover"* as used herein, refers to an immunoglobulin in which the VH domain is exchanged with the VL domain and/or the CH1 domain is exchanged with the CL domain, as described in WO2015/101588 A1 and WO 2009/080253 A1. Further, the domain crossover may contain mutations at the VH/VL and/or CH1/CL interfaces.

The term *"target-binding region"* refers to a region of a binding agent that specifically binds a target, typically a protein on the surface of a cell. In preferred embodiments, the target-binding region is an antigen-binding region of an antibody, but in other embodiments, the target-binding region may be a different type of binding region, for example a ligand of a cell-surface receptor.

The term *"antigen-binding region", "antigen binding region", "binding region"* or *"antigen binding domain"*, as used herein, refers to a region of an antibody which is capable of binding to the antigen. This binding region is typically defined by the VH and VL domains of the antibody which may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). The antigen can be any molecule, such as a polypeptide, e.g. present on a cell, bacterium, or virion.

The term "allosteric modulation of the effector binding site", as used herein, is intended to refer to modulation of the conformational distribution of the effector binding sites in antibodies, such as IgG antibodies, through changes in amino acids that do not directly engage effector molecules, such as in the CH3 domains of (IgG) antibodies. Allostery in antibody Fc domains is e.g. described in Pincetic et al., Nat Immunol. 2014 Aug; 15(8): 707-716.

The term "effector binding region" or "effector binding site" as used herein, is intended to refer to a region or certain amino acids within for example an (IgG) antibody that can directly bind to and engage effector molecules, such as Fc receptors and complement components ( complement components are described under "complement activation")

The term *"vector"* as used herein, is intended to refer to a nucleic acid molecule capable of inducing transcription of a nucleic acid segment ligated into the vector. One type of vector is a *"plasmid"*, which is in the form of a circular double-stranded DNA loop. Another type of vector is a viral vector, wherein the nucleic acid segment may be ligated into the viral genome. Certain vectors are capable of autonomous replication in a host cell into which they are introduced (for instance bacterial vectors having a bacterial origin of replication and episomal mammalian vectors). Other vectors (such as non-episomal mammalian vectors) may be integrated into the genome of a host cell upon introduction into the host cell, and thereby are replicated along with the host genome. Moreover, certain vectors are capable of directing the expression of genes to which they are operatively linked. Such vectors are referred to herein as *"recombinant expression vectors"* (or simply, *"expression vectors"*). In general, expression vectors of utility in recombinant DNA techniques are often in the form of plasmids. However, the present invention is intended to include other forms of expression vectors, such as viral vectors (such as replication defective retroviruses, adenoviruses and adeno-associated viruses), which serve equivalent functions.

The term *"recombinant host cell"*, as used herein, is intended to refer to a cell into which one or more heterologous polynucleotides, such as one or more expression vectors has been introduced. It should be understood that such terms are intended to refer not only to the particular subject cell, but also to the progeny of such a cell. Because certain modifications may occur in succeeding generations due to either mutation or environmental influences, such progeny may not, in fact, be identical to the parent cell, but are still included within the scope of the term *"host cell"* as used herein. Recombinant host cells include, for example, transfectomas, such as CHO cells, HEK-293 cells, PER.C6, NS0 cells, and lymphocytic cells, and prokaryotic cells such as E. *coli* and other eukaryotic hosts such as plant cells and fungi.

The terms *"nucleic acid"* or *"nucleic acid molecule"* or *"polynucleotide"* as used herein, will be understood by a person skilled in the art and refers to a polymer comprising or consisting of nucleotide monomers that are covalently linked to each other by phosphodiester-bonds of a sugar/phosphate-backbone. The terms *"nucleic acid sequence" "nucleotide sequence"* or "polynucleotide sequence" will be recognized and understood by the person of ordinary skill in the art, and e.g. refer to a particular and individual order of the succession of its nucleotides.

The term *"coding sequence (cds)", "coding region"* or *"open reading frame (ORF)",* as used here a sequence of several nucleotide triplets, which may be translated into a peptide or protein. A coding sequence in the context of the present invention, may consist of a number of nucleotides that may be divided by three, which starts with a start codon and which preferably terminates with a stop codon.

The terms 'mutant', 'mutation', 'mutated' or 'substitution', 'substituted' in this context mean that another amino acid is present on the indicated position than in the corresponding parent molecule. A parent molecule in the present context can for example be a constant region, Fc region or CH3 region of a (human) IgG molecule, such as (human) IgG1, or (human) light chain constant regions. These will be understood by a person skilled in the art. Sequences of for example the human IgG1, IgG2, IgG3 and IgG4 constant regions as well as the human kappa and lambda light chain constant regions are disclosed herein. Such parent molecule may exist physically as polypeptide or in the form of nucleic acid encoding such polypeptide, but may also merely exist in silico or on paper as amino acid sequence or a corresponding nucleic acid sequence encoding the amino acid sequence. A mutation or substitution in this context is therefore also considered present for instance if a protein is expressed from a nucleic acid that has been synthesized such that it encodes the mutation or substitution, even though the nucleic acid encoding the corresponding parent molecule was not initially actually prepared during the process, e.g. when the nucleic acid molecule has been prepared entirely by chemical synthesis.

In the context of the present invention, a substitution in a variant may be indicated as:

*Original amino acid - position - substituted amino acid*

The three-letter code, or one letter code, is used, including the codes Xaa and X to indicate amino acid residue. Accordingly, the notation "Q347R" or "Gln347Arg" means that the variant comprises a substitution of Glutamine with Arginine in the variant amino acid position corresponding to the amino acid in position 347 in the reference antibody.

When used herein, phrases such as *"wherein said CH3 domain comprises any two, three or all four of the following residues: D351, K357, K366, E370"* or the like for other positions, mean that the CH3 domain may comprise any combination of two or three or all four of the specified residues, i.e. in this case:

D351 and K357,
D351 and K366,
D351 and E370,
K357 and K366,
K357 and E370,
K366 and E370,
D351, K357 and K366,
D351, K357 and E370,
D351, K366 and E370,
K357, K366 and E370, or
D351, K357, K366 and E370.

For purposes of the present invention, the *"sequence identity"* between two amino acid sequences or nucleotide sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment - Total Number of Gaps in Alignment).

The retention of similar residues may also or alternatively be measured by a similarity score, as determined by use of a BLAST program (e.g., BLAST 2.2.8 available through the NCBI using standard settings BLOSUM62, Open Gap=11 and Extended Gap=1). Suitable variants typically exhibit at least about 45%, such as at least about 55%, at least about 65%, at least about 75%, at least about 85%, at least about 90%, at least about 95%, or more (e.g., about 99%) similarity to the parent sequence.

The terms *"about"* or *"approximately"* are used herein, when parameters or values do not necessarily need to be identical, i.e. 100% the same. Accordingly, *"about"* means, that a parameter or value may be identical but may also diverge by 0.1% to 20%, preferably by 0.1% to 10%; in particular, by 0.5%, 1%, 2%, 3%, 4%, 5%, 6%, 7%, 8%, 9%, 10%, 11%, 12%, 13%, 14%, 15%, 16%, 17%, 18%, 19%, or 20%. The skilled person will know that e.g. certain parameters or values may slightly vary based on the method how the parameter is determined. For example, if a certain parameter or value is defined herein to have e.g. a length of *"about 1000 nucleotides"*, the length may be identical but may also diverge by 0.1%, 0.5%, 1%, 2%, 3%, 4%, 5%, 6%, 7%, 8%, 9%, 10%, 11%, 12%, 13%, 14%, 15%, 16%, 17%, 18%, 19%, 20%. Accordingly, the length may be identical or diverge by 1, 5, 10, 20, 30, 40, 50, 60, 70, 80, 90, 100, 110, 120, 130, 140, 150, 160, 170, 180, 190, 200 nucleotides.

### Binding agents

The inventors have surprisingly found that certain mutations introduced into the CH3 domain (CH3-CH3 interface) of antibodies, such as IgG antibodies, can have an effect on various Fc-mediated effector functions, such as FcgammaRIIa-H activation, FcgammaRIIb activation, FcgammaRIIIa-V, complement-dependent cytotoxicity (CDC) and FcRn binding, despite the fact that the present mutations are located outside the regions known to be involved in binding to the respective effector molecules (Fc receptors or complement components). Immune cells capable of engaging IgG antibodies regularly express different Fc receptors molecules at their surface, which makes approaches that simultaneously modulate the binding to multiple different Fc receptors particularly attractive. Without wishing to be bound by theory, it is believed that the present CH3 mutations can broadly regulate effector molecule engagement through allosteric modulation of the effector binding region.

Accordingly, it is an object of the invention to provide for binding agents having a modified capacity to bind to and/or induce the activation of one or more different effector molecules (e.g. Fc receptors and complement components) through allosteric modulation of the effector binding site.

As described above, in a first main aspect, the invention relates to binding agent comprising at least CH3 domain of a human IgG and a target-binding region, wherein said CH3 domain comprises one or more mutations in the CH3 domain (CH3-CH3 interface), such as at least one of the following amino acid residues:

    a) R347 or K347;
    b) V350;
    c) K351, Y351, D351 or E351;
    d) K356;
    e) Q357 or K357;
    f) E360 or D360;
    g) K364 or W364;
    h) W366, L366, K366 or V366;
    i) D368;
    j) S370, E370 or A370;
    k) D392, L392 or E392;
    l) W394;
    m) K399, V399 or M399;
    n) A405 or T405;
    o) A407 or V407;
    p) D409, W409 or V409; or
    q) E439,

wherein the numbering is according to the EU index.

Thus, the binding agent contains the specified amino acid residue at the specified position of the CH3 domain (for example an arginine (R) at position 347, the original being glutamine (Q)), wherein the position of the CH3 domain is numbered according to the Eu numbering (EU index) (Edelman et al., Proc Natl Acad Sci U SA. 1969 May;63(1) :78-85; Kabat et al., Sequences of proteins of immunological interest. 5th Edition 1991 NIH Publication No. 91-3242). It will be clear to the skilled person, that each of the mutations mentioned herein, when described by reference to (human or humanized) IgG1, can also be applied to the corresponding amino acids present in another IgG subclass, such as (human or humanized) IgG2, IgG3 and IgG4, in particular (human) IgG2 and IgG3, e.g. by making an alignment of the respective sequences. The CH3 regions or Fc regions may comprise variants of the parental IgG CH3/Fc region, such as of (human) IgG1, IgG2, IgG3 or IgG4 (see e.g. SEQ ID NO. 1, 24, 25 and 26)

Thus, in one aspect, the invention relates to a binding agent comprising at least CH3 domain of a human IgG and a target-binding region, wherein said CH3 domain comprises at least the amino acid residue R347. In one embodiment, said CH3 domain is dimeric and consists of two CH3 monomer polypeptides, wherein both CH3 monomer polypeptides contain at least the amino acid residue R347.

Thus, in one aspect, the invention relates to a binding agent comprising at least CH3 domain of a human IgG and a target-binding region, wherein said CH3 domain comprises at least the amino acid residue K347.

In one embodiment, said CH3 domain is dimeric and consists of two CH3 monomer polypeptides, wherein both CH3 monomer polypeptides contain at least the amino acid residue K347.

Thus, in one aspect, the invention relates to a binding agent comprising at least CH3 domain of a human IgG and a target-binding region, wherein said CH3 domain comprises at least the amino acid residue V350. In one embodiment, said CH3 domain is dimeric and consists of two CH3 monomer polypeptides, wherein both CH3 monomer polypeptides contain at least the amino acid residue V350.

Thus, in one aspect, the invention relates to a binding agent comprising at least CH3 domain of a human IgG and a target-binding region, wherein said CH3 domain comprises at least the amino acid residue K351. In one embodiment, said CH3 domain is dimeric and consists of two CH3 monomer polypeptides, wherein both CH3 monomer polypeptides contain at least the amino acid residue K351.

Thus, in one aspect, the invention relates to a binding agent comprising at least CH3 domain of a human IgG and a target-binding region, wherein said CH3 domain comprises at least the amino acid residue Y351. In one embodiment, said CH3 domain is dimeric and consists of two CH3 monomer polypeptides, wherein both CH3 monomer polypeptides contain at least the amino acid residue Y351.

Thus, in one aspect, the invention relates to a binding agent comprising at least CH3 domain of a human IgG and a target-binding region, wherein said CH3 domain comprises at least the amino acid residue D351. In one embodiment, said CH3 domain is dimeric and consists of two CH3 monomer polypeptides, wherein both CH3 monomer polypeptides contain at least the amino acid residue D351.

Thus, in one aspect, the invention relates to a binding agent comprising at least CH3 domain of a human IgG and a target-binding region, wherein said CH3 domain comprises at least the amino acid residue E351. In one embodiment, said CH3 domain is dimeric and consists of two CH3 monomer polypeptides, wherein both CH3 monomer polypeptides contain at least the amino acid residue E351.

Thus, in one aspect, the invention relates to a binding agent comprising at least CH3 domain of a human IgG and a target-binding region, wherein said CH3 domain comprises at least the amino acid residue K356. In one embodiment, said CH3 domain is dimeric and consists of two CH3 monomer polypeptides, wherein both CH3 monomer polypeptides contain at least the amino acid residue K356.

Thus, in one aspect, the invention relates to a binding agent comprising at least CH3 domain of a human IgG and a target-binding region, wherein said CH3 domain comprises at least the amino acid residue Q357.

In one embodiment, said CH3 domain is dimeric and consists of two CH3 monomer polypeptides, wherein both CH3 monomer polypeptides contain at least the amino acid residue Q357.

Thus, in one aspect, the invention relates to a binding agent comprising at least CH3 domain of a human IgG and a target-binding region, wherein said CH3 domain comprises at least the amino acid residue K357. In one embodiment, said CH3 domain is dimeric and consists of two CH3 monomer polypeptides, wherein both CH3 monomer polypeptides contain at least the amino acid residue K357.

Thus, in one aspect, the invention relates to a binding agent comprising at least CH3 domain of a human IgG and a target-binding region, wherein said CH3 domain comprises at least the amino acid residue E360. In one embodiment, said CH3 domain is dimeric and consists of two CH3 monomer polypeptides, wherein both CH3 monomer polypeptides contain at least the amino acid residue E360.

Thus, in one aspect, the invention relates to a binding agent comprising at least CH3 domain of a human IgG and a target-binding region, wherein said CH3 domain comprises at least the amino acid residue D360. In one embodiment, said CH3 domain is dimeric and consists of two CH3 monomer polypeptides, wherein both CH3 monomer polypeptides contain at least the amino acid residue D360.

Thus, in one aspect, the invention relates to a binding agent comprising at least CH3 domain of a human IgG and a target-binding region, wherein said CH3 domain comprises at least the amino acid residue K364. In one embodiment, said CH3 domain is dimeric and consists of two CH3 monomer polypeptides, wherein both CH3 monomer polypeptides contain at least the amino acid residue K364.

Thus, in one aspect, the invention relates to a binding agent comprising at least CH3 domain of a human IgG and a target-binding region, wherein said CH3 domain comprises at least the amino acid residue W364. In one embodiment, said CH3 domain is dimeric and consists of two CH3 monomer polypeptides, wherein both CH3 monomer polypeptides contain at least the amino acid residue W364.

Thus, in one aspect, the invention relates to a binding agent comprising at least CH3 domain of a human IgG and a target-binding region, wherein said CH3 domain comprises at least the amino acid residue W366. In one embodiment, said CH3 domain is dimeric and consists of two CH3 monomer polypeptides, wherein both CH3 monomer polypeptides contain at least the amino acid residue W366.

Thus, in one aspect, the invention relates to a binding agent comprising at least CH3 domain of a human IgG and a target-binding region, wherein said CH3 domain comprises at least the amino acid residue L366.

In one embodiment, said CH3 domain is dimeric and consists of two CH3 monomer polypeptides, wherein both CH3 monomer polypeptides contain at least the amino acid residue L366.

Thus, in one aspect, the invention relates to a binding agent comprising at least CH3 domain of a human IgG and a target-binding region, wherein said CH3 domain comprises at least the amino acid residue K366. In one embodiment, said CH3 domain is dimeric and consists of two CH3 monomer polypeptides, wherein both CH3 monomer polypeptides contain at least the amino acid residue K366.

Thus, in one aspect, the invention relates to a binding agent comprising at least CH3 domain of a human IgG and a target-binding region, wherein said CH3 domain comprises at least the amino acid residue V366. In one embodiment, said CH3 domain is dimeric and consists of two CH3 monomer polypeptides, wherein both CH3 monomer polypeptides contain at least the amino acid residue V366.

Thus, in one aspect, the invention relates to a binding agent comprising at least CH3 domain of a human IgG and a target-binding region, wherein said CH3 domain comprises at least the amino acid residue D368. In one embodiment, said CH3 domain is dimeric and consists of two CH3 monomer polypeptides, wherein both CH3 monomer polypeptides contain at least the amino acid residue D368.

Thus, in one aspect, the invention relates to a binding agent comprising at least CH3 domain of a human IgG and a target-binding region, wherein said CH3 domain comprises at least the amino acid residue S370. In one embodiment, said CH3 domain is dimeric and consists of two CH3 monomer polypeptides, wherein both CH3 monomer polypeptides contain at least the amino acid residue S370.

Thus, in one aspect, the invention relates to a binding agent comprising at least CH3 domain of a human IgG and a target-binding region, wherein said CH3 domain comprises at least the amino acid residue E370. In one embodiment, said CH3 domain is dimeric and consists of two CH3 monomer polypeptides, wherein both CH3 monomer polypeptides contain at least the amino acid residue E370.

Thus, in one aspect, the invention relates to a binding agent comprising at least CH3 domain of a human IgG and a target-binding region, wherein said CH3 domain comprises at least the amino acid residue A370. In one embodiment, said CH3 domain is dimeric and consists of two CH3 monomer polypeptides, wherein both CH3 monomer polypeptides contain at least the amino acid residue A370.

Thus, in one aspect, the invention relates to a binding agent comprising at least CH3 domain of a human IgG and a target-binding region, wherein said CH3 domain comprises at least the amino acid residue D392.

In one embodiment, said CH3 domain is dimeric and consists of two CH3 monomer polypeptides, wherein both CH3 monomer polypeptides contain at least the amino acid residue D392.

Thus, in one aspect, the invention relates to a binding agent comprising at least CH3 domain of a human IgG and a target-binding region, wherein said CH3 domain comprises at least the amino acid residue L392. In one embodiment, said CH3 domain is dimeric and consists of two CH3 monomer polypeptides, wherein both CH3 monomer polypeptides contain at least the amino acid residue L392.

Thus, in one aspect, the invention relates to a binding agent comprising at least CH3 domain of a human IgG and a target-binding region, wherein said CH3 domain comprises at least the amino acid residue E392. In one embodiment, said CH3 domain is dimeric and consists of two CH3 monomer polypeptides, wherein both CH3 monomer polypeptides contain at least the amino acid residue E392.

Thus, in one aspect, the invention relates to a binding agent comprising at least CH3 domain of a human IgG and a target-binding region, wherein said CH3 domain comprises at least the amino acid residue W394. In one embodiment, said CH3 domain is dimeric and consists of two CH3 monomer polypeptides, wherein both CH3 monomer polypeptides contain at least the amino acid residue W394.

Thus, in one aspect, the invention relates to a binding agent comprising at least CH3 domain of a human IgG and a target-binding region, wherein said CH3 domain comprises at least the amino acid residue K399. In one embodiment, said CH3 domain is dimeric and consists of two CH3 monomer polypeptides, wherein both CH3 monomer polypeptides contain at least the amino acid residue K399.

Thus, in one aspect, the invention relates to a binding agent comprising at least CH3 domain of a human IgG and a target-binding region, wherein said CH3 domain comprises at least the amino acid residue V399. In one embodiment, said CH3 domain is dimeric and consists of two CH3 monomer polypeptides, wherein both CH3 monomer polypeptides contain at least the amino acid residue V399.

Thus, in one aspect, the invention relates to a binding agent comprising at least CH3 domain of a human IgG and a target-binding region, wherein said CH3 domain comprises at least the amino acid residue M399. In one embodiment, said CH3 domain is dimeric and consists of two CH3 monomer polypeptides, wherein both CH3 monomer polypeptides contain at least the amino acid residue M399.

Thus, in one aspect, the invention relates to a binding agent comprising at least CH3 domain of a human IgG and a target-binding region, wherein said CH3 domain comprises at least the amino acid residue A405.

In one embodiment, said CH3 domain is dimeric and consists of two CH3 monomer polypeptides, wherein both CH3 monomer polypeptides contain at least the amino acid residue A405.

Thus, in one aspect, the invention relates to a binding agent comprising at least CH3 domain of a human IgG and a target-binding region, wherein said CH3 domain comprises at least the amino acid residue T405. In one embodiment, said CH3 domain is dimeric and consists of two CH3 monomer polypeptides, wherein both CH3 monomer polypeptides contain at least the amino acid residue T405.

Thus, in one aspect, the invention relates to a binding agent comprising at least CH3 domain of a human IgG and a target-binding region, wherein said CH3 domain comprises at least the amino acid residue A407. In one embodiment, said CH3 domain is dimeric and consists of two CH3 monomer polypeptides, wherein both CH3 monomer polypeptides contain at least the amino acid residue A407.

Thus, in one aspect, the invention relates to a binding agent comprising at least CH3 domain of a human IgG and a target-binding region, wherein said CH3 domain comprises at least the amino acid residue V407. In one embodiment, said CH3 domain is dimeric and consists of two CH3 monomer polypeptides, wherein both CH3 monomer polypeptides contain at least the amino acid residue V407.

Thus, in one aspect, the invention relates to a binding agent comprising at least CH3 domain of a human IgG and a target-binding region, wherein said CH3 domain comprises at least the amino acid residue D409. In one embodiment, said CH3 domain is dimeric and consists of two CH3 monomer polypeptides, wherein both CH3 monomer polypeptides contain at least the amino acid residue D409.

Thus, in one aspect, the invention relates to a binding agent comprising at least CH3 domain of a human IgG and a target-binding region, wherein said CH3 domain comprises at least the amino acid residue W409. In one embodiment, said CH3 domain is dimeric and consists of two CH3 monomer polypeptides, wherein both CH3 monomer polypeptides contain at least the amino acid residue W409.

Thus, in one aspect, the invention relates to a binding agent comprising at least CH3 domain of a human IgG and a target-binding region, wherein said CH3 domain comprises at least the amino acid residue V409. In one embodiment, said CH3 domain is dimeric and consists of two CH3 monomer polypeptides, wherein both CH3 monomer polypeptides contain at least the amino acid residue V409.

Thus, in one aspect, the invention relates to a binding agent comprising at least CH3 domain of a human IgG and a target-binding region, wherein said CH3 domain comprises at least the amino acid residue E439.

In one embodiment, said CH3 domain is dimeric and consists of two CH3 monomer polypeptides, wherein both CH3 monomer polypeptides contain at least the amino acid residue E439.

The CH3 domain of the binding agent may contain two or more or the specified amino acid residues at the specified positions, for example, two, three, four, five, six, seven, eight, nine or more the above-specified amino acid residues at the specified positions.

In one embodiment of the binding agent, said CH3 domain comprises any two, three, four or all five of the following residues: R347, E360, V399, T405, W409, i.e. said CH3 may comprise, in one or two CH3 monomer polypeptides:

- R347 and E360,
- R347 and V399,
- R347 and T405,
- R347 and W409,
- E360 and V399,
- E360 and T405,
- E360 and W409,
- V399 and T405,
- V399 and W409,
- T405 and W409,
- R347, E360 and V399,
- R347, E360 and T405,
- R347, E360 and W409,
- R347, V399 and T405,
- R347, V399 and W409,
- R347, T405 and W409,
- E360, V399 and T405,

- E360, V399 and W409,
- E360, T405 and W409,
- V399, T405 and W409,
- R347, E360, V399 and T405,
- R347, E360, V399 and W409,
- R347, E360, T405 and W409,
- R347, V399, T405 and W409,
- E360, V399, T405 and W409, or
- R347, E360, V399, T405 and W409.

In one embodiment, the binding agent is an antibody and comprises a constant region comprising the amino acid residues R347, E360, V399, T405 and W409, for example a constant region comprising the sequence set forth in SEQ ID NO:8.

In one embodiment of the binding agent, said CH3 domain comprises any two, three or all four of the following residues: K356, X392, K399, D409, wherein X is D or E, i.e. said CH3 may comprise, in one or two CH3 monomer polypeptides:

- K356 and X392, wherein X is D or E,
- K356 and K399,
- K356 and D409,
- X392 and K399, wherein X is D or E
- X392 and D409, wherein X is D or E
- K399 and D409,
- K356, X392 and K399, wherein X is D or E
- K356, X392 and D409, wherein X is D or E
- K356, K399 and D409,
- X392, K399 and D409, wherein X is D or E, or
- K356, X392, K399 and D409, wherein X is D or E

In one embodiment, the binding agent is an antibody and comprises a constant region comprising the amino acid residues K356, D392, K399 and D409, for example a constant region comprising the sequence set forth in SEQ ID NO:4.

In one embodiment, the binding agent is an antibody and comprises a constant region comprising the amino acid residues K356, E392, K399 and D409, for example a constant region comprising the sequence set forth in SEQ ID NO:5.

In one embodiment of the binding agent, said CH3 domain comprises any two, three or all four of the following residues: D351, K357, K366, E370, i.e. said CH3 may comprise, in one or two CH3 monomer polypeptides:

- D351 and K357,
- D351 and K366,
- D351 and E370,
- K357 and K366,
- K357 and E370,
- K366 and E370,
- D351, K357 and K366,
- D351, K357 and E370,
- D351, K366 and E370,
- K357, K366 and E370, or
- D351, K357, K366 and E370.

In one embodiment, the binding agent is an antibody and comprises a constant region comprising the amino acid residues D351, K357, K366 and E370, for example a constant region comprising the sequence set forth in SEQ ID NO:21.

In one embodiment of the binding agent, said CH3 domain comprises, in one or two CH3 monomer polypeptides: K347 and/or E360.

In one embodiment of the binding agent, said CH3 domain comprises any two, three, four, five or all six of the following residues: K347, D351, K357, E360, K366 and E370, i.e. said CH3 may comprise, in one or two CH3 monomer polypeptides:

- K347 and D351,
- K347 and K357,

- K347 and E360,
- K347 and K366,
- K347 and E370,
- D351 and K357,
- D351 and E360,
- D351 and K366,
- D351 and E370,
- K357 and E360,
- K357 and K366,
- K357 and E370,
- E360 and K366,
- E360 and E370,
- K366 and E370,
- K347, D351 and K357,
- K347, D351 and E360,
- K347, D351 and K366,
- K347, D351 and E370,
- K347, K357 and E360,
- K347, K357 and K366
- K347, K357 and E370,
- K347, E360 and K366,
- K347, E360 and E370,
- K347, K366 and E370,
- D351, K357 and E360,
- D351, K357 and K366,
- D351, K357 and E370,
- D351, E360 and K366,
- D351, E360 and E370,
- D351, K366 and E370,
- K357, E360 and K366,
- K357, E360 and E370,
- K357, K366 and E370,
- E360, K366 and E370,
- K347, D351, K357 and E360,
- K347, D351, K357 and K366,
- K347, D351, K357 and E370,
- K347, D351, E360 and K366,
- K347, D351, E360 and E370,
- K347, D351, K366 and E370,
- K347, K357, E360 and K366,
- K347, K357, E360 and E370,
- K347, K357, K366 and E370,
- K347, E360, K366 and E370,
- D351, K357, E360 and K366,
- D351, K357, E360 and E370,
- D351, K357, K366 and E370,
- D351, E360, K366 and E370,
- K357, E360, K366 and E370,
- K347, D351, K357, E360 and K366,
- K347, D351, K357, E360 and E370,
- K347, D351, K357, K366 and E370,
- K347, D351, E360, K366 and E370,
- K347, K357, E360, K366 and E370,
- D351, K357, E360, K366 and E370, or
- K347, D351, K357, E360, K366 and E370.

In one embodiment, the binding agent is an antibody and comprises a constant region comprising the amino acid residues K347, D351, K357, E360, K366 and E370, for example a constant region comprising the sequence set forth in SEQ ID NO:17.

In one embodiment of the binding agent, said CH3 domain comprises, in one or two CH3 monomer polypeptides: K356 and E439.

In one embodiment of the binding agent, said CH3 domain comprises, in one or two CH3 monomer polypeptides:

a) any two, three or all four of the following residues: K356, V399, W409, E439, e.g.

- K356 and V399,
- K356 and W409,
- K356 and E439,
- V399 and W409,
- V399 and E439,
- W409 and E439,
- K356, V399 and W409,
- K356, V399 and E439,
- K356, W409 and E439,
- V399, W409 and E439, or
- K356, V399, W409 and E439,

or

b) any two, three or all four of the following residues: D351, K356, K366, E439, e.g.

- D351 and K356,
- D351 and K366,
- D351 and E439,
- K356 and K366,
- K356 and E439,
- K366 and E439,
- D351, K356 and K366,
- D351, K356 and E439,
- D351, K366 and E439,
- K356, K366 and E439, or
- D351, K356, K366 and E439,

or

c) any two, three, four or all five of the following residues: K356, D392, V399, W409, E439, e.g.

- K356 and D392,
- K356 and V399,
- K356 and W409,
- K356 and E439,
- D392 and V399,
- D392 and W409,
- D392 and E439,
- V399 and W409,
- V399 and E439,
- W409 and E439,
- K356, D392 and V399,
- K356, D392 and W409,
- K356, D392 and E439,
- K356, V399 and W409,
- K356, V399 and E439,
- K356, W409 and E439,
- D392, V399 and W409,
- D392, W409 and E439,
- D392, V399 and E439,
- V399, W409 and E439,
- K356, D392, V399 and W409,

- K356, D392, V399 and E439,
- K356, D392, W409 and E439,
- K356, V399, W409 and E439,
- D392, V399, W409 and E439, or
- K356, D392, V399, W409 and E439.

In one embodiment, the binding agent is an antibody and comprises a constant region comprising the amino acid residues K356, V399, W409 and E439, for example a constant region comprising the sequence set forth in SEQ ID NO:15.

In one embodiment, the binding agent is an antibody and comprises a constant region comprising the amino acid residues D351, K356, K366 and E439, for example a constant region comprising the sequence set forth in SEQ ID NO:12.

In one embodiment, the binding agent is an antibody and comprises a constant region comprising the amino acid residues K356, D392, V399, W409 and E439, for example a constant region comprising the sequence set forth in SEQ ID NO:18.

In one embodiment of the binding agent, said CH3 domain comprises, in one or two CH3 monomer polypeptides, K399 and D409.

In one embodiment of the binding agent, said CH3 domain comprises, in one or two CH3 monomer polypeptides:

a) any two or all three of the following residues: K351, K399, D409, e.g.

- K351 and K399,
- K351 and D409,
- K399 and D409, or
- K351, K399 and D409,

or
b) any two, three, four or all five of the following residues: D351, K366, D392, K399, D409, e.g.:

- D351 and K366,
- D351 and D392,
- D351 and K399,
- D351 and D409,
- K366 and D392,
- K366 and K399,
- K366 and D409,
- D392 and K399,
- D392 and D409,
- K399 and D409,
- D351, K366 and D392,
- D351, K366 and K399,
- D351, K366 and D409,
- D351, D392 and K399,
- D351, D392 and D409,
- D351, K399 and D409,
- K366, D392 and K399,
- K366, D392 and D409,
- K366, K399 and D409,
- D392, K399 and D409,
- D351, K366, D392 and K399,
- D351, K366, D392 and D409,
- D351, K366, K399 and D409,
- D351, D392, K399 and D409,
- K366, D392, K399 and D409, or
- D351, K366, D392, K399 and D409,

or
c) any two, three, four, five or all six of the following residues: K356, W366, D392, K399, A407, D409, e.g.:

- K356 and W366,

- K356 and D392,
- K356 and K399,
- K356 and A407,
- K356 and D409,
- W366 and D392,
- W366 and K399,
- W366 and A407,
- W366 and D409,
- D392 and K399,
- D392 and A407,
- D392 and D409,
- K399 and A407,
- K399 and D409,
- A407 and D409,
- K356, W366 and D392,
- K356, W366 and K399,
- K356, W366 and A407,
- K356, W366 and D409,
- K356, D392 and K399,
- K356, D392 and A407,
- K356, D392 and D409,
- K356, K399 and A407,
- K356, K399 and D409,
- K356, A407 and D409,
- W366, D392 and K399,
- W366, D392 and A407,
- W366, D392 and 409,
- W366, K399 and A407,
- W366, K399 and D409,
- W366, A407 and D409,
- D392, K399 and A407,
- D392, K399 and D409,
- D392, A407 and D409,
- K399, A407 and D409,
- K356, W366, D392 and K399,
- K356, W366, D392 and A407,
- K356, W366, D392 and D409,
- K356, W366, K399 and A407,
- K356, W366, K399 and D409,
- K356, W366, A407 and D409,
- K356, D392, K399 and A407,
- K356, D392, K399 and D409,
- K356, D392, A407 and D409,
- K356, K399, A407 and D409,
- W366, D392, K399 and A407,
- W366, D392, K399 and D409,
- W366, D392, A407 and D409,
- W366, K399, A407 and D409
- D392, K399, A407 and D409,
- K356, W366, D392, K399 and A407,
- K356, W366, D392, K399 and D409,
- K356, W366, D392, A407 and D409,
- K356, W366, K399, A407 and D409,
- K356, D392, K399, A407 and D409,
- W366, D392, K399, A407 and D409, or
- K356, W366, D392, K399, A407 and D409.

In one embodiment, the binding agent is an antibody and comprises a constant region comprising the amino acid residues K351, K399 and D409, for example a constant region comprising the sequence set forth in SEQ ID NO:11.

In one embodiment, the binding agent is an antibody and comprises a constant region comprising the amino acid residues D351, K366, D392, K399 and D409, for example a constant region comprising the sequence set forth in SEQ ID NO:16.

In one embodiment, the binding agent is an antibody and comprises a constant region comprising the amino acid residues K356, W366, D392, K399, A407 and D409, for example a constant region comprising the sequence set forth in SEQ ID NO:22.

In one embodiment of the binding agent, said CH3 domain comprises K347 and E360, in one or two CH3 monomer polypeptides.

In one embodiment of the binding agent, said CH3 domain comprises D351 and K366.

In one embodiment of the binding agent, said CH3 domain comprises, in one or two CH3 monomer polypeptides:

a) any two, three or all four of the following residues: K347, E360, V399, W409, e.g.:

- K347 and E360,
- K347 and V399,
- K347 and W409,
- E360 and V399,
- E360 and W409,
- V399 and W409,
- K347, E360 and V399,
- K347, E360, and W409,
- K347, V399 and W409,
- E360, V399 and W409,
- K347, E360, V399 and W409

or

b) any two, three or all four of the following residues: K347, D351, E360, K366, e.g.:

- K347 and D351,
- K347 and E360,
- K347 and K366,
- D351 and E360,
- D351 and K366,
- E360 and K366,
- K347, D351 and E360,
- K347, D351 and K366,
- K347, E360 and K366,
- D351, E360 and K366, or
- K347, D351, E360 and K366.

In one embodiment, the binding agent is an antibody and comprises a constant region comprising the amino acid residues K347, E360, V399 and W409, for example a constant region comprising the sequence set forth in SEQ ID NO:20.

In one embodiment, the binding agent is an antibody and comprises a constant region comprising the amino acid residues K347, D351, E360 and K366, for example a constant region comprising the sequence set forth in SEQ ID NO:13.

In one embodiment of the binding agent, said CH3 domain comprises, in one or two CH3 monomer polypeptides:

a) any two, three or all four of the following residues: Q357, K364, D368, S370, e.g.;

- Q357 and K364,
- Q357 and D368,
- Q357 and S370,
- K364 and D368,
- K364 and S370,
- D368, S370,
- Q357, K364 and D368,
- Q357, K364 and S370,
- Q357, D368 and S370,

- K364, D368 and S370, or
- Q357, K364, D368 and S370,

or

b) any two, three or all four of the following residues: W364, A370, D392, K399, e.g.;

- W364 and A370,
- W364 and D392,
- W364 and K399,
- A370 and D392,
- A370 and K399,
- D392 and K399,
- W364, A370 and D392,
- W364, A370, and K399,
- W364, D392 and K399,
- A370, D392 and K399, or
- W364, A370, D392 and K399,

or

c) any two, three, four, five or all six of the following residues: R347, D360, V366, M399, A407, V409, e.g.:

- R347 and D360,
- R347 and V366,
- R347 and M399,
- R347 and A407,
- R347 and V409,
- D360 and V366,
- D360 and M399
- D360 and A407,
- D360 and V409,
- V366 and M399,
- V366 and A407,
- V366 and V409,
- M399 and A407,
- M399 and V409,
- A407 and V409,
- R347, D360 and V366,
- R347, D360 and M399,
- R347, D360 and A407,
- R347, D360 and V409,
- R347, V366 and M399,
- R347, V366 and A407,
- R347, V366 and V409,
- R347, M399 and A407,
- R347, M399 and V409,
- R347, A407 and V409,
- D360, V366 and M399,
- D360, V366 and A407,
- D360, V366 and V409,
- D360, M399 and A407,
- D360, M399 and V409,
- D360, A407 and V409,
- V366, M399 and A407,
- V366, M399 and V409,
- V366, A407 and V409,
- M399, A407 and V409,
- R347, D360, V366 and M399,
- R347, D360, V366 and A407,

- R347, D360, V366 and V409,
- R347, D360, M399 and A407,
- R347, D360, M399 and V409,
- R347, D360, A407 and V409,
- R347, V366, M399 and A407,
- R347, V366, M399 and V409,
- R347, V366, A407 and V409,
- R347, M399, A407 and V409,
- D360, V366, M399 and A407,
- D360, V366, M399 and V409,
- D360, V366, A407 and V409,
- D360, M399, A407 and V409,
- V366, M399, A407 and V409,
- R347, D360, V366, M399 and A407,
- R347, D360, V366, M399 and V409,
- R347, D360, V366, A407 and V409,
- R347, D360, M399, A407 and V409,
- R347, V366, M399, A407 and V409,
- D360, V366, M399, A407 and V409, or
- R347, D360, V366, M399, A407 and V409.

or any of the above and R345,
or
d) any two, three, four, five, six or all seven of the following residues: V350, Y351, L366, L392, W394, A405, V407, e.g.:

- V350 and Y351,
- V350 and L366,
- V350 and L392,
- V350 and W394,
- V350 and A405,
- V350 and V407,
- Y351 and L366,
- Y351 and L392,
- Y351 and W394,
- Y351 and A405,
- Y351 and V407,
- L366 and L392,
- L366 and W394,
- L366 and A405,
- L366 and V407,
- L392 and W394,
- L392 and A405,
- L392 and V407,
- W394 and A405,
- W394 and V407,
- A405 and V407,
- V350, Y351 and L366,
- V350, Y351 and L392,
- V350, Y351 and W394,
- V350, Y351 and A405,
- V350, Y351 and V407,
- V350, L366 and L392,
- V350, L366 and W394,
- V350, L366 and A405,
- V350, L366 and V407,
- V350, L392 and W394,
- V350, L392 and A405,
- V350, L392 and V407,

- V350, W394 and A405,
- V350, W394 and V407,
- V350, A405 and V407,
- Y351, L366 and L392,
- Y351, L366 and W394,
- Y351, L366 and A405,
- Y351, L366 and V407,
- Y351, L392 and W394,
- Y351, L392 and A405,
- Y351, L392 and V407,
- Y351, W394 and A405,
- Y351, W394 and V407,
- Y351, A405 and V407,
- L366, L392 and W394,
- L366, L392 and A405,
- L366, L392 and V407,
- L366, W394 and A405,
- L366, W394 and V407,
- L366, A405 and V407,
- L392, W394 and A405,
- L392, W394 and V407,
- L392, A405 and V407,
- W394, A405 and V407,
- V350, Y351, L366 and L392,
- V350, Y351, L366 and W394,
- V350, Y351, L366 and A405,
- V350, Y351, L366 and V407,
- V350, Y351, L392 and W394,
- V350, Y351, L392 and A405,
- V350, Y351, L392 and V407,
- V350, Y351, W394 and A405,
- V350, Y351, W394 and V407,
- V350, Y351, A405 and V407,
- V350, L366, L392 and W394,
- V350, L366, L392 and A405,
- V350, L366, L392 and V407,
- V350, L366, W394 and A405,
- V350, L366, W394 and V407,
- V350, L366, A405 and V407,
- V350, L392, W394 and A405,
- V350, L392, W394 and V407,
- V350, L392, A405 and V407,
- V350, W394, A405 and V407,
- Y351, L366, L392 and W394,
- Y351, L366, L392 and A405,
- Y351, L366, L392 and V407,
- Y351, L366, W394 and A405,
- Y351, L366, W394 and V407,
- Y351, L366, A405 and V407,
- Y351, L392, W394 and A405,
- Y351, L392, W394 and V407,
- Y351, L392, A405 and V407,
- Y351, W394, A405 and V407,
- L366, L392, W394 and A405,
- L366, L392, W394 and V407,
- L366, L392, A405 and V407,
- L366, W394, A405 and V407,
- L392, W394, A405 and V407,

- V350, Y351, L366, L392 and W394,
- V350, Y351, L366, L392 and A405,
- V350, Y351, L366, L392 and V407,
- V350, Y351, L366, W394 and A405,
- V350, Y351, L366, W394 and V407,
- V350, Y351, L366, A405 and V407,
- V350, Y351, L392, W394 and A405,
- V350, Y351, L392, W394 and V407,
- V350, Y351, L392, A405 and V407,
- V350, Y351, W394, A405 and V407,
- V350, L366, L392, W394 and A405,
- V350, L366, L392, W394 and V407,
- V350, L366, L392, A405 and V407,
- V350, L366, W394, A405 and V407,
- V350, L392, W394, A405 and V407,
- Y351, L366, L392, W394 and A405,
- Y351, L366, L392, W394 and V407,
- Y351, L366, L392, A405 and V407,
- Y351, L366, W394, A405 and V407,
- Y351, L392, W394, A405 and V407,
- L366, L392, W394, A405 and V407,
- V350, Y351, L366, L392, W394 and A405,
- V350, Y351, L366, L392, W394 and V407,
- V350, Y351, L366, L392, A405 and V407,
- V350, Y351, L366, W394, A405 and V407,
- V350, Y351, L392, W394, A405 and V407,
- V350, L366, L392, W394, A405 and V407,
- Y351, L366, L392, W394, A405 and V407, or
- V350, Y351, L366, L392, W394, A405 and V407,

or

e) any two, three, four, five, six or all seven of the following residues: K347, E351, K357, E360, K364, D368, E370, e.g:

- K347 and E351,
- K347 and K357,
- K347 and E360,
- K347 and K364,
- K347 and D368,
- K347 and E370,
- E351 and K357,
- E351 and E360,
- E351 and K364,
- E351 and D368,
- E351 and E370,
- K357 and E360,
- K357 and K364,
- K357 and D368,
- K357 and E370,
- E360 and K364,
- E360 and D368,
- E360 and E370,
- K364 and D368,
- K364 and E370,
- D368 and E370,
- K347, E351 and K357,
- K347, E351 and E360,
- K347, E351 and K364,
- K347, E351 and D368,

- K347, E351 and E370,
- K347, K357 and E360,
- K347, K357 and K364,
- K347, K357 and D368,
- K347, K357 and E370,
- K347, E360 and K364,
- K347, E360 and D368,
- K347, E360 and E370,
- K347, K364 and D368,
- K347, K364 and E370,
- K347, D368 and E370,
- E351, K357 and E360,
- E351, K357 and K364,
- E351, K357 and D368,
- E351, K357 and E370,
- E351, E360 and K364,
- E351, E360 and D368,
- E351, E360 and E370,
- E351, K364 and D368,
- E351, K364 and E370,
- E351, D368 and E370,
- K357, E360 and K364,
- K357, E360 and D368,
- K357, E360 and E370,
- K357, K364 and D368,
- K357, K364 and E370,
- K357, D368 and E370,
- E360, K364 and D368,
- E360, K364 and E370,
- E360, D368 and E370,
- K364, D368 and E370,
- K347, E351, K357 and E360,
- K347, E351, K357 and K364,
- K347, E351, K357 and D368,
- K347, E351, K357 and E370,
- K347, E351, E360 and K364,
- K347, E351, E360 and D368,
- K347, E351, E360 and E370,
- K347, E351, K364 and D368,
- K347, E351, K364 and E370,
- K347, E351, D368 and E370,
- K347, K357, E360 and K364,
- K347, K357, E360 and D368,
- K347, K357, E360 and E370,
- K347, K357, K364 and D368,
- K347, K357, K364 and E370,
- K347, K357, D368 and E370,
- K347, E360, K364 and D368,
- K347, E360, K364 and E370,
- K347, E360, D368 and E370,
- K347, K364, D368 and E370,
- E351, K357, E360 and K364,
- E351, K357, E360 and D368,
- E351, K357, E360 and E370,
- E351, K357, K364 and D368,
- E351, K357, K364 and E370,
- E351, K357, D368 and E370,
- E351, E360, K364 and D368,

- E351, E360, K364 and E370,
- E351, E360, D368 and E370,
- E351, K364, D368 and E370,
- K357, E360, K364 and D368,
- K357, E360, K364 and E370,
- K357, E360, D368 and E370,
- K357, K364, D368 and E370,
- E360, K364, D368 and E370,
- K347, E351, K357, E360 and K364,
- K347, E351, K357, E360 and D368,
- K347, E351, K357, E360 and E370,
- K347, E351, K357, K364 and D368,
- K347, E351, K357, K364 and E370,
- K347, E351, K357, D368 and E370,
- K347, E351, E360, K364 and D368,
- K347, E351, E360, K364 and E370,
- K347, E351, E360, D368 and E370,
- K347, E351, K364, D368 and E370,
- K347, K357, E360, K364 and D368,
- K347, K357, E360, K364 and E370,
- K347, K357, E360, D368 and E370,
- K347, K357, K364, D368 and E370,
- K347, E360, K364, D368 and E370,
- E351, K357, E360, K364 and D368,
- E351, K357, E360, K364 and E370,
- E351, K357, E360, D368 and E370,
- E351, K357, K364, D368 and E370,
- E351, E360, K364, D368 and E370,
- K357, E360, K364, D368 and E370,
- K347, E351, K357, E360 and K364,
- K347, E351, K357, E360, K364 and D368,
- K347, E351, K357, E360, K364 and E370,
- K347, E351, K357, E360, D368 and E370,
- K347, E351, K357, K364, D368 and E370,
- K347, E351, E360, K364, D368 and E370,
- K347, K357, E360, K364, D368 and E370,
- E351, K357, E360, K364, D368 and E370,
- K347, E351, K357, E360, K364, D368 and E370.

In one embodiment, the binding agent is an antibody and comprises a constant region comprising the amino acid residues Q357, K364, D368 and S370, for example a constant region comprising the sequence set forth in SEQ ID NO:6.

In one embodiment, the binding agent is an antibody and comprises a constant region comprising the amino acid residues W364, A370, D392 and K399, for example a constant region comprising the sequence set forth in SEQ ID NO:14.

In one embodiment, the binding agent is an antibody and comprises a constant region comprising the amino acid residues R347, D360, V366, M399, A407 and V409, and optionally R345, for example a constant region comprising the sequence set forth in SEQ ID NO:9 or 10.

In one embodiment, the binding agent is an antibody and comprises a constant region comprising the amino acid residues V350, Y351, L366, L392, W394, A405 and V407, for example a constant region comprising the sequence set forth in SEQ ID NO:7.

In one embodiment, the binding agent is an antibody and comprises a constant region comprising the amino acid residues K347, E351, K357, E360, K364, D368 and E370, for example a constant region comprising the sequence set forth in SEQ ID NO:19.

### Binding agent formats and further modifications

In preferred embodiments, the CH3 domain of the binding agent is dimeric and consists of two CH3 monomer polypeptides. The dimeric CH3 domain may be a homodimer or a heterodimer. Preferably, both CH3 monomer polypeptides in the CH3 dimer contain the specified amino acid residues at the specified positions, e.g., both CH3 monomer polypeptides contain R347, etc.

The binding agent may, e.g., be an antibody or a fusion protein comprising fragments of an antibody.

The binding agent of the present invention comprises at least a CH3 domain of a human IgG, but may contain further regions or domains of other antibody classes. In one embodiment, the binding agent is of mixed isotype or mixed subclass, such as e.g. IgG1/IgG3, IgG1/IgG4, IgG2/IgG3, IgG2/IgG4, or IgG1/IgA.

Preferably, the binding agent is an antibody and the target-binding region is an antigen-binding region. Various classes of antibodies are known in the art, such as IgG, IgA, IgM, IgE antibodies, etc.

The antibody may be a mammalian antibody for example selected from the group consisting of human, primates, mouse, rabbit, camelid, and rat. In one embodiment, the antibody is a human antibody. In one embodiment, the antibody is a humanized antibody.

The CH3 domain of the binding region may be of the IgG1, IgG2, IgG3 or IgG4 isotype. In one embodiment, the CH3 domain is of the IgG1 isotype. In one embodiment, the CH3 domain is of the IgG2 isotype. In one embodiment, the CH3 domain is of the IgG3 isotype. In one embodiment, the CH3 domain is of the IgG4 isotype.

The binding agent may further comprise a CH2 domain, for example of the same isotype as the CH3 domain.

The binding agent may further comprise a hinge region, this hinge region may be of the same isotype as the CH3 domain or of a different isotype.

Thus, the binding agent comprises may comprises a full Fc region, i.e. a hinge region, CH2 domain and CH3 domain. The full Fc region may be of the IgG1, IgG2, IgG3 or IgG4 subclass. In one embodiment, the full Fc region is of the IgG1 isotype. In one embodiment, the full Fc region is of the IgG2 isotype. In one embodiment, the full Fc region is of the IgG3 isotype. In one embodiment, the full Fc region is of the IgG4 isotype.

In embodiments where the binding agent is an antibody, it contains an antigen-binding region. The antigen-binding region may comprise a heavy chain variable region and a light chain variable region or it may, for example, comprise a single-domain antigen binding region, such as a VHH, e.g. a camelid VHH or humanized VHH. The antigen-binding region may be human, humanized or chimeric. The heavy chain variable region may be followed by a CH1 domain. If a light chain is present, it may be a kappa or a lambda light chain. The light chain variable region may be followed by a CL domain.

In one embodiment, the light chain may be a kappa or lambda light chain as represented by SEQ ID NO: 3 and SEQ ID NO: 4, respectively (constant regions of human kappa and lambda light chains respectively).

In some embodiments, the binding agent is a full-length antibody containing all regions and domain of a naturally-occurring antibody of that type, for example of the IgG1, IgG2, IgG3 or IgG4 subclass. In one embodiment, the binding agent is a full-length antibody of the IgG1 isotype. In one embodiment, the binding agent is a full-length antibody of the IgG2 isotype. In one embodiment, the binding agent is a full-length antibody of the IgG3 isotype. In one embodiment, the binding agent is a full-length antibody of the IgG4 isotype.

In some embodiments, the CH3 regions or Fc regions of the binding agent or antibody do not comprise any further mutations than the mutations specified herein. In other embodiments, however the binding agent comprises one or more further mutations, deletions or insertion besides the ones specified, for example in the Fc region, such as in the CH3 domain.

In some embodiment, the binding agent is an antibody which comprises one or more further mutations, deletions or insertion besides the ones specified, for example in the heavy chain constant region, such as in the Fc region or the CH3 domain.

In one embodiment, the binding agent is an antibody and comprises up to 20, such as up to 10, e.g. 1, 2, 3, 4, 5, 6, 7, 8, 9 or 10 further mutations besides the ones specified in the constant region, for example relative to the sequence set forth in SEQ ID NO:1.

In one embodiment, the binding agent is an antibody and comprises up to 20, such as up to 10, e.g. 1, 2, 3, 4, 5, 6, 7, 8, 9 or 10 further mutations besides the ones specified in the Fc region, for example relative to the Fc region of the sequence set forth in SEQ ID NO:1.

In one embodiment, the binding agent is an antibody and comprises up to 20, such as up to 10, e.g. 1, 2, 3, 4, 5, 6, 7, 8, 9 or 10 further mutations besides the ones specified in the CH3 domain, for example relative to the CH3 domain of the sequence set forth in SEQ ID NO:1.

In one embodiment, said further mutation(s) are ones that enhances Fc:Fc interactions between individual binding agent molecules. Such mutations include E430G, E345R and E345K (numbering is according to the EU index) (i.e. HexaBody® technology; WO2013004842A2).

Thus:
In one embodiment, the binding agent comprises the substitution E430G and any one, two, three, four or all five of the following residues: R347, E360, V399, T405, W409.

In one embodiment, the binding agent comprises the substitution E430G and any one, two, three, or all four of the following residues: K356, X392, K399, D409, wherein X is D or E .

In one embodiment, the binding agent comprises the substitution E430G and any one, two, three or all four or the following residues: D351, K357, K366, E370.

In one embodiment, the binding agent comprises the substitution E430G and any one, two, three, four, five or all six of the following residues: D351, K357, K366, E370, K347, E360.

In one embodiment, the binding agent comprises the substitution E430G and the residues K356 and E439.

In one embodiment, the binding agent comprises the substitution E430G and any one, two, three or all four of the following residues: K356, V399, W409, E439.

In one embodiment, the binding agent comprises the substitution E430G and any one, two, three or all four of the following residues: D351, K356, K366, E439.

In one embodiment, the binding agent comprises the substitution E430G and any one, two, three, four or all five of the following residues: K356, D392, V399, W409, E439.

In one embodiment, the binding agent comprises the substitution E430G and any one, two or all three of the following residues: K351, K399, D409.

In one embodiment, the binding agent comprises the substitution E430G and any one, two, three, four, five or all six of the following residues: K356, W366, D392, K399, A407, D409.

In one embodiment, the binding agent comprises the substitution E430G and any one, two, three or all four of the following residues: K347, E360, V399, W409.

In one embodiment, the binding agent comprises the substitution E430G and any one, two, three or all four of the following residues: K347, D351, E360, K366.

In one embodiment, the binding agent comprises the substitution E430G and any one, two, three or all four of the following residues: Q357, K364, D368, S370.

In one embodiment, the binding agent comprises the substitution E430G and any one, two, three or all four of the following residues: W364, A370, D392, K399.

In one embodiment, the binding agent comprises the substitution E430G and any one, two, three, four or all five of the following residues: D351, K366, D392, K399, D409.

In one embodiment, the binding agent comprises the substitution E430G and any one, two, three, four, five or all six of the following residues: R 347, D360, V366, M399, A407, V409.

In one embodiment, the binding agent comprises the substitution E430G and any one, two, three, four, five, six or all seven of the following residues: V350, Y351, L366, L392, W394, A405, V407.

In one embodiment, the binding agent comprises the substitution E430G and any one, two, three, four, five, six or all seven of the following residues: K347, E351, K357, E360, K364, D368, E370.

In one embodiment, the binding agent comprises the substitution E345R and any one, two, three, four or all five of the following residues: R347, E360, V399, T405, W409.

In one embodiment, the binding agent comprises the substitution E345R and any one, two, three, or all four of the following residues: K356, X392, K399, D409, wherein X is D or E .

In one embodiment, the binding agent comprises the substitution E345R and any one, two, three or all four or the following residues: D351, K357, K366, E370.

In one embodiment, the binding agent comprises the substitution E345R and any one, two, three, four, five or all six of the following residues: D351, K357, K366, E370, K347, E360.

In one embodiment, the binding agent comprises the substitution E345R and the residues K356 and E439.

In one embodiment, the binding agent comprises the substitution E345R and any one, two, three or all four of the following residues: K356, V399, W409, E439.

In one embodiment, the binding agent comprises the substitution E345R and any one, two, three or all four of the following residues: D351, K356, K366, E439.

In one embodiment, the binding agent comprises the substitution E345R and any one, two, three, four or all five of the following residues: K356, D392, V399, W409, E439.

In one embodiment, the binding agent comprises the substitution E345R and any one, two or all three of the following residues: K351, K399, D409.

In one embodiment, the binding agent comprises the substitution E345R and any one, two, three, four, five or all six of the following residues: K356, W366, D392, K399, A407, D409.

In one embodiment, the binding agent comprises the substitution E345R and any one, two, three or all four of the following residues: K347, E360, V399, W409.

In one embodiment, the binding agent comprises the substitution E345R and any one, two, three or all four of the following residues: K347, D351, E360, K366.

In one embodiment, the binding agent comprises the substitution E345R and any one, two, three or all four of the following residues: Q357, K364, D368, S370.

In one embodiment, the binding agent comprises the substitution E345R and any one, two, three or all four of the following residues: W364, A370, D392, K399.

In one embodiment, the binding agent comprises the substitution E345R and any one, two, three, four or all five of the following residues: D351, K366, D392, K399, D409.

In one embodiment, the binding agent comprises the substitution E345R and any one, two, three, four, five or all six of the following residues: R 347, D360, V366, M399, A407, V409.

In one embodiment, the binding agent comprises the substitution E345R and any one, two, three, four, five, six or all seven of the following residues: V350, Y351, L366, L392, W394, A405, V407.

In one embodiment, the binding agent comprises the substitution E345R and any one, two, three, four, five, six or all seven of the following residues: K347, E351, K357, E360, K364, D368, E370.

In one embodiment, the binding agent comprises the substitution E345K and any one, two, three, four or all five of the following residues: R347, E360, V399, T405, W409.

In one embodiment, the binding agent comprises the substitution E345K and any one, two, three, or all four of the following residues: K356, X392, K399, D409, wherein X is D or E .

In one embodiment, the binding agent comprises the substitution E345K and any one, two, three or all four or the following residues: D351, K357, K366, E370.

In one embodiment, the binding agent comprises the substitution E345K and any one, two, three, four, five or all six of the following residues: D351, K357, K366, E370, K347, E360.

In one embodiment, the binding agent comprises the substitution E345K and the residues K356 and E439.

In one embodiment, the binding agent comprises the substitution E345K and any one, two, three or all four of the following residues: K356, V399, W409, E439.

In one embodiment, the binding agent comprises the substitution E345K and any one, two, three or all four of the following residues: D351, K356, K366, E439.

In one embodiment, the binding agent comprises the substitution E345K and any one, two, three, four or all five of the following residues: K356, D392, V399, W409, E439.

In one embodiment, the binding agent comprises the substitution E345K and any one, two or all three of the following residues: K351, K399, D409.

In one embodiment, the binding agent comprises the substitution E345K and any one, two, three, four, five or all six of the following residues: K356, W366, D392, K399, A407, D409.

In one embodiment, the binding agent comprises the substitution E345K and any one, two, three or all four of the following residues: K347, E360, V399, W409.

In one embodiment, the binding agent comprises the substitution E345K and any one, two, three or all four of the following residues: K347, D351, E360, K366.

In one embodiment, the binding agent comprises the substitution E345K and any one, two, three or all four of the following residues: Q357, K364, D368, S370.

In one embodiment, the binding agent comprises the substitution E345K and any one, two, three or all four of the following residues: W364, A370, D392, K399.

In one embodiment, the binding agent comprises the substitution E345K and any one, two, three, four or all five of the following residues: D351, K366, D392, K399, D409.

In one embodiment, the binding agent comprises the substitution E345K and any one, two, three, four, five or all six of the following residues: R 347, D360, V366, M399, A407, V409.

In one embodiment, the binding agent comprises the substitution E345K and any one, two, three, four, five, six or all seven of the following residues: V350, Y351, L366, L392, W394, A405, V407.

In one embodiment, the binding agent comprises the substitution E345K and any one, two, three, four, five, six or all seven of the following residues: K347, E351, K357, E360, K364, D368, E370.

In some embodiments, the binding agent is capable of forming a hexamer.

In some embodiments, the binding agent is capable of inducing complement activation, for example the binding agent has increased capacity for inducing complement activation, e.g. when compared to a parent binding agent which is identical except that it has the wild-type amino acid residue(s) at the specified position(s), such as a binding agent with a WT CH3 region.

In some embodiments, the binding agent is capable of inducing CDC, for example the binding agent has increased capacity for CDC activation, e.g. when compared to a parent binding agent which is identical except that it has the wild-type amino acid residue(s) at the specified position(s), such as a binding agent with a WT CH3 region.

In one embodiment, the binding agent comprises a CH2 domain and further comprises a mutation that reduces or eliminates Fc-mediated effector functions, optionally wherein one or more amino acids in the positions corresponding to positions 236, 237, 234, 235, 265, 297, 329 and 331 are not G, G, L, L, D, N, P and P, respectively, wherein the numbering is according to the EU index.

In one embodiment the binding agent comprises a CH2 domain and wherein the positions corresponding to positions 234, 235, and 236 are F, E, and R (L234F-L235E-G236R [FER]), respectively, and/or wherein the positions corresponding to positions 234, 235, and 265 are F, E, and A (L234F-L235E-D265A [FEA]), respectively, and/or wherein the positions corresponding to positions 236, 237, 234, 235, and 329 are R, A, A, A, and G, respectively, and/or wherein the position corresponding to position 329 is R, wherein the numbering is according to the EU index.

In one embodiment, the binding agent comprises a CH2 domain and further comprises glycan modifications that modulate Fc-mediated effector functions, such as galactosylation, biantennary glycans or afucosylated glycans.

In one embodiment, the binding agent comprises a CH2 domain and:

a) the positions corresponding to 298, 333 and 334 are A, A and A, respectively;
b) the positions corresponding to 239 and 332 are D and E, respectively;
c) the positions corresponding to 239, 330 and 332 are D, L and E, respectively;
d) the positions corresponding to 236, 239 and 332 are A, D and E, respectively;
e) the positions corresponding to 267 and 328 are E and F, respectively;
f) the positions corresponding to 243, 292, 300, 305 and 396 are L, P, L, I and L, respectively;
g) the positions corresponding to 235, 243, 292, 300 and 396 are V, L, P, L and L, respectively; or
h) the CH2 and CH3 domains are dimers and the positions corresponding to 234, 235, 236, 239, 268, 270 and 298 are Y, Q, W, M, D, E and A in one monomer and the positions corresponding to 270, 326, 330 and 334 are E, D, M and E, respectively in the other monomer,

wherein the numbering is according to the EU index.

In yet another embodiment, the binding agent comprises one or more mutations, for example in the Fc region, that prolong IgG (e.g. IgG1) half-life.

Thus, in one embodiment, the binding agent comprises a CH3 region wherein:

a) the position corresponding to 435 is H;
b) the position corresponding to 434 is A; and/or
c) the positions corresponding to 428 and 434 are Land S, respectively,

wherein the numbering is according to the EU index.

In a further embodiment, the binding agent further comprises a CH2 domain and wherein

a) the positions corresponding to 252, 254, and 256 are Y, T and E respectively;
b) the positions corresponding to 252, 253 and 254 are L, S and F, respectively;
c) the position corresponding to 294 is deleted;
d) the position corresponding to 294 is deleted and the positions corresponding to 307 and 434 are P and T, respectively; and/or
e) the positions corresponding to 256, 378, 383 and 434 are N, V, N and Y, respectively,

wherein the numbering is according to the EU index.

In yet another embodiment, the binding agent further comprises one or more mutations in the Fc region that inhibit self-oligomerization, such as wherein the amino acid in the position corresponding to position K439 as in a human IgG1 heavy chain (EU numbering) is E (E439) and/or wherein the amino acid in the position corresponding to S440 as in a human IgG1 heavy chain (EU numbering) is K (K440) (HexElect®; WO2019211472). Corresponding positions in other isotypes can be determined by the skilled person, e.g. by sequence alignment.

In yet another embodiment, the binding agent further comprises one or more mutations in the Fc region that combine two or more of the above strategies, for example wherein in the amino acids at the positions corresponding to positions E345/E430 and K439 as in a human IgG1 heavy chain (EU numbering) are R/G and E, respectively (R345-E439 [RE] or G430-E439 [GE]), or wherein the amino acids at the positions corresponding to positions E345/E430, and S440 as in a human IgG1 heavy chain (EU numbering) are R/G and K , respectively (R345-K440 [RK] or G430-K440 [GK]). Corresponding positions in other isotypes can be determined by the skilled person, e.g. by sequence alignment.

In yet another embodiment, the binding agent further comprises one or more mutations in the Fc region, such as wherein the positions corresponding to positions G236, E345/E430, K439 in a human IgG1 heavy chain (EU numbering) are R, R/G and E, respectively (R236-R345-E439 [RRE] or R236-G430-E439 [RGE]), or wherein in the positions corresponding to positions G237, E345/E430, and S440 in a human IgG1 heavy chain (EU numbering) are A, R and K, respectively (A237-R345-K440 [ARK] or A237-G430-K440 [AGK]).

In an even further embodiment, the binding agent is a stable half antibody molecule which does not form dimers. In one embodiment the binding agent is a UniBody antibody. In one embodiment, the binding agent is a stable half IgG4 antibody comprising T405-E407, and wherein (optionally) the amino acid sequence ESKYGPPCPSCP of the hinge has been deleted [E].

In one embodiment, the binding agent is a monospecific antibody, such as a bivalent monospecific antibody.

In other embodiment, the binding agent is a multispecific antibody, such as a bispecific antibody.

Multispecific antibodies, such as bispecific antibodies may comprise mutations to promote the formation of heterodimers rather than homodimers, e.g. using the *DuoBody*® technology.

In one embodiment, the binding agent comprises a dimeric CH3 domain consisting of a first and second CH3 monomer polypeptide, wherein in said first CH3 monomer polypeptide at least one of the amino acids in a position corresponding to a position selected from the group consisting of 366, 368, 370, 399, 405, 407, and 409 has been substituted, and in said second CH3 monomer polypeptide at least one of the amino acids in a position corresponding to a position selected from the group consisting of 366, 368, 370, 399, 405, 407, and 409 has been substituted, and wherein said first and said second CH3 monomer polypeptides are not substituted in the same positions, and wherein the numbering is according to the EU index.

In one embodiment, the binding agent is a binding agent as described herein not comprising any of the amino acid residues A405, T405, D409, W409, V409, wherein the binding agent comprises a dimeric CH3 domain consisting of a first and second CH3 monomer polypeptide, wherein (i) the amino acid in the position corresponding to 405 is L in said first CH3 monomer polypeptide, and the amino acid in the position corresponding to 409 is R in said second CH3 monomer polypeptide, or (ii) the amino acid in the position corresponding to 409 is R in said first CH3 monomer polypeptide, and the amino acid in the position corresponding to 405 is L in said second CH3 monomer polypeptide, wherein the numbering is according to the EU index.

A bispecific antibody may have a common light chain, capable of forming functional dimers with both heavy chains.

In another embodiment, the light chain may comprise one or more mutations to create an orthogonal heavy-light chain interface that allows for correct heavy and light chain pairing. Examples of such light chain mutations include D1R-Q38D-L135Y-S176W or Q38R, which can respectively be combined with heavy chain mutations Q39K-R62E-H172A-F174G or Q39Y (Kabat numbering). The light chain may also comprise D1R-Q38D-E123K-S131R or Q38R-K42D or D1R-Q38D-E123K-S131R-S176I or D1R-Q38D-S131R-S176I, which can be respectively combined with heavy chain mutations Q39K-R62E-K145A-K221E or Q39Y-Q105R or Q39K-R62E-K145A-S188G-K221E or Q39K-R62E-K145A-S188A (Kabat numbering;). The light chain may also comprise E123K-Q124K or E123K-Q124R which can be combined with heavy chain mutations K147E-K213E or K147D-K213D (EU numbering). The light chain may also comprise the mutations S131K-Q160C-S162C-C214S or Q160C-S162C-S174K-C214S or S131K-Q160C-S162C-S174K-C214S, which can be combined with heavy chain mutations K147D-F170C-V173C-C220G or H168D-F170C-V173C-C220S or K147D-H168D-F170C-V173C-C220S (EU numbering). The light chain may also comprise S121C-C214S or S121C-C214G or F116C-C220G or T164C-C220G, which can be combined with heavy chain mutations F126C-C220S or F126C-C220G or A141C-C220G or H168C-C220G (EU numbering). The light chain may also comprise Q38K-V133E or Q38E-V133K or Q38K-F116A-L135V-S174A-S176F-T178V, which can be combined with heavy chain mutations Q39E-S183K or Q39K-S183E or Q39E-A141I-F170S-S181M-S183A-V185A -(Kabat numbering for V domains and EU numbering for C domains). The light chain may also comprise Q38K-A43K-S176D or Q38D-A43D-S176K, which can be combined with heavy chain mutations Q39D-Q105D-S183K or Q39K-Q105K-S183D (Kabat numbering for V domains and EU numbering for C domains). The light chain may also comprise F118V or T129R, which can be respectively combined with heavy chain mutations A141L or K147D -(EU numbering). The light chain may also comprise S131E-T180E or S131K-T180K or S131E-Q160E or S131K-E160K, which can be combined with heavy chain mutations K147-Q175K or K147E-Q175E -(Kabat numbering for V domains and EU numbering for C domains). The light chain may also Q38R-L135Y-S176W, which can be combined with heavy chain mutations Q39Y-H168A-F170G. (Kabat numbering for V domains and EU numbering for C domains),

In another embodiment, the light chain may comprise a domain crossover, for example a replacement of a VL domain with a VH domain and/ or a CL domain with a CH1 domain, which may also contain VH-VL and/or CH1-CL interface mutations.

In other embodiment, the binding agent is a multispecific antibody, such as a bi- or trispecific antibody, comprising a full-length antibody and further comprising a third antigen-binding region attached to one of the antigen-binding regions of the full-length antibody via a cleavable or non-cleavable linker.

In addition to the modifications mentioned above, the CH3 domain or Fc region or constant region may contain further mutations, deletions or insertions for modulation of pharmacokinetic properties (e.g. increasing half-life) and/or the improvement of drug development properties (e.g. reduce aggregation propensity, increase solubility or reduce heterogeneity).

In one embodiment, the binding agent comprises a C-terminal Lys (K).

In another embodiment, the binding agent does not comprise a C-terminal Lys (K).

In a further embodiment, the binding agent comprises a cytotoxic agent or a precursor thereof, an isotope or a label. Thus, in some embodiments, the binding agent is an antibody-drug conjugate (ADC).

The binding agent may have a modified capacity to induce the activation of one or more different effector molecules (e.g. Fc receptors and complement components)through allosteric modulation of the effector binding site relative to a comparator binding agent which is identical except that it has the wild-type amino acid residue(s) at the specified position(s). These Fc receptors include e.g. FcgammaRIIa-H and/or FcgammaRIIIa-V and/or FcgammaRIIb and/or FcRn. Complement components include e.g. those as described above, such as C1q and C4d.

The binding agent of the invention typically has an altered capacity to activate Fc-mediated effector functions relative to a comparator binding agent which is identical except that it has the wild-type amino acid residue(s) at the specified position(s).

In one such embodiment, the altered capacity comprises an increased capacity to activate complement-dependent cytotoxicity, such as an at least 20% increased capacity to activate complement-dependent cytotoxicity, on target-expressing cells, such as Raji or Daudi cells, for example when assayed by determining the viability of the cells after incubation with human serum and binding agent, for example as described in Example 8 herein. In further embodiments hereof, the capacity to activate complement-dependent cytotoxicity is increased with at least 25%, at least 30%, at least 40%, at least 50%, at least 75%, at least 100%, at least 150% or at least 200%.

Alternatively, or in addition, the altered capacity comprises an increased capacity to activate FcgammaRIIa-H and/or FcgammaRIIIa-V, such as an at least 20% increased capacity to activate FcgammaRIIa-H and/or FcgammaRIIIa-V, for example when assayed by measuring bioluminescence after incubation of the binding agent with target cells expressing the target of the binding agent and effector cells expressing the specific FcgammaR and expressing a NFAT-induced luciferase reporter, for example as described in Example 9 herein. In further embodiments hereof, the capacity to activate FcgammaRIIa-H and/or FcgammaRIIIa-V is increased with at least 25%, at least 30%, at least 40%, at least 50%, at least 75%, at least 100%, at least 150% or at least 200%.

In another embodiment, the altered capacity comprises an increased capacity to activate FcgammaRIIa-H and/or FcgammaRIIIa-V, such as an at least 20% increased capacity to activate FcgammaRIIa-H and/or FcgammaRIIIa-V, for example when assayed by measuring bioluminescence after incubation of the binding agent with target cells expressing the target of the binding agent and effector cells expressing the specific FcgammaR and expressing a NFAT-induced luciferase reporter (for example as described in Example 9 herein), and wherein the capacity to activate complement-dependent cytotoxicity is unaltered or reduced relative to a comparator binding agent which is identical except that it has the wild-type amino acid residue(s) at the specified position(s) (for example as described in Example 8 herein). In further embodiments hereof, the capacity to activate FcgammaRIIa-H and/or FcgammaRIIIa-V is increased with at least 25%, at least 30%, at least 40%, at least 50%, at least 75%, at least 100%, at least 150% or at least 200%.

In another embodiment, the altered capacity comprises an increased capacity to activate complement-dependent cytotoxicity, such as an at least 20% increased capacity to activate complement-dependent cytotoxicity on target-expressing cells, such as Raji or Daudi cells, for examples when assayed by determining the viability of the cells after incubation with human serum and binding agent (for example as described in Example 8 herein) and wherein the capacity to activate FcgammaRIIa-H and/or FcgammaRIIIa-V is unaltered or reduced relative to a comparator binding agent which is identical except that it has the wild-type amino acid residue(s) at the specified position(s) (for example as described in Example 9 herein). In further embodiments hereof, the capacity to activate complement-dependent cytotoxicity is increased with at least 25%, at least 30%, at least 40%, at least 50%, at least 75%, at least 100%, at least 150% or at least 200%.

Alternatively, or in addition, the altered capacity comprises an increased capacity to activate/bind to FcRn, such as an at least 20% increased capacity to bind/activate FcRn, for example when assayed as described in Example 10 herein. In further embodiments hereof, the capacity to bind to FcRn, is increased with at least 25%, at least 30%, at least 40%, at least 50%, at least 75%, at least 100%, at least 150% or at least 200%.

In a further aspect, the invention relates to a method for altering one or more effector functions of a parent binding agent comprising a CH3 domain of a human IgG and a target-binding region, which method comprises introducing one or more mutations in the CH3 domain, such as introducing one or more of the following amino acids into the parent binding agent:

a) R347 or K347;
b) V350;
c) K351, Y351, D351 or E351;
d) K356;
e) Q357 or K357;
f) E360 or D360;
g) K364 or W364;
h) W366, L366, K366 or V366;
i) D368;
j) S370, E370 or A370;
k) D392, L392 or E392;
l) W394;

m) K399, V399 or M399;

n) A405 or T405;

o) A407 or V407;

p) D409, W409 or V409; or

q) E439, wherein the numbering is according to the EU index and wherein the binding agent has an altered effector function relative to a comparator binding agent which is identical except that it has the wild-type amino acid residue(s) at the specified position(s).

Also provided is a method for allosteric modulation of one or more effector binding sites of a binding agent parent binding agent comprising a CH3 domain of a human IgG and a target-binding region, which method comprises introducing one or more mutations into the CH3 domain, such as introducing one or more of the above amino acids a) to q) and into the parent binding agent.

Also provided a method for producing a binding agent having one or more altered effector functions, said binding agent comprising a CH3 domain of a human IgG and a target-binding region, which method comprises introducing one or more mutations into the CH3 domain, such as introducing one or more of the above amino acids a) to q) into a parent binding agent.

Also provided is a method for producing a binding agent that has one or more allosterically modulated effector binding sites, said binding agent comprising a CH3 domain of a human IgG and a target-binding region, wherein said method comprises introducing one or more of the above amino acids a) to q) into a parent binding agent.

The effector functions may be any of the effector functions as described herein, such as capacity for FcgammaRIIb activation, capacity for FcgammaRIIIa-V activation, capacity for FcgammaRIIa-H activation, capacity for FcRn binding/activation, capacity for complement activation (binding and/or activation of complement components), and/or capacity for CDC activation.

These methods may further involve introducing one or more further mutations known to be involved in effector function modulation, as described elsewhere herein, such as mutation(s) known to be involved in the same or in different effector functions.

Said methods may contain any one or more of the further features described for the previous aspect.

### *Polynucleotides, host cells and methods for production*

The invention further relates to a polynucleotide construct, or a kit of polynucleotide constructs, encoding a binding agent according to the invention. Such a polynucleotide constructs may, e.g., be used for *in vitro, ex vivo, in situ* or *in vivo* expression of the binding agent, for example the production of the binding agent in a host cell or for administration to a patient for *in vivo* expression.

In order to optimize expression of the binding agent in the cell, the polynucleotide sequence encoding the binding agent may be flanked by a 5' UTR sequence and/or 3' UTR sequence. The 5'UTR and/or 3' UTR may be heterologous with respect to the polynucleotide sequence encoding the binding agent. A UTR or "untranslated region", as used herein refers to a part of a polynucleotide sequence preceding (5' UTR) or following (3' UTR) a coding sequence, such as the polynucleotide sequence encoding the polypeptide, and that is thus not part of the coding sequence and typically not translated. An UTR may be part of a nucleic acid and may comprise elements for controlling gene expression.

Further, the polynucleotide sequence encoding the binding agent (the coding sequence or open reading frame "ORF") may also be optimized for expression in the cell or organism where expression of the binding agent is desired, e.g. in mammalian or avian cells as indicated above, as long as this does not change the amino acid sequence of the encoded binding agent. For example, the codon usage may be adapted toward the preferred codon usage of a particular organism or edited to remove potential unwanted elements, such as splice sites, destabilizing motifs or improve the GC content/index, according to any method known in the art.

The formation of an antibody, such as a full-length antibody may require the co-expression of light chain constant and variable domains corresponding to those that are normally found in a wild-type antibody of that isotype.

The (isolated) polynucleotide may further comprise an operably-linked promoter sequence capable of driving expression of the operably-linked coding sequence in the target cell or organism or system (e.g., in vitro system) of interest.

As will be appreciated by the skilled person, for any of these operable elements, these are to be chosen so as to be functional in the cell or organism where expression of the binding agent is desired (target cell or target organism). In other words, they are suitable for expression in a certain cell type or organism, such as mammalian cells (e.g. human, primates, mouse, rabbit, camelid, and rats) or avian cells, e.g. chicken. They may also be optimized for enhanced protein expression in the target cell or target organism.

For example, the (isolated) polynucleotide(s) may be provided in the form of one or more vectors, such as expression vectors. In expression vectors, the polynucleotide sequence encoding the binding agent is typically operably linked to a promoter sequence suitable for expression in a target cell (host cell) or system. Thus, in one embodiment, the promoter sequence is a mammalian promoter sequence, such as a human promoter sequence or a viral promoter sequence. In a further embodiment, wherein said promoter sequence is a bacteriophage promoter sequence or a bacterial promoter sequence.

One or more of the (isolated) polynucleotides may be viral vectors. In one embodiment, one or more of the (isolated) polynucleotides is a viral vector selected from the group consisting of an adenovirus-based vector, an adeno-associated virus (AAV) based vector, a retroviral vector, a vaccinia virus-based vector, a modified vaccinia virus Ankara (MVA) based vector, a herpes virus-based vector, a lentiviral vector, or a poxvirus vector.

In a further aspect, the invention relates to a cell, such as a recombinant host cell, capable of producing a binding agent according to the invention. Such a cell may for comprise polynucleotide constructs as described herein. In one embodiment, the cell is a mammalian cell, such as a human cell. In one embodiment, the cell is a mammalian cell or cell line, such as a CHO cell line or human epithelial cell, for example a HEK293 cell line. In one embodiment, the polynucleotide is present in episomal form. In another embodiment, the polynucleotide is integrated into the host genome. The skilled person will be aware of methods to provide the polynucleotides(s) encoding the binding agent according to the invention to the cell, e.g. by transfection or transformation, so as to obtain a transgenic or recombinant cell.

In a further aspect, the invention relates to a method for producing a binding agent, comprising culturing recombinant host cells according to the invention and allowing production of the binding agent to take place.

### *Pharmaceutical compositions*

In a further aspect, the present invention provides a pharmaceutical composition comprising the binding agent, the polynucleotide or cell according to the invention and a pharmaceutically-acceptable carrier and/or excipient. The pharmaceutical composition is typically formulated for parenteral delivery to a human patient. Carriers and/or excipients for antibodies and polynucleotides are well-known in the art.

Polynucleotides may for example be in a lipid formulation, composed of constituents such as lipids, phospholipids, cholesterol, PEGylated lipids, and cationic or ionizable lipids or any combination thereof. The polynucleotides may be encapsulated in a delivery vehicle intended to protect said polynucleotides. Such a delivery vehicle may be modified in such a way that delivery to the relevant tissue is optimally achieved, e.g. by external attachment of target-specific antibodies, vehicle size adjustment or by means of modulating physicochemical properties of the vehicle. The composition may be formulated for the introduction in a proper host cell for *in situ* expression of the binding agent. In one embodiment, the cell is a mammalian cell, such as a human cell.

### Medical use

One aspect of the present invention provides the binding agent, polynucleotide(s) or host cell according to the present invention for use as a medicament.

Similarly, there is provided a method for the treatment of a disease or disorder, comprising administration, to a subject in need thereof, of a binding agent, polynucleotide(s) or host cell according to the present invention, for example in the form of a pharmaceutical composition.

In one aspect, the invention relates to the use of a binding agent, polynucleotide(s) or host cell according to the present invention as described herein in the preparation of a medicament for treating or preventing or ameliorating a disease or disorder.

In one aspect, the present invention relates to a method of treating or ameliorating a disease or disorder, comprising the steps of:

- selecting a subject suffering from the disease or disorder, and

- administering to the subject the the binding agent, polynucleotide(s) or host cell according to the present invention, or a pharmaceutical composition comprising the the binding agent, polynucleotide(s) or host cell according to the present invention, typically in a therapeutically effective amount and/or for a time sufficient to treat or ameliorate the disease or disorder.

In one embodiment, the disease or disorder is cancer, i.e. a tumorigenic disorder, such as for example, a hematological cancer or a solid tumor malignancy. In another embodiment the disease or disorder is an inflammatory and/or autoimmune disease or disorder. In a further embodiment, the disease is an infectious disease.

As mentioned, the binding agent, polynucleotide(s) or host cell according to the present invention or pharmaceutical composition of the present invention may be administered in combination therapy, i.e., combined with other therapeutic agents relevant for the disease or condition to be treated.

Each reference cited herein, is hereby specifically incorporated by reference in its entirety.

**Clauses**

1) A binding agent comprising at least CH3 domain of a human IgG and a target-binding region, wherein said CH3 domain comprises at least one of the following amino acid residues:

    a. K356;
    b. R347 or K347;
    c. V350;
    d. K351, Y351, D351 or E351;
    e. Q357 or K357;
    f. E360 or D360;
    g. K364 or W364;
    h. W366, L366, K366 or V366;
    i. D368;
    j. S370, E370 or A370;
    k. D392, L392 or E392;
    l. W394;
    m. K399, V399 or M399;
    n. A405 or T405;
    o. A407 or V407;
    p. D409, W409 or V409; or
    q. E439,

wherein the numbering is according to the EU index.

2) The binding agent according to clause 1, wherein said CH3 domain comprises any two, three, four or all five of the following residues: R347, E360, V399, T405, W409.

3) The binding agent according to clause 1, wherein said CH3 domain comprises any two, three or all four of the following residues: K356, X392, K399, D409, wherein X is D or E.

4) The binding agent according to clause 1, wherein said CH3 domain comprises any two, three or all four of the following residues: D351, K357, K366, E370.

5) The binding agent according to clause 1 or 4, wherein said CH3 domain comprises K347 and/or E360.

6) The binding agent according to clause 1, wherein said CH3 domain comprises K356 and E439.

7) The binding agent according to clause 1 or 6, wherein said CH3 domain comprises

    a. any two, three or all four of the following residues: K356, V399, W409, E439; or
    b. any two, three or all four of the following residues: D351, K356, K366, E439; or
    c. any two, three, four or all five of the following residues: K356, D392, V399, W409, E439.

8) The binding agent according to clause 1, wherein said CH3 domain comprises K399 and D409.

9) The binding agent according to clause 1 or 8, wherein said CH3 domain comprises

    a. any two or all three of the following residues: K351, K399, D409;
    b. any two, three, four or all five of the following residues: D351, K366, D392, K399, D409; or
    c. any two, three, four, five or all six of the following residues: K356, W366, D392, K399, A407, D409.

10) The binding agent according to clause 1, wherein said CH3 domain comprises K347 and E360.

11) The binding agent according to clause 1, wherein said CH3 domain comprises D351 and K366.

12) The binding agent according to clause 1, 10 or 11, wherein said CH3 domain comprises

a. any two, three or all four of the following residues: K347, E360, V399, W409; or
b. any two, three or all four of the following residues: K347, D351, E360, K366.

13) The binding agent according to clause 1, wherein said CH3 domain comprises

a. any two, three or all four of the following residues: Q357, K364, D368, S370; or
b. any two, three or all four of the following residues: W364, A370, D392, K399; or
c. any two, three, four, five or all six of the following residues: R347, D360, V366, M399, A407, V409 and optionally R345; or
d. any two, three, four, five, six or all seven of the following residues: V350, Y351, L366, L392, W394, A405, V407; or
e. any two, three, four, five, six or all seven of the following residues: K347, E351, K357, E360, K364, D368, E370.

14) The binding agent according to any one of the preceding clauses, wherein said CH3 domain is dimeric and consists of two CH3 monomer polypeptides, wherein both CH3 monomer polypeptides contain the specified amino acid residues at the specified positions.

15) The binding agent according to any one of the preceding clauses, wherein the binding agent is an antibody and the target-binding region is an antigen-binding region.

16) The binding agent according to any one of the preceding clauses, wherein the CH3 domain is of the IgG1, IgG2, IgG3 or IgG4 subclass.

17) The binding agent according to any one of the preceding clauses, wherein the binding agent further comprises a CH2 domain, preferably of the same isotype as the CH3 domain.

18) The binding agent according to any one of the preceding clauses, wherein the binding agent further comprises a hinge region.

19) The binding agent according to any one of the preceding clauses, wherein the binding agent comprises a full Fc region.

20) The binding agent according to any one of the preceding clauses, wherein the binding agent is a full-length antibody.

21) The binding agent according to any one of the preceding clauses, wherein the binding agent comprises one or more further mutations, for example in the Fc region, such as in the CH3 domain.

22) The binding agent according to any one of the preceding clauses, wherein the binding agent comprises a mutation that enhances Fc:Fc interactions between individual binding agent molecules, optionally where said mutation is a substitution selected from the group consisting of E430G, E345R and E345K, wherein the numbering is according to the EU index.

23) The binding agent according to any one of the preceding clauses, wherein the binding agent is capable of forming a hexamer.

24) The binding agent according to any one of the preceding clauses, wherein the binding agent comprises a CH2 domain and further comprises a mutation that reduces or eliminates Fc-mediated effector functions, optionally wherein one or more amino acids in the positions corresponding to positions 236, 237, 234, 235, 265, 297, 329 and 331 are not G, G, L, L, D, N, P and P, respectively, wherein the numbering is according to the EU index.

25) The binding agent according to any one of the preceding clauses, wherein the binding agent comprises a CH2 domain and further comprises glycan modifications that modulate Fc-mediated effector functions, such as galactosylation, biantennary glycans or afucosylated glycans.

26) The binding agent according to any one of the preceding clauses, wherein the binding agent comprises a CH2 domain and wherein the positions corresponding to positions 234, 235, and 236 are F, E, and R, respectively, and/or wherein the positions corresponding to positions 234, 235, and 265 are F, E, and A, respectively, and/or wherein the positions corresponding to positions 236, 237, 234, 235, and 329 are R, A, A, A, and G, respectively, and/or wherein the position corresponding to position 329 is R, wherein the numbering is according to the EU index.

27) The binding agent according to any one of the preceding clauses, wherein the binding agent comprises a CH2 domain and wherein:

    a. the positions corresponding to 298, 333 and 334 are A, A and A, respectively;
    b. the positions corresponding to 239 and 332 are D and E, respectively;
    c. the positions corresponding to 239, 330 and 332 are D, L and E, respectively;
    d. the positions corresponding to 236, 239 and 332 are A, D and E, respectively;
    e. the positions corresponding to 267 and 328 are E and F, respectively;
    f. the positions corresponding to 243, 292, 300, 305 and 396 are L, P, L, I and L, respectively;
    g. the positions corresponding to 235, 243, 292, 300 and 396 are V, L, P, L and L, respectively; or
    h. the CH2 and CH3 domains are dimers and the positions corresponding to 234, 235, 236, 239, 268, 270 and 298 are Y, Q, W, M, D, E and A in one monomer and the positions corresponding to 270, 326, 330 and 334 are E, D, M and E, respectively in the other monomer,

wherein the numbering is according to the EU index.

28) The binding agent according to any one of the preceding clauses, wherein:

    a. the position corresponding to 435 is H;
    b. the position corresponding to 434 is A; and/or
    c. the positions corresponding to 428 and 434 are L and S, respectively,

wherein the numbering is according to the EU index.

29) The binding agent according to any one of the preceding clauses, wherein the binding agent comprises a CH2 domain and wherein

    a. the positions corresponding to 252, 254, and 256 are Y, T and E respectively;
    b. the positions corresponding to 252, 253 and 254 are L, S and F, respectively;
    c. the position corresponding to 294 is deleted;
    d. the position corresponding to 294 is deleted and the positions corresponding to 307 and 434 are P and T, respectively; and/or
    e. the positions corresponding to 256, 378, 383 and 434 are N, V, N and Y, respectively,

wherein the numbering is according to the EU index.

30) The binding agent according to any one of the preceding clauses, wherein the position corresponding to 439 is E, and/or wherein the position corresponding to 440 is K, optionally wherein the positions corresponding to positions 236, 345/430, 439 in a human IgG1 heavy chain are R, R/G and E, respectively, or wherein in the positions corresponding to positions 237, 345/430, and 440 in a human IgG1 heavy chain are A, R and K, respectively, wherein the numbering is according to the EU index.

31) The binding agent according to any one of the preceding clauses, wherein the binding agent is a monospecific antibody.

32) The binding agent according to any one of clauses 1 to 30, wherein the binding agent is a multispecific antibody, such as a bispecific antibody, wherein optionally the antibody is a full-length antibody further comprising a third antigen-binding region attached to one of the antigen-binding regions of the full-length antibody, optionally via a cleavable or non-cleavable linker.

33) The binding agent according to any one of the preceding clauses, wherein the binding agent comprises a dimeric CH3 domain consisting of a first and second CH3 monomer polypeptide, wherein in said first CH3 monomer polypeptide at least one of the amino acids in a position corresponding to a position selected from the group consisting of 366, 368, 370, 399, 405, 407, and 409 has been substituted, and in said second CH3 monomer polypeptide at least one of the amino acids in a position corresponding to a position selected from the group consisting of 366, 368, 370, 399, 405, 407, and 409 has been substituted, and wherein said first and said second CH3 monomer polypeptides are not substituted in the same positions, and wherein the numbering is according to the EU index.

34) A binding agent according to any one of the preceding clauses not comprising any of the amino acid residues A405, T405, D409, W409, V409, wherein the binding agent comprises a dimeric CH3 domain consisting of a first and second CH3 monomer polypeptide, wherein (i) the amino acid in the position corresponding to 405 is L in said first CH3 monomer polypeptide, and the amino acid in the position corresponding to 409 is R in said second CH3 monomer polypeptide, or (ii) the amino acid in the position corresponding to 409 is R in said first CH3 monomer polypeptide, and the amino acid in the position corresponding to 405 is L in said second CH3 monomer polypeptide, wherein the numbering is according to the EU index.

35) The binding agent according to any one of the preceding clauses, wherein the binding agent comprises a cytotoxic agent or a precursor thereof, an isotope or a label.

36) The binding agent according to any one of the preceding clauses, wherein the binding agent has an altered capacity to activate Fc-mediated effector functions relative to a comparator binding agent which is identical except that it has the wild-type amino acid residue(s) at the specified position(s), optionally wherein the one or more effector functions are selected from: FcgammaRIIb activation, capacity for FcgammaRIIIa-V activation, capacity for FcgammaRIIa-H activation, capacity for FcRn binding/activation, capacity for complement activation and/or capacity for CDC activation .

37) The binding agent according to any of the preceding clauses, wherein the binding agent has an altered capacity to induce the binding to and/or activation of one or more Fc receptors and/or complement components through allosteric modulation of the effector binding site relative to a comparator binding agent which is identical except that it has the wild-type amino acid residue(s) at the specified position(s), optionally wherein the Fc receptors are selected from FcgammaRIIb, FcgammaRIIIa-V and/or FcgammaRIIa-H and/or FcRN

38) The binding agent according to clause 36 or 37, wherein the altered capacity comprises an increased capacity to activate complement-dependent cytotoxicity, such as an at least 20% increased capacity to activate complement-dependent cytotoxicity on target-expressing cells, such as Raji or Daudi cells, when assayed by determining the viability of the cells after incubation with human serum and binding agent.

39) The binding agent according to any one of clauses 36 to 38, wherein the altered capacity comprises an increased capacity to activate FcgammaRIIa-H and/or FcgammaRIIIa-V, such as an at least 20% increased capacity to activate FcgammaRIIa-H and/or FcgammaRIIIa-V, when assayed by measuring bioluminescence after incubation of the binding agent with target cells expressing the target of the binding agent and effector cells expressing the specific FcgammaR and expressing a NFAT-induced luciferase reporter.

40) The binding agent according to clause 36 or 37, wherein the altered capacity comprises an increased capacity to activate FcgammaRIIa-H and/or FcgammaRIIIa-V, such as an at least 20% increased capacity to activate FcgammaRIIa-H and/or FcgammaRIIIa-V, when assayed by measuring bioluminescence after incubation of the binding agent with target cells expressing the target of the binding agent and effector cells expressing the specific FcgammaR and expressing a NFAT-induced luciferase reporter, and wherein the capacity to activate complement-dependent cytotoxicity is unaltered or reduced relative to a comparator binding agent which is identical except that it has the wild-type amino acid residue(s) at the specified position(s).

41) The binding agent according to clause 36 or 37, wherein the altered capacity comprises an increased capacity to activate complement-dependent cytotoxicity, such as an at least 20% increased capacity to activate complement-dependent cytotoxicity on target-expressing cells, such as Raji or Daudi cells, when assayed by determining the viability of the cells after incubation with human serum and binding agent and wherein the capacity to activate FcgammaRIIa-H and/or FcgammaRIIIa-V is unaltered or reduced relative to a comparator binding agent which is identical except that it has the wild-type amino acid residue(s) at the specified position(s).

42) A polynucleotide construct, or a kit of polynucleotide constructs, encoding a binding agent according to any one of the preceding clauses.

43) A host cell capable of producing a binding agent according to any one of the preceding clauses.

44) A method for producing a binding agent, comprising culturing recombinant host cells according to clause 43 and allowing production of the binding agent to take place.

45) The binding agent, polynucleotide(s) or host cell according to the present invention for use as a medicament according to any one of clauses 1 to 43, for use as a medicament.

46) A method for the treatment of a disease or disorder, comprising administration, to a subject in need thereof, of a binding agent, polynucleotide(s) or host cell according to any one of clauses 1 to 43.

47) A pharmaceutical composition comprising the binding agent, the polynucleotide or the host cell according to any one of clauses 1 to 43 and a pharmaceutically-acceptable carrier and/or excipient.

48) A method for altering one or more effector functions or for allosteric modulation of one or more effector binding sites of a parent binding agent comprising a CH3 domain of a human IgG and a target-binding region, which method comprises introducing one or more of the following amino acids into the parent binding agent:

    a. R347 or K347;
    b. V350;
    c. K351, Y351, D351 or E351;
    d. K356;
    e. Q357 or K357;
    f. E360 or D360;
    g. K364 or W364;
    h. W366, L366, K366 or V366;
    i. D368;
    j. S370, E370 or A370;
    k. D392, L392 or E392;
    l. W394;
    m. K399, V399 or M399;
    n. A405 or T405;
    o. A407 or V407;
    p. D409, W409 or V409; or
    q. E439,

wherein the numbering is according to the EU index and wherein the binding agent has an altered effector function relative to a comparator binding agent which is identical except that it has the wild-type amino acid residue(s) at the specified position(s).

49) The method according to clause 48, comprising one or more of the further features of clauses 2 to 41.

Sequences disclosed herein

| SEQ ID NO | Name | Sequence |
|---|---|---|
| 1. | constant region human HC IgG1m(f) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKL TVDKSRWQQGNVFSCSVMHEALHNHYTQKSLSLSPG |
| 2. | IgG1 constant region human kappa LC | RTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSP VTKSFNRGEC |
| 3. | IgG1 constant region human lambda LC | GQPKAAPSVTLFPPSSEELQANKATLVCLISDFYPGAVTVAWKADSSPV KAGVETTTPSKQSNNKYAASSYLSLTPEQWKSHRSYSCQVTHEGSTVE KTVAPTECS |
| 4. | constant region human HC IgG1m(f)-E356K-K392D-D399K-K409D | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSR**K**EMTKN QVSLTCLVKGFYPSDIAVEWESNGQPENNY**D**TTPPVL**K**SDGSFFLYS**D**L TVDKSRWQQGNVFSCSVMHEALHNHYTQKSLSLSPG |
| 5. | constant region human HC IgG1m(f)-E356K-K392E-D399K-K409D | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSR**K**EMTKN QVSLTCLVKGFYPSDIAVEWESNGQPENNY**E**TTPPVL**K**SDGSFFLYS**D**L TVDKSRWQQGNVFSCSVMHEALHNHYTQKSLSLSPG |
| 6. | constant region human HC IgG1m(f)-E357Q-S364K-L368D-K370S | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSRE**Q**MTK NQV**K**LTC**D**V**S**GFYPSDIAVEWESNGQPENNYKTTPPVLDSDGSFFLYS KLTVDKSRWQQGNVFSCSVMHEALHNHYTQKSLSLSPG |

| SEQ ID NO | Name | Sequence |
|---|---|---|
| 7. | constant region human HC IgG1m(f)-T350V-L351Y-T366L-K392L-T394W-F405A-Y407V | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVY**VY**PPSREEMTK NQVSL**L**CLVKGFYPSDIAVEWESNGQPENNY**LTW**PPVLDSDGSF**A**L**VS** KLTVDKSRWQQGNVFSCSVMHEALHNHYTQKSLSLSPG |
| 8. | constant region human HC IgG1m(f)-Q347R-K360E-D399V-F405T-K409W | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREP**R**VYTLPPSREEMT**E**N QVSLTCLVKGFYPSDIAVEWESNGQPENNYKTTPPVL**V**SDGSF**T**LYS**W** LTVDKSRWQQGNVFSCSVMHEALHNHYTQKSLSLSPG |
| 9. | constant region human HC IgG1m(f)-E345R-Q347R-K360D-T366V-D399M-Y407A-K409V | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPR**RPR**VYTLPPSREEMT**D** NQVSL**V**CLVKGFYPSDIAVEWESNGQPENNYKTTPPVL**M**SDGSFFL**AS** **V**LTVDKSRWQQGNVFSCSVMHEALHNHYTQKSLSLSPG |
| 10. | constant region human HC IgG1m(f)-Q347R-K360D-T366V-D399MY407A-K409V | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREP**R**VYTLPPSREEMT**D**N QVSL**V**CLVKGFYPSDIAVEWESNGQPENNYKTTPPVL**M**SDGSFFL**ASV** LTVDKSRWQQGNVFSCSVMHEALHNHYTQKSLSLSPG |
| 11. | constant region human HC IgG1m(f)-L351K-D399K-K409D | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD |

| SEQ ID NO | Name | Sequence |
|---|---|---|
| | | VSHEDPEVKFNWYVDGVEVHNAKTKPREEQYNSTYRVVSVLTVLHQD WLNGKEYKCKVSNKALPAPIEKTISKAKGQPREPQVYT**K**PPSREEMTK NQVSLTCLVKGFYPSDIAVEWESNGQPENNYKTTPPVL**K**SDGSFFLYS**D** LTVDKSRWQQGNVFSCSVMHEALHNHYTQKSLSLSPG |
| 12. | constant region human HC IgG1m(f)-L351D-E356K-T366K-K439E | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYT**D**PPSR**K**EMTK NQVSL**K**CLVKGFYPSDIAVEWESNGQPENNYKTTPPVLDSDGSFFLYSK LTVDKSRWQQGNVFSCSVMHEALHNHYTQ**E**SLSLSPG |
| 13. | constant region human HC IgG1m(f)-Q347K-L351D-K360E-T366K | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREP**K**VYT**D**PPSREEMT**E**N QVSL**K**CLVKGFYPSDIAVEWESNGQPENNYKTTPPVLDSDGSFFLYSKL TVDKSRWQQGNVFSCSVMHEALHNHYTQKSLSLSPG |
| 14. | constant region human HC IgG1m(f)-S364W-K370A-K392D-D399K | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTKN QV**W**LTCLV**A**GFYPSDIAVEWESNGQPENNY**D**TTPPVL**K**SDGSFFLYSK LTVDKSRWQQGNVFSCSVMHEALHNHYTQKSLSLSPG |
| 15. | constant region human HC IgG1m(f)-E356K-D399V-K409W-K439E | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSR**K**EMTKN QVSLTCLVKGFYPSDIAVEWESNGQPENNYKTTPPVL**V**SDGSFFLYS**W** LTVDKSRWQQGNVFSCSVMHEALHNHYTQ**E**SLSLSPG |

(continued)

| SEQ ID NO | Name | Sequence |
|---|---|---|
| 16. | constant region human HC IgG1m(f)-L351D-T366K-K392D-D399K-K409D | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYT**D**PPSREEMTK NQVSL**K**CLVKGFYPSDIAVEWESNGQPENNY**D**TTPPVL**K**SDGSFFLYS **D**LTVDKSRWQQGNVFSCSVMHEALHNHYTQKSLSLSPG |
| 17. | constant region human HC IgG1m(f)-Q347K-L351D-E357K-K360E-T366K-K370E | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREP**K**VYT**D**PPSRE**K**MT**E** NQVSL**K**CLV**E**GFYPSDIAVEWESNGQPENNYKTTPPVLDSDGSFFLYSK LTVDKSRWQQGNVFSCSVMHEALHNHYTQKSLSLSPG |
| 18. | constant region human HC IgG1m(f)-E356K-K392D-D399V-K409W-K439E | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSR**K**EMTKN QVSLTCLVKGFYPSDIAVEWESNGQPENNY**D**TTPPVL**V**SDGSFFLYS**W** LTVDKSRWQQGNVFSCSVMHEALHNHYTQ**E**SLSLSPG |
| 19. | constant region human HC IgG1m(f)-Q347K-L351E-E357K-K360E-S364K-L368D-K370E | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREP**K**VYT**E**PPSRE**K**MT**E**N QV**K**LTC**DV**EGFYPSDIAVEWESNGQPENNYKTTPPVLDSDGSFFLYSKL TVDKSRWQQGNVFSCSVMHEALHNHYTQKSLSLSPG |

(continued)

| SEQ ID NO | Name | Sequence |
|---|---|---|
| 20. | constant region human HC IgG1m(f)-Q347K-K360E-D399V-K409W | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREP**K**VYTLPPSREEMT**E**N QVSLTCLVKGFYPSDIAVEWESNGQPENNYKTTPPVL**V**SDGSFFLYS**W** LTVDKSRWQQGNVFSCSVMHEALHNHYTQKSLSLSPG |
| 21. | constant region human HC IgG1m(f)-L351D-E357K-T366K-K370E | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYT**D**PPSRE**K**MTK NQVSL**K**CLV**E**GFYPSDIAVEWESNGQPENNYKTTPPVLDSDGSFFLYSK LTVDKSRWQQGNVFSCSVMHEALHNHYTQKSLSLSPG |
| 22. | constant region human HC IgG1m(f)-E356K-T366W-K392D-D399K-Y407A-K409D | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSR**K**EMTKN QVSL**W**CLVKGFYPSDIAVEWESNGQPENNY**D**TTPPVL**K**SDGSFFL**AS**D LTVDKSRWQQGNVFSCSVMHEALHNHYTQKSLSLSPG |
| 23. | constant region human HC IgG1m(f)-E430G | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKL TVDKSRWQQGNVFSCSVMH**G**ALHNHYTQKSLSLSPG |

(continued)

| SEQ ID NO | Name | Sequence |
|---|---|---|
| 24. | constant region human HC IgG2 | ASTKGPSVFPLAPCSRSTSESTAALGCLVKDYFPEPVTVSWNSGALTSG VHTFPAVLQSSGLYSLSSVVTVPSSNFGTQTYTCNVDHKPSNTKVDKTV ERKCCVECPPCPAPPVAGPSVFLFPPKPKDTLMISRTPEVTCVVVDVSH EDPEVQFNWYVDGVEVHNAKTKPREEQFNSTFRVVSVLTVVHQDWL NGKEYKCKVSNKGLPAPIEKTISKTKGQPREPQVYTLPPSREEMTKNQV SLTCLVKGFYPSDIAVEWESNGQPENNYKTTPPMLDSDGSFFLYSKLTV DKSRWQQGNVFSCSVMHEALHNHYTQKSLSLSPG |
| 25. | constant region human HC IgG3 | ASTKGPSVFPLAPCSRSTSGGTAALGCLVKDYFPEPVTVSWNSGALTSG VHTFPAVLQSSGLYSLSSVVTVPSSSLGTQTYTCNVNHKPSNTKVDKRV ELKTPLGDTTHTCPRCPEPKSCDTPPPCPRCPEPKSCDTPPPCPRCPEPK SCDTPPPCPRCPAPELLGGPSVFLFPPKPKDTLMISRTPEVTCVVVDVSH EDPEVQFKWYVDGVEVHNAKTKPREEQYNSTFRVVSVLTVLHQDWL NGKEYKCKVSNKALPAPIEKTISKTKGQPREPQVYTLPPSREEMTKNQV SLTCLVKGFYPSDIAVEWESSGQPENNYNTTPPMLDSDGSFFLYSKLTV DKSRWQQGNIFSCSVMHEALHNRFTQKSLSLSPG |
| 26. | constant region human HC IgG4 | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPSCPAPEFLGGPSVFLFPPKPKDTLMISRTPEVTCVVVDVSQ EDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQV SLTCLVKGFYPSDIAVEWESNGQPENNYKTTPPVLDSDGSFFLYSRLTV DKSRWQEGNVFSCSVMHEALHNHYTQKSLSLSLG |
| 27. | constant region human HC IgG1m(f)-E430G | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTKN QVSLTCLVKGFYPSDIAVEWESNGQPENNYKTTPPVLDSDGSFFLYSKL TVDKSRWQQGNVFSCSVMH**G**ALHNHYTQKSLSLSPG |

(continued)

| SEQ ID NO | Name | Sequence |
|---|---|---|
| 28. | constant region human HC IgG1m(f)-E345K | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPR**K**PQVYTLPPSREEMTKN QVSLTCLVKGFYPSDIAVEWESNGQPENNYKTTPPVLDSDGSFFLYSKL TVDKSRWQQGNVFSCSVMHEALHNHYTQKSLSLSPG |
| 29. | constant region human HC IgG1m(f)-E345R | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPR**R**PQVYTLPPSREEMTK NQVSLTCLVKGFYPSDIAVEWESNGQPENNYKTTPPVLDSDGSFFLYSK LTVDKSRWQQGNVFSCSVMHEALHNHYTQKSLSLSPG |
| 30. | constant region human HC IgG1m(f)-E356K-K392D-D399K-K409D-E430G | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSR**K**EMTKN QVSLTCLVKGFYPSDIAVEWESNGQPENNY**D**TTPPVL**K**SDGSFFLYS**D**L TVDKSRWQQGNVFSCSVMH**G**ALHNHYTQKSLSLSPG |
| 31. | constant region human HC IgG1m(f)-Q347R-K360E-D399V-F405T-K409W-E430G | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREP**R**VYTLPPSREEMT**E**N QVSLTCLVKGFYPSDIAVEWESNGQPENNYKTTPPVL**V**SDGSF**T**LYS**W** LTVDKSRWQQGNVFSCSVMH**G**ALHNHYTQKSLSLSPG |

(continued)

| SEQ ID NO | Name | Sequence |
|---|---|---|
| 32. | constant region human HC IgG1m(f)-E345K-Q347R-K360E-D399V-F405T-K409W | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPR**K**P**R**VYTLPPSREEMT**E**N QVSLTCLVKGFYPSDIAVEWESNGQPENNYKTTPPVL**V**SDGSF**T**LYS**W** LTVDKSRWQQGNVFSCSVMHEALHNHYTQKSLSLSPG |
| 33. | constant region human HC IgG1m(f)-E345K-E356K-K392D-D399K-K409D | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPR**K**PQVYTLPPSR**K**EMTK NQVSLTCLVKGFYPSDIAVEWESNGQPENNY**D**TTPPVL**K**SDGSFFLYS **D**LTVDKSRWQQGNVFSCSVMHEALHNHYTQKSLSLSPG |
| 34. | constant region human HC IgG1m(f)-E345R E357Q-S364K-L368D-K370S | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPR**R**PQVYTLPPSRE**Q**MTK NQV**K**LTC**D**V**S**GFYPSDIAVEWESNGQPENNYKTTPPVLDSDGSFFLYS KLTVDKSRWQQGNVFSCSVMHEALHNHYTQKSLSLSPG |
| 35. | constant region human HC IgG1m(f)-G236R-E345R-K439E | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELL**R**GPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPR**R**PQVYTLPPSREEMTK NQVSLTCLVKGFYPSDIAVEWESNGQPENNYKTTPPVLDSDGSFFLYSK LTVDKSRWQQGNVFSCSVMHEALHNHYTQ**E**SLSLSPG |

(continued)

| SEQ ID NO | Name | Sequence |
|---|---|---|
| 36. | constant region human HC IgG1m(f)-G237A-E345R-S440K | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGAPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPRRPQVYTLPPSREEMTK NQVSLTCLVKGFYPSDIAVEWESNGQPENNYKTTPPVLDSDGSFFLYSK LTVDKSRWQQGNVFSCSVMHEALHNHYTQKKLSLSPG |

Mutations with respect to the WT sequence are in bold and underlined.

**Examples**

**Example 1: Design of novel IgG1 CH3:CH3 interface mutations**

In human IgG1, dimerization of the two heavy-chains in the Fc domain is mainly mediated by the large and tightly packed CH3:CH3 interface, which is comprised of a hydrophobic core in the middle with several charged residue pairs and hydrogen bonds surrounding it (**Figure 1 & Table 1**). Because of the 2-fold rotational symmetry present in the CH3:CH3 interface, each of these residue pairs is represented twice in the interface.

We explored different structure-guided strategies to select potential CH3 interface mutation candidates: (1) swap the charged residue pairs; (2) convert charged residues to hydrophobic residues; (3) hydrophobic residues to paired charged residues (**Figure 2**).

Regarding the design of charged residue pair swaps, all the charged pairs were first clustered into three different clusters: I, K439-E356 and Q437-K360; II, K370-E357 and D399-K409; and III, D399-K392. For cluster I and II, all the paired residues (K439E-E356K, Q437K/R-K360E, E357K-K370E and D399K-K409D) could be swapped without introducing side chain clashes. In cluster III, however, once we switched the D399 and K392 pairs (K392D-D399K), we also combined with K409D (**Figure 3**). In addition, we also explored converting the charged residue pair E357-K370 to an uncharged residue pair with the E357Qand K370S mutation.

The hydrophobic patches were clustered in two groups for converting to charged pairs. Cluster IV consists of L351, T366 and S364. L351 was replaced by Asp and T366 by Lys to create one charged pair option (L351D-T366K), and L351 was replaced with Glu, and S364 with Lys, to create another charged pair option (L351E-S364K) (**Figure 4**). Furthermore, K392 was replaced with Leu and F405 was replaced with Ala (K392L-F405A).

In order to enable the charged residue pairs to hydrophobic residue conversion, it was decided to only mutate the most buried residues to avoid potential development issues. Hence, the main focus was on the E357-K370-S364-K409-D399 interface and the K409V:D399M, K409W:D399V and S364W:K370A mutations were introduced (**Figure 5**). In addition, the residues T366 and T394 were mutated to Trp, Val or Leu, and the residues F405 and T366 were mutated to Ala or Val (T366W-Y407A, T366L-Y407V, T366V-Y407A, T394W-F405A) for fine tuning steric complementarity of the CH3 dimer interface.

**TABLE 1:** List of CH3 interface residues in the first antibody chain (A) and their side chain contacting residues in the second antibody chain (B)*

| Residues in chain A | Contacting residues in Chain B |
|---|---|
| GLN 347 | LYS 360 |
| TYR 349 | SER 354, ASP 356, GLU 357, LYS 360 |
| THR 350 | SER 354, ARG 355 |
| LEU 351 | LEU 351, SER 354, THR 366 |
| SER 354 | TYR 349, THR 350, LEU 351 |

(continued)

| Residues in chain A | Contacting residues in Chain B |
|---|---|
| ARG 355 | THR 350 |
| ASP 356 | TYR 349, LYS 439 |
| GLU 357 | TYR 349, LYS 370 |
| LYS 360 | GLN 347, TYR 349 |
| SER 364 | LEU 368, LYS 370 |
| THR 366 | LEU 351, TYR 407 |
| LEU 368 | SER 364, LYS 409 |
| LYS 370 | GLU 357, SER 364 |
| ASN 390 | SER 400 |
| LYS 392 | ASP 399, SER 400, PHE 405 |
| THR 394 | THR 394, VAL 397, PHE 405, TYR 407 |
| PRO 395 | VAL 397 |
| VAL 397 | THR 394, PRO 395 |
| ASP 399 | LYS 392, LYS 409 |
| SER 400 | ASN 390, LYS 392 |
| PHE 405 | LYS 392, THR 394, LYS 409 |
| TYR 407 | THR 366, THR 394, TYR 407, LYS 409 |
| LYS 409 | LEU 368, ASP 399, PHE 405, TYR 407 |
| LYS 439 | ASP 356 |
| *Due to the 2-fold symmetry present in the CH3-CH3 interface, each pairwise interaction is represented twice in the Fc region. Eu numbering scheme is used for residue position identification. | |

In the end, the charged pair swap mutations were combined together or with either hydrophobic to charged pair mutations or (un)charged pair to hydrophobic mutations to further enhance the selectivity, resulting in 19 candidates that were shortlisted for experimental screening (**Table A**).

**Table A**

| CH3 domain mutations | Short code | Sequence comprising mutations |
|---|---|---|
| K356*-D392-K399-D409 | KDKD | SEQ ID NO: 4 |
| K356-E392-K399-D409 | KEKD | SEQ ID NO: 5 |
| Q357-K364-D368-S370 | QKDS | SEQ ID NO: 6 |
| V350-Y351-L366-L392-W394-A405-V407 | VYLLWAV | SEQ ID NO: 7 |
| R347-E360-V399-T405-W409 | REVTW | SEQ ID NO: 8 |
| R345-R347-D360-V366-M399-A407-V409 | RRDVMAV | SEQ ID NO: 9 |
| R347-D360-V366-M399-A407-V409 | RDVMAV | SEQ ID NO: 10 |
| K351-K399-D409 | KKD | SEQ ID NO: 11 |
| D351-K356-K366-E439 | L351D-KKE | SEQ ID NO: 12 |
| K347-D351-E360-K366 | KDEK | SEQ ID NO: 13 |
| W364-A370-D392-K399 | WADK | SEQ ID NO: 14 |
| K356-V399-W409-E439 | KVWE | SEQ ID NO: 15 |
| D351-K366-D392-K399-D409 | DKDKD | SEQ ID NO: 16 |

(continued)

| CH3 domain mutations | Short code | Sequence comprising mutations |
|---|---|---|
| K347-D351-K357-E360-K366-E370 | KDKEKE | SEQ ID NO: 17 |
| K356-D392-V399-W409-E439 | KDVWE | SEQ ID NO: 18 |
| K347-E351-K357-E360-K364-D368-E370 | KEKEKDE | SEQ ID NO: 19 |
| K347-E360-V399-W409 | KEVW | SEQ ID NO: 20 |
| D351-K357-K366-E370 | DKKE | SEQ ID NO: 21 |
| K356-W366-D392-K399-A407-D409 | KWDKAD | SEQ ID NO: 22 |

*Where reference is made to position 356, depending on the IgG1 allotype, the original (WT) amino acid at this position can be E or D.

**Example 2: DNA expression vectors for the expression of human IgG1 and variants thereof**

The VH and VL coding regions of relevant antibodies (**Table 2**) were synthesized *de novo* (Geneart) in frame with fully codon-optimized coding regions of the heavy chain constant region in the pcDNA3.3 vector (Invitrogen) or the human kappa light or lambda light chain constant region in the pcDNA3.3 vector. Details of the VH specificities cloned in the different expression constructs are presented in **Table 2.** The heavy and light chain constant region amino acid sequences as used are presented in **Table 3.**

**TABLE 2:** Details of the VH specificities cloned in different expression constructs

| Clone | VH gene | Antigen specificity |
|---|---|---|
| II | IGHV3-23 | CD20 |
| VIII | IGHV3-9 | CD20 |

**TABLE 3:** Details of the heavy and light chain constant region sequences

| Name | SEQ ID No. |
|---|---|
| Human IgG1 HC (IgG1*03 CH1-Hinge-CH2-CH3) | 1 |
| Human kappa LC (IGKC*01 CL) | 2 |
| Human lambda LC (IGLC2*01 CL) | 3 |

To generate CH3 interface mutants, (codon-optimized) mutations were introduced in the CH3 domain coding sequence, as listed in **Figure 6** and **Table A**, and synthesized *de novo* (Geneart). For the mutations in the H and L chain constant regions, Eu-numbering conventions are used throughout (Kabat et al., Sequences of Proteins of Immunological Interest, 5th Ed. Public Health Service, National Institutes of Health, Bethesda, MD; 1991).

**Example 3: Expression and antibody production**

Antibodies were produced under serum-free conditions by co-transfecting relevant H and L chain expression vectors in FreeStyle™ 293-F cells (LifeTechnologies), using 293fectin™ (LifeTechnologies), according to the manufacturer's instructions. Alternatively, antibodies were produced under serum-free conditions by co-transfecting relevant H and L chain expression vectors in ExpiCHO™ cells (ThermoFisher) and purified using a KingFisher (ThermoFisher) in combination with magnetic protein A beads (Pierce™ Protein A/G Magnetic Agarose Beads; Thermo Scientific, Cat # 78609), according to the manufacturer's instructions. Alternatively, antibodies were produced under serum-free conditions by co-transfecting relevant H and L chain expression vectors in Expi293F™ cells (LifeTechnologies), using ExpiFectamine™ 293 (LifeTechnologies), according to the manufacturer's instructions. IgG concentration of cell culture samples was quantified using Bio-Layer Interferometry using Protein A biosensors with the Octet QK (FortéBio). Samples were diluted 4-fold and 20-fold in Sample Diluent (FortéBio). The initial binding rate of each sample was measured using a read time of 60 seconds and a shaking speed of 200 rpm. The concentration was inferred by reference to a standard curve. 10 mM glycine pH 1.0 was used as a regeneration solution.

Protein A or protein G purifications were used to purify antibodies or antibody mixtures from cellular materials for use in biochemical experiments or subsequent chromatography experiments. (isolated) antibodies were bulk purified by protein A or protein G affinity chromatography. In short, culture supernatants were loaded on 5 mL MabSelect SuRe columns (Cat # 11003494, Cytiva), washed with PBS and 0.02 M sodium citrate-NaOH, pH5 and eluted with 0.02 M sodium citrate-NaOH, pH 3, or loaded on 5 mL HiTrap Prot G columns (Cat # 17040501, Cytiva), washed with PBS and eluted with 0.1 M Glycine-HCl, pH 2.7, respectively The eluate was loaded on a HiPrep Desalting column (Cat # 17508701, Cytiva) immediately after purification and the antibody was exchanged into 12.6 mM $NaH_2PO_4$, 140 mM NaCl, pH 7.4 buffer (PBS, Cat #SH3A3830.03, B.Braun). Alternatively, eluted fractions were pooled and dialyzed into PBS using 10 kDa molecular-weight cut-off Slide-A-Lyzer carriages (Cat # 87730, ThermoFisher) of the appropriate size. After buffer exchange, samples were sterile filtered over 0.2 μm dead-end filters (Cat # 431219, Corning). Concentration of the purified IgGs was determined by absorbance at 280 nm. Purified proteins were analyzed by CE-SDS, HP-SEC and mass spectrometry. Purified antibodies were stored at 2-8°C.

HP-SEC analysis was performed using a Waters Alliance 2795 separation module (Waters) equipped with a Waters 2487 dual λ absorbance detector (Waters), using a TSK column (G3000SWxl; Tosoh Biosciences, Cat # TOYO08541) and a TSK-gel SWxl guard column (Tosoh Biosciences, Cat #TOYO08543). The samples (1 mg/mL in PBS pH 7.4) were run at 1 mL/min using mobile phase 0.1 M sodium sulfate ($Na_2SO_4$, Sigma-Aldrich, Cat # 71958)/0.1 M sodium phosphate pH 6.8 ($NaH_2PO_4$, Sigma-Aldrich Cat # 17844/$Na_2HPO_4$.$2H_2O$, Sigma-Aldrich Cat # 71633). Results were processed using Empower 3 software and expressed per peak as percentage of total peak height. A summary of the results is shown in **Table 4.**

The addition of CH3 interface mutations did not affect the formation of intact IgG molecules.

**TABLE 4:** Quantification of percentage of intact IgG by HP-SEC

| Antibody | Monomer (%) | Multimer (%) |
|---|---|---|
| IgG1-VIII | 99.4 | 0.4 |
| IgG1-VIII-REVTW | 98.3 | 1.6 |
| IgG1-VIII-KEVW | 99.2 | 0.8 |
| IgG1-VIII-QKDS | 99.2 | 0.7 |
| IgG1-VIII-KDKEKE | 97.4 | 0.8 |
| IgG1-VIII-KEKEKDE | 98.2 | 1.7 |
| IgG1-VIII-L351D-KKE | 96.7 | 1.9 |
| IgG1-VIII-KEKD | 98.9 | 1.1 |
| IgG1-VIII-KDKD | 98.8 | 1.1 |
| IgG1-VIII-DKDKD | 96.5 | 1.1 |
| IgG1-VIII-KWDKAD | 98.9 | 1 |
| IgG1-VIII-WADK | 99.1 | 0.8 |
| IgG1-VIII-KDEK | 97.4 | 0.7 |
| IgG1-VIII-KVWE | 99 | 0.9 |
| IgG1-VIII-DKKE | 98.5 | 0.8 |
| IgG1-VIII-KDVWE | 99.2 | 0.7 |
| IgG1-VIII-VYLLWAV | 98.8 | 1.2 |
| IgG1-VIII-KKD | 98.9 | 1.1 |
| IgG1-VIII-RRDVMAV | 98.7 | 1.2 |

**Example 4: Glyco-analysis of IgG antibody variants containing CH3 interface mutations**

IgG molecules have a single conserved Asn (N)-linked glycosylation site in the CH2 region (at position N297 for IgG1). The core structure of this N-linked glycan is comprised of N-Acetylglucosamine (GlcNAc) and mannose residues. Further extension can take place with galactose, sialic acid, core fucosylation, and bisecting GlcNAc (Vidarsson et al., 2014, Front. Immunology Oct 20;5:520), resulting in heterogeneously glycosylated IgG1 molecules at N297. Glycans play an important role in protein conformation, stability, and biological function (Costa et al., Crit Rev Biotechnol 34(4): 281-99 (2014)). Therefore, glycosylation changes in IgG1 molecules are unwanted as the efficacy and pharmacokinetic properties may be impacted. Furthermore, glycan heterogeneity needs to be analyzed and controlled during manufacturing, and charged glycans may further increase the complexity of charge-based analytical assays such as imaged capillary isoelectric focusing (iCIEF) that are used for release testing or characterization. Here, the effect of the introduction of CH3 interface mutations on N-linked glycosylation of IgG molecules was investigated. To this end, N-linked glycan profiling was performed on variants of the IgG1-VIII antibody (**Table 2**), with either a wild-type IgG1 Fc domain or an Fc domain containing CH3 interface mutations.

N-linked glycan profiling was performed by liquid chromatography-mass spectrometry (LC-MS) using an Orbitrap Eclipse mass spectrometer (ThermoFisher). Identification and relative quantitation of the N-linked glycosylation was performed on the reduced glycosylated antibody heavy chain. Given the known primary amino acid sequence of the heavy chain, the mass identity of the attached N-linked glycans could be identified. Briefly, antibody samples were diluted to 200 μg/mL in Milli-Q to a total volume of 60 μL. Next, 1 μL 1 M Dithiothreitol (DTT; Sigma, Cat # D9163) was added to 60 μL sample and incubated for 15 min at 37°C. Subsequently, the sample was transferred to a glass Qsert vial (Water, Cat # 186001128c) and placed into the LC-MS. 2 μL sample was injected onto the LC column and the antibody was eluted using a mobile phase A (Formic acid 0.1% in Water ULC/MS - CC/SFC [Bio Solve, Cat #0023244101BS]) - mobile phase B (0.1% Formic Acid in Acetonitrile ULC/MS - CC/SFC [Bio Solve, Cat # 0001934101BS]) gradient from 20% (B) to 50% (B) in 4.5 min at 0.12 mL/min flow rate. The obtained raw m/z spectra were deconvoluted with Genedata Expressionist Refiner MS software (Genedata). As a result, the deconvoluted spectra provided reduced glycosylated heavy chain masses. The obtained peak intensities (after spectral deconvolution) were used to calculate the abundances of individual N-linked glycans (e.g., G0F, G1F, G2F, etc.) relative to the total population of oligosaccharides. **Figure 7** shows schematic representations of the detected glycan species.

N-linked glycan analysis revealed that IgG1-VIII antibody variants containing the CH3 interface mutations REVTW, QKDS, KDKEKE, KEKEKDE, L351-DKKE, KVWE, DKKE did not have a relative abundance of agalactosylated N-glycans (G0F) or di-galactosylated N-glycans (G2F) significantly differing from that of their corresponding antibody without CH3 interface mutations (**Table 5**). However, IgG1-VIII antibody variants with the CH3 interface mutations KEVW, KEKD, KDKD, DKDKD, KWDKAD, WADK, KDEK, KDVWE, VYLLWAV and KKD had a lower abundance of agalactosylated N-glycans (G0F) and a higher abundance of di-galactosylated N-glycans (G2F) than their corresponding antibody without CH3 interface mutations (**Table 5**). IgG1-VIII antibody variants containing CH3 interface mutations REVTW, QKDS, KEKD, KDKD, DKDKD, KWDKAD, KDEK KDVWE and VYLLWAV had slightly higher abundance of the N-linked mannose-5 glycan (ManS), whereas a lower abundance was seen for IgG1-VIII containing the CH3 interface mutation KKD.

The core fucose of the conserved N-linked glycan modulates the affinity of the Fc region for FcgRIIIa/b, whereby afucosylated IgG exhibits higher affinity binding, resulting in augmented ADCC in vitro and in vivo [Falconer et al. (2018) ACS Chem Biol 13:2179; Shinkawa et al. (2003) J Biol Chem 278:3466]. The N-linked glycan analysis revealed that the majority of IgG1-VIII antibody variants containing CH3 interface mutations did not have significant changes in their relative abundance of fucosylation (**Figure 8A**), compared to IgG1-VIII wild type. However, IgG1-VIII-KWDAD had a significant decrease in relative abundance of fucosylation (**Figure 8A**; >5% decrease).

IgG-Fc galactosylation has been proposed to promote C1q binding and complement activity, by enhancing the potential of IgG1 hexamerization [van Osch et al. (2021) J Immunoll 207:1545]. The N-linked glycan analysis revealed that the introduction of CH3 interface mutations to IgG1-VIII antibody variants resulted in variable, albeit not significant, increases in relative abundance of galactosylation compared to IgG1-VIII wild-type (**Figure 8B**; significance determined as a 2-fold change in relative abundance).

These data show that introduction of certain CH3 interfaces could modulate the relative abundance of N-linked glycans, whereas other CH3 interface mutations did not contribute to a significant change in relative abundance of N-linked glycans.

**Table 5.** Relative abundance (%) of N-glycans on the heavy chain of IgG1 antibody variants with or without

CH3 interface mutations, as assessed by liquid chromatography-mass spectrometry (LC-MS)

| Antibody | Glycan species* (%) | | | | | | | | | | | | | | | | |
|---|---|---|---|---|---|---|---|---|---|---|---|---|---|---|---|---|---|
| | A1-Gal | G0 | G0+GN | G0F | G0F+GN | G1 | G1+GN | G1F | G1F+GN | G2 | G2+GN | G2F | Man4 | Man5 | Man6 | Man7 | Man8 |
| IgG1-VIII | nd± | 0.2 | 0.5 | 55.2 | 3.9 | 1.1 | nd | 16.5 | 0.4 | nd | nd | 3.1 | nd | 5.5 | 0.9 | 1.2 | 0.5 |
| IgG1-VIII-REVTW | nd | 0.2 | 0.5 | 46.3 | 3.6 | 1.1 | nd | 20.1 | 0.3 | nd | nd | 4.0 | nd | 8.6 | 1.4 | 1.7 | 0.8 |
| IgG1-VIII-KEVW | nd | 0.2 | 0.4 | 41.5 | 2.5 | 1.1 | nd | 26.0 | 0.4 | nd | nd | 6.7 | nd | 5.8 | 0.7 | 1.2 | 0.3 |
| IgG1-VIII-QKDS | nd | 0.2 | 0.4 | 48.3 | 3.4 | 1.0 | nd | 21.0 | 0.4 | nd | nd | 4.3 | nd | 7.1 | 1.1 | 1.4 | 0.6 |
| IgG1-VIII-KDKEKE | nd | 0.2 | 0.4 | 46.2 | 2.4 | 0.9 | nd | 23.8 | 0.4 | nd | nd | 5.8 | nd | 5.0 | 0.6 | 1.0 | 0.2 |
| IgG1-VIII-KEKEKDE | nd | 0.2 | 0.4 | 48.2 | 2.5 | 0.8 | nd | 22.5 | 0.3 | nd | nd | 4.8 | nd | 5.7 | 0.7 | 1.1 | 0.3 |
| IgG1-VIII-L351D-KKE | nd | 0.1 | 0.5 | 47.2 | 2.8 | 0.8 | nd | 22.2 | 0.4 | nd | nd | 5.7 | nd | 5.4 | 0.5 | 1.0 | 0.2 |
| IgG1-VIII-KEKD | nd | 0.5 | 0.6 | 34.4 | 2.0 | 0.9 | nd | 28.5 | 0.5 | nd | nd | 9.9 | nd | 7.4 | 1.0 | 1.4 | 0.5 |
| IgG1-VIII-KDKD | nd | 0.4 | 0.6 | 31.8 | 2.5 | 1.3 | nd | 29.1 | 0.5 | nd | nd | 9.4 | nd | 8.6 | 1.0 | 1.6 | 0.5 |
| IgG1-VIII-DKDKD | nd | 0.3 | 0.5 | 39.6 | 3.1 | 1.1 | nd | 26.9 | 0.4 | nd | nd | 5.4 | nd | 8.5 | 1.3 | 1.7 | 0.5 |
| IgG1-VIII-KWDKAD | nd | 0.3 | 0.6 | 37.1 | 3.2 | 1.2 | nd | 25.6 | 0.5 | nd | nd | 6.3 | nd | 8.6 | 1.5 | 2.2 | 0.8 |
| IgG1-VIII-WADK | nd | 0.2 | 0.4 | 34.3 | 2.5 | 1.1 | nd | 33.2 | 0.5 | nd | nd | 7.2 | nd | 6.7 | 0.9 | 1.3 | 0.4 |
| IgG1-VIII-KDEK | nd | 0.2 | 0.4 | 38.6 | 2.7 | 1.0 | nd | 23.7 | 0.4 | nd | nd | 7.9 | nd | 7.9 | 1.1 | 1.6 | 0.5 |
| IgG1-VIII-KVWE | nd | 0.2 | 0.4 | 46.5 | 3.1 | 1.1 | nd | 22.0 | 0.4 | nd | nd | 4.4 | nd | 6.8 | 1.1 | 1.4 | 0.6 |
| IgG1-VIII-DKKE | nd | 0.2 | 0.4 | 49.2 | 3.1 | 1.1 | nd | 19.6 | 0.4 | nd | nd | 4.1 | nd | 6.6 | 1.0 | 1.3 | 0.5 |
| IgG1-VIII-KDVWE | nd | 0.3 | 0.5 | 41.4 | 2.9 | 1.1 | nd | 28.6 | 0.4 | nd | nd | 4.6 | nd | 7.1 | 0.9 | 1.3 | 0.5 |
| IgG1-VIII-VYLLWAV | nd | 0.3 | 0.5 | 44.7 | 2.9 | 1.2 | nd | 23.1 | 0.4 | nd | nd | 4.7 | nd | 7.5 | 1.1 | 1.5 | 0.5 |
| IgG1-VIII-KKD | nd | 0.2 | 0.2 | 44.8 | 1.5 | 1.1 | nd | 26.7 | 0.4 | nd | nd | 5.9 | nd | 3.4 | 0.9 | 1.0 | 0.4 |

* Schematic representation of the glycan species as shown in Fig 7

± nd: not detected, the relative abundance of the N-glycan is below the threshold level defined based on the highest peak in the spectrum

## Example 5: Immunogenicity assessment of antibody variants containing CH3 interface mutations

Risk of clinical immunogenicity of the constant domains of IgG antibody variants containing CH3 interface mutations were assessed using Abzena's iTope™ and TCED™ *in silico* technologies.

The iTope™ software uses a Machine Learning algorithm to predict overlapping 9-mer peptide binding specificities across 46 major HLA-DR, DP and DQ isotypes. The selected alleles represent the most common HLA class II alleles found worldwide, with no weighing attributed to those found most prevalent in any particular ethnic population. By comparing these predictions to the predictions for MHC class II binding sequences present in the germline human IgG1(m) f reference sequence, it's possible to assess the "potential risk" of MHC class II binding. Individual peptides, sequentially spanning the whole sequence, are given a binding score from 0 to 3 for each allotype, and those scores are added together for all HLA-DR, DP, DQ alleles to provide an overall risk score ('Position Risk Score'). Peptides are considered weak, medium or strong promiscuous MHC class II binders when their 'Position Risk Scores' reach 1-2, 3-5 or 6+, respectively. The 'Total Score' for the test protein is calculated by adding the 'Position Risk Scores' obtained for all individual peptides. The highest 'Position Risk Score' is also provided to inform the presence of strong binders and/or highly promiscuous peptides ('Hotspot Max'). 9-mer peptides fully homologous to sequences from the human proteome are typically excluded from the analysis, since germline sequences are unlikely to have immunogenic potential in healthy individuals due to T-cell tolerance. Promiscuous binding peptides, i.e., with a 'Position Risk Score' >0, are interrogated against the TCED™ database in order to identify any high sequence homology with >10,000 peptides from unrelated proteins and antibodies that stimulated T cell responses in previous *ex vivo* EpiScreen™ studies (T-cell epitopes; Bryson et al., 2010, BioDrugs 24(1), pp.1-8). The database is interrogated by a BLAST search to confirm whether peptides with predicted promiscuous moderate or high affinity are also present in the database.

iTope™ analysis identified potentially promiscuously binding peptides for IgG constant HC regions containing the CH3 interface mutations listed in **Table A.** For the variants for which promiscuously binding peptides were identified, the analysis showed a range of different calculated scores, where the QKDS mutations exhibited the lowest predicted immunogenicity score and RRDVMAV had the highest. However, the predicted scores for all variants were within a range associated with previously tested human therapeutic monoclonal antibodies (Abzena; internal data). In addition, when interrogating these sequences against the TCED™ database, no shared homology to known T-cell epitopes were identified. Therefore, based on this analysis, there is no apparent increased risk of clinical immunogenicity for antibodies harboring CH3 domain mutations promoting mutually exclusive homodimerization interfaces.

**Example 6: Biochemical stability of antibody variants containing CH3 interface mutations**

Protein stability characteristics of variants of IgG1-VIII containing CH3 interface mutations were studied using biochemical assays and analytical methods. Dynamic light scattering (DLS) was performed to determine the particle size of the samples before Static Light Scattering (SLS) and differential scanning fluorimetry (DSF) were applied to compare the temperature-dependent onset of aggregation, and protein unfolding, respectively. Results from these assays for CH3 interface mutants were compared to those of corresponding wild-type (WT) antibodies to determine whether the introduction of the indicated CH3 interface mutations impacted protein stability.

Samples of variants of antibody clone VIII targeting human antigen were normalized at 1 mg/mL in PBS pH 7.4 and centrifuged for 5 min at 15000×g. 9 μL of each antibody variant was analyzed with DLS, SLS and DSF using an Uncle instrument (Unchained Labs) with Uncle Client Software. Samples were applied in triplicate in Uni's (Unchained Labs, Cat # 201-1010). Antibody variants contained the mutations as presented in **Table A.**

DLS analysis was performed to assess the quality of the samples at the initial temperature using 5 acquisitions at an acquisition time of 5 seconds at 25ᵒC. A refractive index of 1.3334 and a viscosity of 1.0219 cP were used for PBS buffer at 20 °C (standard values supplied in the Uncle Client software). The standard values were corrected for the actual temperature at the measurement (~25ᵒ). Z-average diameters were reported.

SLS analysis was performed to assess the propensity of the antibody variants mentioned above to aggregate in solution as a measure of colloidal stability. Thermal scan measurements were performed with a continuously increasing temperature from 25ᵒC to 95ᵒC (0.5 °C increase/min) throughout the experiment. $T_{agg}$ is determined by the Uncle Client software as the temperature at which the particle size starts to increase using the SLS 266 read-out.

Conformational protein stability was assessed using DSF. The Uncle is capable of detecting changes in intrinsic fluorescence caused by exposure of tryptophane, tyrosine and phenylalanine due to protein unfolding. Thermal scan measurements were performed with a continuously increasing temperature from 25ᵒC to 95ᵒC (0.5 °C increase/min) throughout the experiment. $T_m$ values are reported by the Uncle Client software as the infliction points in the Ratio (350/330 nm) versus temperature graphs, using the first derivative with exception of mutant IgG1-VIII-WADK for which the Peak Height versus temperature graph was used as for this mutant no infliction points were observed in the Ratio 350/330 graph.

All results are summarized in **Tables 6.** DLS analysis revealed that the average particle size (radius) at 25 °C for wild-type IgG1-VIII and variants thereof containing the CH3 interface mutations were comparable, while SLS analysis revealed similar $T_{agg}$ for wild-type IgG1-VIII and variants. for wild-type IgG1-VIII and variants. The $T_m$ as determined through DSF for IgG1-VIII-WT and IgG1-VIII CH3 interface mutants REVTW, KEVW, QKDS, WADK, KDEK, KVWE, DKKE, KDVWE and VYLLWAV were highly comparable while the $T_m$ for IgG1-VIII CH3 interface mutants KDKEKE, KEKEKDE, L351D-KKE, KEKD, KDKD, DKDKD, KWDKAD and KKD were 4.6°C (for KWDKAD) to 8.1°C (for L351-KKE) lower, but still within an acceptable range (**Table 6**).

Overall, it was concluded that the tested IgG1 variants containing CH3 interface demonstrated an overall comparable propensity to aggregate and protein stability profile. The acquired data did not reveal any concerns regarding developability for any of the tested wild-type and mutated variants of IgG1-VIII.

**TABLE 6:** Details the propensity to aggregate (as determined through SLS analysis), and protein conformational stability (as determined through DSF analysis) for IgG1-VIII variants containing CH3 interface mutations which promote mutually exclusive homodimerization. $T_{agg}$: Aggregation temperature; DLS: dynamic light scattering, SLS: static light scattering, DSF: Differential Scanning Fluorimetry; $T_m$: melting temperature.

| | DLS | SLS | DSF |
|---|---|---|---|
| **IgG-MEDI** | **Radius (nm)** | **$T_{agg}$ (°C)** | **$T_m$1 (°C)** |
| IgG1-VIII | 10.2 | 62.5 | 66.5 |
| IgG1-VIII-REVTW | 10.2 | 60.9 | 66.7 |
| IgG1-VIII-KEVW | 10.3 | 59.8 | 67.3 |
| IgG1-VIII-QKDS | 10.1 | 60.9 | 66.4 |
| IgG1-VIII-KDKEKE | 10.3 | 60.7 | 60.0 |
| IgG1-VIII-KEKEKDE | 10.2 | 60.5 | 59.7 |
| IgG1-VIII-L351D-KKE | 10.4 | 59.8 | 58.4 |
| IgG1-VIII-KEKD | 10.6 | 59.8 | 60.4 |
| IgG1-VIII-KDKD | 10.4 | 60.5 | 60.2 |
| IgG1-VIII-DKDKD | 10.1 | 61.2 | 58.7 |

(continued)

| IgG-MEDI | DLS | SLS | DSF |
| | Radius (nm) | $T_{agg}$ (°C) | $T_m1$ (°C) |
| --- | --- | --- | --- |
| IgG1-VIII-KWDKAD | 10.3 | 60.9 | 61.9 |
| IgG1-VIII-WADK | 9.9 | 62.3 | 64.8 |
| IgG1-VIII-KDEK | 10.1 | 62.5 | 65.2 |
| IgG1-VIII-KVWE | 10.2 | 61.7 | 65.9 |
| IgG1-VIII-DKKE | 10.1 | 61.6 | 65.4 |
| IgG1-VIII-KDVWE | 10.2 | 61.5 | 65.3 |
| IgG1-VIII-VYLLWAV | 10.1 | 62.3 | 67.0 |
| IgG1-VIII-KKD | 10.1 | 60.2 | 60.9 |

## Example 7: Biochemical stability under temperature stress conditions of antibody variants containing CH3 interface mutations

Protein stability after 1 month of storage at 2-8 °C and 40 °C of antibody variants was assessed using spectrophotometry measurements (A280), High-Performance Size-Exclusion Chromatography (HP-SEC) and Capillary Electrophoresis-Sodium Dodecyl Sulfate (CE-SDS). The stability of IgG1-VIII-WT was compared to variants thereof containing CH3 interface mutations. Samples were formulated in PBS pH 7.4 at a concentration of ~20 mg/mL with the exception of IgG1-VIII-DKDKD that was at a concentration of 13 mg/mL. Antibody variants contained the mutations as presented in **Table A.**

Spectrophotometry analysis was performed on a Stunner (Unchained Labs), according to the manufacturer's instructions. Both the absorbance at 280nm as a surrogate for protein concentration, and absorbance at 350nm as a surrogate for protein aggregation, were measured.

HP-SEC analysis was performed using an Acquity Arc system separation module (Waters) equipped with a Waters 2489 dual λ absorbance detector (Waters), using a TSK column (G3000SWxl; Tosoh Biosciences, Cat # TOYO08541) and a TSK-gel SWxl guard column (Tosoh Biosciences, Cat #TOYO08543). The samples (~5 mg/mL in PBS pH 7.4) were run at 1 mL/min using mobile phase 0.1 M sodium sulfate ($Na_2SO_4$, Sigma-Aldrich, Cat # 71958)/0.1 M sodium phosphate pH 6.8 ($NaH_2PO_4$, Sigma-Aldrich Cat # 17844/$Na_2HPO_4.2H_2O$, Sigma-Aldrich Cat # 71633). Results were processed using Empower 3 software and expressed per peak as percentage of total peak height.

CE-SDS was performed using a LabChip® GXII Touch (Perkin Elmer, Cat # CLS138160) on a HT Protein Express LabChip (Perkin Elmer, Cat # 760499) using the HT Protein Express Reagent kit (Perkin Elmer, Cat # CLS960008). For the analysis under non-reducing conditions, samples were diluted 20 times to ~1 mg/mL in PBS pH 7.4 and samples were prepared by 2 μL diluted sample + 7 μL HT Protein Express Sample Buffer (provided in kit) + 35 μL MilliQ water. For the analysis under reducing conditions, samples were diluted 20 times to ~1 mg/mL in PBS pH 7.4 and samples were prepared by 2 μL diluted sample + 7 μL denaturing solution + 35 μL MilliQ water. Samples were prepared in FrameStar 96-well Skirted PCR plates (Cat # 4TI-0960). Samples were denatured by incubation at 70°C for 3 minutes. The chip was prepared according to manufacturer's instructions and the samples were run with the HT antibody analysis 200 high sensitivity settings. Protein purity (%) was analyzed using the LabChip GXII software V5.3.2115.0.

All results are summarized in Tables 7 (Stunner), 8 (HP-SEC) and 9 (CE-SDS).

Absorbance measurements at 280nm and 350nm of the wild-type IgG1-VIII antibody and variants thereof containing CH3 interface mutations did not reveal any major differences in protein concentration or aggregation for samples stored at 40°C for 1 month, as compared to samples stored at 2-8°C for 1 month **(Table 7).** Gain of concentration for samples stored at 40°C can be explained by buffer evaporation at this temperature during the time of incubation. No impact of the CH3 interface mutations were observed on protein concentration or aggregation, as compared to the WT reference antibodies (**Table 7**).

HP-SEC analysis revealed a significant increase in the percentage of multimers (exceeding 5%) present in the IgG1-VIII-KEKEKDE, L351-KKE, KDKD, DKDKD samples after 1 month of storage at 40°C (**Table 8**). No significant differences in the percentages of multimericity, monomericity or degradation were observed for other variants of IgG1-VIII, as compared to the WT control antibodies.

The percentage of intact protein detected by CE-SDS served as a measure for protein integrity and degradation in response to the storage at 40°C for 1 month. For all antibodies tested, with the exception of IgG1-VIII-WT and KDVWE, the percentage of intact IgG is reduced after 1 month of storage at 40°C, as observed under non-reducing conditions (**Table 9**). Using reducing conditions, no differences were observed between WT antibodies and the mutated variants thereof. The percentages of L chain remained stable for all tested variants upon 1 month of storage at 40°C, with a minor decrease in the percentage of H chain was observed for most tested variants.

In conclusion, WT antibodies and variants thereof containing CH3 interface mutations generally demonstrate a comparable stability profile upon exposure to 40°C for 1 month with the exception of IgG1-VIII-KEKEKDE, L351-KKE, KDKD, DKDKD.

**TABLE 7:** Absorbances measured by spectrometry using a Stunner at 280nm and 350nm of IgG1-VIII variants with WT or mutated CH3 domains after 1 month of storage at either 2-8°C or 40°C.

| | Stunner (A280/A350) | | | | |
|---|---|---|---|---|---|
| | A280nm Concentration (mg/mL) | | | A350 Aggregates | |
| **IgG-MEDI** | 2-8°C reference | 40°C stressed | Difference (stress/reference) | 2-8°C reference | 40°C stressed |
| IgG1-VIII | 24.46 | 25.19 | 103% | -0.08 | -0.08 |
| IgG1-VIII-REVTW | 19.75 | 21.02 | 106% | -0.07 | 0.02 |
| IgG1-VIII-KEVW | 19.84 | 20.67 | 104% | -0.05 | 0.02 |
| IgG1-VIII-QKDS | 25.12 | 25.81 | 103% | 0.02 | 0.03 |
| IgG1-VIII-KDKEKE | 20.57 | 21.10 | 103% | -0.04 | 0.01 |
| IgG1-VIII-KEKEKDE | 20.69 | 20.76 | 100% | 0.00 | 0.05 |
| IgG1-VIII-L351D-KKE | 20.83 | 21.01 | 101% | -0.06 | 0.03 |
| IgG1-VIII-KEKD | 21.32 | 21.59 | 101% | 0.05 | 0.06 |
| IgG1-VIII-KDKD | 20.89 | 22.51 | 108% | -0.10 | -0.01 |
| IgG1-VIII-DKDKD | 13.78 | 15.65 | 114% | 0.04 | 0.09 |
| IgG1-VIII-KWDKAD | 20.22 | 21.88 | 108% | 0.00 | 0.03 |
| IgG1-VIII-WADK | 19.67 | 21.62 | 110% | -0.01 | 0.03 |
| IgG1-VIII-KDEK | 20.60 | 21.41 | 104% | 0.00 | 0.11 |
| IgG1-VIII-KVWE | 19.87 | 20.30 | 102% | -0.04 | -0.02 |
| IgG1-VIII-DKKE | 21.04 | 21.16 | 101% | -0.03 | -0.04 |
| IgG1-VIII-KDVWE | 20.04 | 20.36 | 102% | 0.02 | 0.05 |
| IgG1-VIII-VYLLWAV | 19.89 | 21.38 | 107% | -0.04 | -0.01 |
| IgG1-VIII-KKD | 20.90 | 22.14 | 106% | -0.06 | 0.02 |

**TABLE 8:** Percentages of multimers, monomers and degraded protein as detected through HP-SEC analysis, of IgG1-VIII variants with WT or mutated CH3 domains after 1 month of storage at either 2-8°C or 40°C. HP-SEC: High Performance Size Exclusion Chromatography.

| | HP-SEC | | | | | |
|---|---|---|---|---|---|---|
| | Multimer % | | Monomer % | | Degradation % | |
| **IgG-MEDI** | 2-8°C reference | 40°C stressed | 2-8°C reference | 40°C stressed | 2-8°C reference | 40°C stressed |
| IgG1-VIII | 0.5 | 0.8 | 99.2 | 98.3 | 0.2 | 0.8 |
| IgG1-VIII-REVTW | 1.8 | 2.7 | 98.2 | 96.1 | 0.0 | 1.2 |
| IgG1-VIII-KEVW | 0.9 | 1.3 | 99.1 | 97.9 | 0.0 | 0.8 |
| IgG1-VIII-QKDS | 1.1 | 2.4 | 98.8 | 96.4 | 0.1 | 1.2 |
| IgG1-VIII-KDKEKE | 1.0 | 4.4 | 97.5 | 94.7 | 1.5 | 0.8 |

(continued)

| IgG-MEDI | HP-SEC | | | | | |
| | Multimer % | | Monomer % | | Degradation % | |
| | 2-8°C reference | 40°C stressed | 2-8°C reference | 40°C stressed | 2-8°C reference | 40°C stressed |
|---|---|---|---|---|---|---|
| IgG1-VIII-KEKEKDE | 1.7 | 8.3 | 98.3 | 90.8 | 0.0 | 0.9 |
| IgG1-VIII-L351D-KKE | 2.0 | 19.3 | 96.9 | 79.9 | 1.2 | 0.8 |
| IgG1-VIII-KEKD | 1.3 | 2.7 | 98.6 | 96.4 | 0.1 | 0.9 |
| IgG1-VIII-KDKD | 1.2 | 5.5 | 98.8 | 93.5 | 0.0 | 1.0 |
| IgG1-VIII-DKDKD | 0.9 | 54.0 | 97.2 | 45.0 | 1.9 | 1.0 |
| IgG1-VIII-KWDKAD | 1.0 | 4.6 | 99.0 | 94.5 | 0.0 | 0.9 |
| IgG1-VIII-WADK | 0.9 | 2.0 | 99.0 | 97.1 | 0.0 | 0.9 |
| IgG1-VIII-KDEK | 1.2 | 2.6 | 96.9 | 96.6 | 2.0 | 0.8 |
| IgG1-VIII-KVWE | 1.0 | 1.4 | 99.0 | 97.6 | 0.0 | 1.0 |
| IgG1-VIII-DKKE | 1.0 | 1.9 | 99.0 | 97.2 | 0.1 | 0.9 |
| IgG1-VIII-KDVWE | 0.8 | 1.3 | 99.2 | 97.9 | 0.0 | 0.8 |
| IgG1-VIII-VYLLWAV | 1.2 | 2.0 | 98.8 | 96.8 | 0.0 | 1.2 |
| IgG1-VIII-KKD | 1.1 | 2.8 | 98.8 | 96.2 | 0.0 | 1.0 |

**TABLE 9:** CE-SDS analysis determining the percentages of intact protein under non-reducing conditions, and the percentages of each HC and LC and the sum of HC and LC (% purity), determined under reducing conditions, detected in IgG1-VIII variants with WT or mutated CH3 domains after 1 month of storage at either 2-8°C or 40°C. CE-SDS: Capillary Electrophoresis Sodium Dodecyl Sulfate; HC: Heavy Chain; LC: Light Chain.

| IgG-MEDI | CE-SDS Non-Reducing | | CE-SDS Reducing | | | |
| | Intact IgG | | HC | | LC | |
| | 2-8°C reference | 40°C stressed | 2-8°C reference | 40°C stressed | 2-8°C reference | 40°C stressed |
|---|---|---|---|---|---|---|
| IgG1-VIII | 154 (97%) | 153 (94%) | 62 (68%) | 61 (67%) | 26 (32%) | 25 (32%) |
| IgG1-VIII-REVTW | 154 (98%) | 155 (83%) | 61 (67%) | 60 (63%) | 26 (32%) | 25 (33%) |
| IgG1-VIII-KEVW | 155 (96%) | 154 (84%) | 61 (67%) | 61 (64%) | 26 (33%) | 26 (33%) |
| IgG1-VIII-QKDS | 156 (96%) | 157 (84%) | 63 (67%) | 63 (64%) | 26 (32%) | 25 (33%) |
| IgG1-VIII-KDKEKE | 158 (95%) | 157 (84%) | 62 (67%) | 62 (64%) | 26 (32%) | 26 (33%) |
| IgG1-VIII-KEKEKDE | 159 (98%) | 157 (84%) | 66 (65%) | 66 (61%) | 26 (34%) | 25 (35%) |
| IgG1-VIII-L351D-KKE | 159 (95%) | 157 (84%) | 62 (68%) | 62 (64%) | 26 (32%) | 25 (34%) |
| IgG1-VIII-KEKD | 160 (96%) | 156 (86%) | 62 (68%) | 62 (64%) | 26 (32%) | 25 (33%) |
| IgG1-VIII-KDKD | 160 (96%) | 157 (83%) | 62 (68%) | 62 (64%) | 26 (32%) | 25 (32%) |
| IgG1-VIII-DKDKD | 161 (97%) | 157 (81%) | 63 (64%) | 63 (59%) | 26 (35%) | 26 (37%) |
| IgG1-VIII-KWDKAD | 162 (97%) | 158 (85%) | 62 (66%) | 62 (63%) | 26 (33%) | 26 (35%) |
| IgG1-VIII-WADK | 160 (97%) | 156 (84%) | 61 (67%) | 62 (64%) | 26 (33%) | 26 (33%) |
| IgG1-VIII-KDEK | 159 (96%) | 156 (87%) | 62 (66%) | 62 (63%) | 26 (33%) | 26 (35%) |
| IgG1-VIII-KVWE | 161 (97%) | 157 (83%) | 62 (67%) | 63 (63%) | 26 (33%) | 26 (33%) |
| IgG1-VIII-DKKE | 160 (97%) | 157 (85%) | 63 (67%) | 63 (63%) | 26 (33%) | 26 (34%) |
| IgG1-VIII-KDVWE | 160 (97%) | 157 (93%) | 63 (67%) | 63 (64%) | 26 (32%) | 26 (33%) |

(continued)

| IgG-MEDI | CE-SDS Non-Reducing | | CE-SDS Reducing | | | |
| --- | --- | --- | --- | --- | --- | --- |
| | Intact IgG | | HC | | LC | |
| | 2-8°C reference | 40°C stressed | 2-8°C reference | 40°C stressed | 2-8°C reference | 40°C stressed |
| IgG1-VIII-VYLLWAV | 149 (97%) | 151 (83%) | 60 (67%) | 61 (63%) | 26 (32%) | 26 (33%) |
| IgG1-VIII-KKD | 153 (97%) | 156 (84%) | 63 (68%) | 62 (64%) | 26 (32%) | 26 (33%) |

## Example 8: CDC activity by IgG1-II and IgG1-VIII antibody variants harbouring CH3 interface mutations

The effect of CH3 interface mutations on complement-dependent cytotoxicity (CDC) activity was tested in an in vitro CDC assay, using anti-CD20 IgG1-VIII and anti-CD20 IgG1-II antibody variants with either a wild-type (WT) or an engineered Fc domain. Specifically, the effect of introduction of various CH3 interface mutations on CDC induction in Daudi and Raji cells was measured.

Daudi cells (ATCC, Cat # CCL-213) and Raji cells (ATCC, Cat # CCL-86) in assay medium (RPMI-1640 [Gibco, cat. no. A10491] supplemented with 0.2% bovine serum albumin [BSA; Roche, Cat # 10735086001]) were plated in polystyrene white opaque 96-well plates (10,000 cells/well; Perkin Elmer, Cat # 6007680) and incubated with concentration series of antibodies (final concentration of $9.16 \times 10^{-4}$ to 60.0 $\mu$g/mL in 4-fold dilution steps) in a total volume of 80 $\mu$L for 15 min on a shaker at RT. Next, 20 $\mu$L normal human serum (NHS; final concentration of 20%; Sanquin) was added as a source of complement and incubated at 37°C for 45 min. After incubation, cell viability was assessed using the CellTiter-Glo® Luminescent Cell Viability Assay (Promega, Cat # G7572), according to the manufacturer's instructions. Briefly, kit-provided CellTiter-Glo® reagent was added in a 1:10 ratio (v/v) to the cells and the mixture was incubated for 30 min at RT. Luminescence was then measured by an Envision plate reader (Perkin Elmer) and normalized to the luminescence measured for cells incubated with 1% (v/v) Triton X-100 (representing total lysis; Sigma, Cat # T8787). The normalized data, expressed as percentage, were analyzed using best-fit values of a non-linear dose-response fit using log-transformed concentrations in GraphPad Prism, and the $EC_{50}$ values as well as the area under the dose-response curves (AUC) of three experimental replicates was calculated. AUC values are reported relative to the AUC calculated for non-binding control antibody IgG1-b12 (set to 0%) and to positive control antibodies IgG1-VIII-WT or IgG1-II-E430G (set to 100%). Relative AUC values in combination with $EC_{50}$ values were used to compare CDC activity of CH3 interface mutation-containing antibodies with that of their corresponding antibody without CH3 interface mutations.

For all IgG1-VIII antibody variants tested, CDC induction in Raji cells was consistent with that in Daudi cells (Figure 9A and 9B, respectively). Positive control antibodies IgG1-VIII-E430G efficiently induced CDC, either measured as AUC or $EC_{50}$ (**Figure 9, Table 10).** While IgG1-VIII-WT induced CDC (**Figure 9**), no CDC activity was observed for IgG1-II-WT on either Raji (**Figure 10A**) or Daudi cells (**Figure 10B**). IgG1-II-WT is a type II CD20 mAb, which is ineffective in CDC induction. The majority of IgG1-VIII antibody variants containing CH3 interface mutations had enhanced CDC activity, as evidenced by a higher AUC and/or a lower $EC_{50}$, while introduction of other mutations (i.e. REVTW, KEKEKDE) did not affect this activity (**Figure 9A, Table 10**). Similarly, IgG1-II antibody variants containing certain CH3 interface mutations (i.e. KEKD, KDKD, KWDKAD) had enhanced CDC activity, as evidenced by a higher AUC and/or a lower $EC_{50}$, while introduction of other mutations (i.e. REVTW, QKDS, VYLLWAV, KKD) did not affect this activity (**Figure 10,**

**Table 10**).

In summary, it was observed that only certain CH3 interface mutations were able to enhance the CDC activity of anti-CD20 antibodies.

**TABLE 10:** $EC_{50}$ and lysis at specific IgG concentration for CDC induction in Daudi cells and Raji cells by antibody variants of IgG1-VIII and IgG1-II harboring CH3 interface mutations

| IgG1-VIII antibody variants | | | | |
| --- | --- | --- | --- | --- |
| | Daudi cells | | Raji cells | |
| Antibody | $EC_{50}$ (mean $\mu$g/mL $\pm$ SD) | Lysis at 0.94 $\mu$g/mL IgG (% $\pm$ SD) | $EC_{50}$ (mean $\mu$g/mL $\pm$ SD) | Lysis at 60 $\mu$g/mL IgG (% $\pm$ SD) |
| IgG1-VIII | 1.224 $\pm$ 0.345 | 37.1 $\pm$ 18.7 | 1.722 $\pm$ 0.563 | 26.1 $\pm$ 0.5 |
| IgG1-VIII-REVTW | 1.163 $\pm$ 0.195 | 36.2 $\pm$ 11.2 | 1.803 $\pm$ 0.352 | 24.5 $\pm$ 0.9 |

(continued)

| IgG1-VIII antibody variants | | | | |
|---|---|---|---|---|
| | **Daudi cells** | | **Raji cells** | |
| **Antibody** | **EC$_{50}$ (mean $\mu$g/mL $\pm$ SD)** | **Lysis at 0.94 $\mu$g/mL IgG (% $\pm$ SD)** | **EC$_{50}$ (mean $\mu$g/mL $\pm$ SD)** | **Lysis at 60 $\mu$g/mL IgG (% $\pm$ SD)** |
| IgG1-VIII-KEVW | 0.535 $\pm$ 0.093 | 86 $\pm$ 8 | 0.865 $\pm$ 0.168 | 43.7 $\pm$ 1.2 |
| IgG1-VIII-QKDS | 0.362 $\pm$ 0.074 | 96.1 $\pm$ 2.4 | 0.606 $\pm$ 0.109 | 53.9 $\pm$ 2.2 |
| IgG1-VIII-KDKEKE | 0.77 $\pm$ 0.217 | 67.2 $\pm$ 11.8 | 1.116 $\pm$ 0.226 | 39 $\pm$ 4 |
| IgG1-VIII-KEKEKDE | 1.76 $\pm$ 0.513 | 19.4 $\pm$ 16.8 | 2.165 $\pm$ 0.456 | 23.6 $\pm$ 3.2 |
| IgG1-VIII-L351D-KKE | 0.757 $\pm$ 0.189 | 64.1 $\pm$ 15.2 | 1.35 $\pm$ 0.359 | 36.8 $\pm$ 4.3 |
| IgG1-VIII-KEKD | 0.245 $\pm$ 0.042 | 99.9 $\pm$ 1.9 | 0.575 $\pm$ 0.153 | 61.5 $\pm$ 7 |
| IgG1-VIII-KDKD | 0.238 $\pm$ 0.025 | 97.7 $\pm$ 2.9 | 0.564 $\pm$ 0.184 | 59 $\pm$ 7.7 |
| IgG1-VIII-DKDKD | 0.239 $\pm$ 0.057 | 102.4 $\pm$ 2.6 | 0.627 $\pm$ 0.207 | 57.9 $\pm$ 4.6 |
| IgG1-VIII-KWDKAD | 0.147 $\pm$ 0.047 | 102.3 $\pm$ 0.5 | 0.4 $\pm$ 0.109 | 63.8 $\pm$ 10.2 |
| IgG1-VIII-WADK | 0.498 $\pm$ 0.048 | 86.5 $\pm$ 5 | 0.919 $\pm$ 0.253 | 43.4 $\pm$ 3.9 |
| IgG1-VIII-KDEK | 0.502 $\pm$ 0.09 | 90.4 $\pm$ 5.1 | 0.875 $\pm$ 0.184 | 45.4 $\pm$ 4.6 |
| IgG1-VIII-KVWE | 0.299 $\pm$ 0.055 | 100.2 $\pm$ 1 | 0.626 $\pm$ 0.126 | 55.8 $\pm$ 6 |
| IgG1-VIII-DKKE | 0.411 $\pm$ 0.03 | 90.5 $\pm$ 1.7 | 0.864 $\pm$ 0.251 | 46.9 $\pm$ 7.1 |
| IgG1-VIII-KDVWE | 0.288 $\pm$ 0.035 | 98.2 $\pm$ 1.5 | 0.584 $\pm$ 0.136 | 59.5 $\pm$ 6.5 |
| IgG1-VIII-VYLLWAV | 0.306 $\pm$ 0.071 | 96.8 $\pm$ 1.5 | 0.774 $\pm$ 0.341 | 53.5 $\pm$ 11.6 |
| IgG1-VIII-KKD | 0.459 $\pm$ 0.085 | 88.2 $\pm$ 4.6 | 0.904 $\pm$ 0.322 | 43.9 $\pm$ 7.3 |
| IgG1-VIII-E430G | 0.185 $\pm$ 0.015 | 100 $\pm$ 2.9 | 0.486 $\pm$ 0.175 | 57.8 $\pm$ 8 |
| **IgG1-II antibody variants** | | | | |
| | **Daudi cells** | | **Raji cells** | |
| **Antibody** | **EC$_{50}$ (mean $\mu$g/mL $\pm$ SD)** | **Lysis at 0.94 $\mu$g/mL IgG (% $\pm$ SD)** | **EC$_{50}$ (mean $\mu$g/mL $\pm$ SD)** | **Lysis at 60 $\mu$g/mL IgG (% $\pm$ SD)** |
| IgG1-II-L2C6 | >60* | 2.9 $\pm$ 6.3 | >60* | 2.9 $\pm$ 4 |
| IgG1-II-REVTW | >60* | 3.5 $\pm$ 5.9 | >60* | -0.5 $\pm$ 2.8 |
| IgG1-II-QKDS | >60* | -0.2 $\pm$ 5.2 | >60* | 6.4 $\pm$ 3 |
| IgG1-II-KEKD | 10.004 $\pm$ 1.994 | 63.2 $\pm$ 4.3 | 4.355 $\pm$ 3.244 | 20.1 $\pm$ 0.8 |
| IgG1-II-KDKD | 7.206 $\pm$ 3.965 | 74.3 $\pm$ 5.2 | 2.599 $\pm$ 0.44 | 23 $\pm$ 2.3 |
| IgG1-II-KWDKAD | 3.768 $\pm$ 1.484 | 95.7 $\pm$ 1.8 | 7.158 $\pm$ 3.899** | 45.7 $\pm$ 3.5 |
| IgG1-II-VYLLWAV | >60* | 8.8 $\pm$ 0.2 | >60* | 6.2 $\pm$ 5.7 |
| IgG1-II-KKD | >60* | -3.8 $\pm$ 12.1 | >60* | -1.6 $\pm$ 2.4 |
| IgG1-II-E430G | 2.834 $\pm$ 0.332 | 98.3 + 3 | 4.282 $\pm$ 1.847 | 48.9 + 3.1 |
| * No CDC was induced at the highest tested antibody concentration (i.e. 60 $\mu$g/mL) ** Estimate because no maximum lysis was reached in the dose range tested | | | | |

## Example 9: Fc$\gamma$R activation by IgG1-II and IgG1-VIII antibody variants harboring CH3 interface mutations

The effect of CH3 interface mutations on the capacity of IgG1-VIII antibody to activate Fc gamma receptors (Fc$\gamma$Rs) in vitro was tested in bioluminescent cell-based reporter assays, using target-expressing Raji cells and a Jurkat reporter cell line expressing human, Fc$\gamma$RI, Fc$\gamma$RIIb or the high affinity allotype variant of human Fc$\gamma$RIIa or of human Fc$\gamma$RIIIa.

Activation of FcγR-mediated signaling was quantified in Promega bioluminescent reporter assays using Raji cells (ATCC, Cat # CCL-86) as target cells and Jurkat human T cells engineered to stably express human FcγRI (Promega, Cat # GA133A), FcγRIIb (Promega, Cat # CS1781E01) or the high affinity allotype variant of human FcγRIIa (FcγRIIa-H131; Promega, Cat # G988A) or of human FcγRIIIa (FcγRIIIa-V158; Promega, Cat # G701A) and to express a nuclear factor of activated T cells (NFAT)-response element driving expression of firefly luciferase as effector cells. Raji cells were seeded in white opaque 384-well plates (5.000 cells/well; Perkin Elmer, Cat # 6007680) in assay buffer (RPMI 1640 [Promega, Cat # G7080] supplemented with 4% [v/v] Low IgG Serum [Promega, Cat # G711A]) and preincubated with antibody concentration series (final concentration of $3.9 \times 10^{-9}$ to 100 μg/mL in 20-fold dilution steps) for 15 min at 37°C/5% $CO_2$ in a total volume of 20 μL. Next, effector cells in assay buffer were added to the Raji cell-antibody mixture according to the manufacturer's instructions and the mixture was incubated for 5 h at 37°C/5% $CO_2$ in a total volume of 30 μL. After incubating the plates for 15 minutes at RT, 30 μL of Bio-Glo™ Assay Luciferase Reagent (Promega, Cat # G7941) was added and incubated for 5 minutes at RT. Luciferase production was quantified by luminescence readout on an Envision plate reader (Perkin Elmer). Luminescence signals were normalized by subtraction of background luminescence signal determined from medium-only samples (no Raji cells, no antibody, and no effector cells). The data were analyzed using best-fit values of a non-linear agonist dose-response model using log-transformed concentrations in GraphPad Prism and the area under the dose-response curves (AUC) of three experimental replicates was calculated. AUC values are reported relative to the AUC calculated for non-binding control antibody IgG1-b12 (set to 0%) and to positive control antibodies IgG1-VIII-WT (set to 100%).

While all tested CH3 interface mutation-containing antibody variants of IgG1-VIII mutations showed similar or slightly lower activation of FcγRI (**Figure 11**) as IgG1-VIII-WT, most CH3 interface mutation-containing antibody variants of IgG1-VIII showed FcγRIIa, FcγRIIb and FcγRIIIa activation differing from that of IgG1-II-WT (**Figure 11**).

*Activation of FcγRIIa and FcγRIIb*

Compared with IgG1-VIII-WT, IgG1-VIII antibody variants with the KEVW, QKDS, DKDKD, KWDKAD, WADK, KDEK, DKKE, VYLLWAV and KKD mutations showed a higher activation of FcγRIIa and FcγRIIb (i.e. a higher AUC), and the variant with the KEKEKDE mutations showed a lower activation of FcγRIIa and FcγRIIb (i.e. a lower AUC; **Figure 11**). The IgG1-VIII antibody variants with the KDKEKE, L351D-KKE, KEKD and KDKD mutations showed a similar activation of FcγRIIa and FcγRIIb, compared with IgG1-VIII-WT (**Figure 11**). The IgG1-VIII antibody variant with the REVTW mutations showed only a higher activation of FcγRIIa whereas the IgG1-VIII antibody variant with the KVWE and KDVWE showed only a higher activation of FcγRIIb (**Figure 11**).

*Activation of FcγRIIIa*

Compared with IgG1-VIII-WT, IgG1-VIII antibody variants with the REVTW, KWDKAD, DKKE and VYLLWAV mutations showed a higher activation of FcγRIIIa (i.e. a higher AUC), and the variants with the KDKEKE, KEKEKDE, L351D-KKE, KEKD and KDKD mutations showed a lower activation of FcγRIIIa (i.e. a lower AUC; **Figure 11**). The IgG1-VIII antibody variants with the KEVW, QKDS, DKDKD, WADK, KDEK, KVWE, KDVWE and KKD mutations showed a similar activation of FcγRIIIa, compared with IgG1-VIII-WT (**Figure 11**).

In summary (**Table 11**), FcγRIIa, FcγRIIb and FcγRIIIa activation by IgG1-VIII-WT was affected by introduction of most of the tested CH3 interface mutations.

**TABLE 11:** The effects of introduction of CH3 interface mutations on FcγRIIa, FcγRIIb, and FcγRIIIa activation efficacy presented as the AUC normalized (± standard deviation) to non-binding control antibody IgG1-b12 (0%) and IgG1-VIII-WT (100%).

| Antibody | FcγRIIa | FcγRIIb | FcγRIIIa |
|---|---|---|---|
| IgG1-VIII-WT | 100.0 ± 18 | 100.0 ± 20 | 100.0 ± 3.8 |
| IgG1-VIII-REVTW | 139.9 ± 23.4 | 133.8 ± 23.4 | 132.8 ± 7.8 |
| IgG1-VIII-KEVW | 143.6 ± 15.2 | 162.5 ± 24.8 | 106.1 ± 4.7 |
| IgG1-VIII-QKDS | 133.7 ± 16.8 | 193.0 ± 28.4 | 102.8 ± 3.4 |
| IgG1-VIII-KDKEKE | 84.0 ± 10.6 | 91.7 ± 11.3 | 87.2 ± 2.6 |
| IgG1-VIII-KEKEKDE | 65.2 ± 6.4 | 58.7 ± 6.3 | 80.3 ± 3.5 |
| IgG1-VIII-L351D-KKE | 83.2 ± 8.9 | 90.5 ± 11.7 | 89.3 ± 2.7 |
| IgG1-VIII-KEKD | 82.0 ± 4.8 | 115.0 ± 5.5 | 39.8 ± 1.0 |

(continued)

| Antibody | FcγRIIa | FcγRIIb | FcγRIIIa |
|---|---|---|---|
| IgG1-VIII-KDKD | 98.3 ± 9.4 | 136.0 ± 9.8 | 70.0 ± 1.8 |
| IgG1-VIII-DKDKD | 171.8 ± 11.6 | 228.8 ± 13.8 | 104.7 ± 3.8 |
| IgG1-VIII-KWDKAD | 198.2 ± 11 | 307.0 ± 18.1 | 129.2 ± 3.4 |
| IgG1-VIII-WADK | 152.0 ± 12.6 | 170.3 ± 17 | 102.0 ± 2.7 |
| IgG1-VIII-KDEK | 141.1 ± 6.4 | 158.6 ± 7.6 | 114.0 ± 2.6 |
| IgG1-VIII-KVWE | 116.2 ± 6.1 | 146.2 ± 7.6 | 107.3 ± 8.5 |
| IgG1-VIII-DKKE | 129.8 ± 9.8 | 154.7 ± 9.1 | 129.7 ± 9.1 |
| IgG1-VIII-KDVWE | 104.2 ± 9.2 | 156.5 ± 10.9 | 105.4 ± 7.4 |
| IgG1-VIII-VYLLWAV | 143.7 ± 10.9 | 179.6 ± 15.1 | 129.3 ± 9.2 |
| IgG1-VIII-KKD | 124.7 ± 10.4 | 184.1 ± 17.2 | 91.9 ± 8 |

**Example 10: FcRn binding of IgG1-II and IgG1-VIII antibody variants harbouring CH3 interface mutations**

The neonatal Fc receptor (FcRn) binds to Fc regions of IgG antibodies with high affinity in an acidic (pH < 6.0) environment such as in endosomes, but with low affinity at neutral pH (pH 7.4). This pH-dependent binding of antibodies to FcRn prevents internalized antibodies from entering late endosomes and subsequent lysosomal degradation and causes recycling of the internalized antibody into the circulation by exocytic release from FcRn upon return on the cell surface where the environment is neutral, extending the antibodies' plasma half-life. As such, pH-dependent binding to FcRn is crucial for an IgG antibody to retain the long half-life that is typical for IgG molecules. Here, the effect of CH3 interface mutations on FcRn binding at pH 6.0 and 7.4 was assessed for IgG1-II and IgG1-VIII antibody variants with a wild-type or engineered Fc domains.

FcRn binding was analyzed by surface plasmon resonance (SPR) using a Biacore 8K SPR system (Cytvia). Biacore Series S Sensor Chips CM5 (Cytiva, Cat # 29104988) were covalently coated with anti-histidine (His) antibody using amine coupling and His capture kits (Cytiva, Cat # BR100050 and Cat # 29234602) according to the manufacturer's instructions. Next, 5nM His-tagged human recombinant FcRn protein (SinoBiological, Cat # CT009-H08H-B) in PBS-P+ buffer pH 7.4 (Cytiva, Cat # 28995084) or in PBS-P+ buffer with the pH adjusted to 6.0 (by addition of hydrochloric acid [Sigma-Aldrich, Cat # 30721-M]) was captured onto the surface of the anti-His antibody-coated sensor chips, using a flow rate of 10 $\mu$L/min and a contact time of 60s. After three start-up cycles using pH 6.0 or pH 7.4 PBS-P+ buffer (contact time: 120s, dissociation time: 60s; flow rate: 30 $\mu$l/min), concentration series of antibodies (100 to 6.25nM in two-fold dilution steps) in pH 6.0 or pH 7.4 PBS-P+ buffer were injected in separate cycles to generate binding curves (contact time: 120s; dissociation time: 50s; flow rate: 30 $\mu$L/min). At the end of each cycle, the injected His-tagged human recombinant FcRn protein and antibodies were dissociated from the anti-His antibody-coated surface using 10mM Glycine-HCl pH 1.5 (Cytiva, Cat # BR100354; contact time: 30s; flow rate: 30 $\mu$L/min) to regenerate the surface for the next cycle. The data were corrected for background signal using sensors with captured FcRn that were incubated with pH 6.0 or pH 7.4 PBS-P+ buffer without antibodies (reference sensors). Data was analyzed using the Biacore Insight Evaluation software (Cytiva) to determine the apparent dissociation equilibrium constant ($K_D$), dissociation rate constant ($k_d$), and association rate constant ($k_a$) of the interaction between the antibody and FcRn. Apparent $K_D$, $k_d$, and $k_a$ values of CH3 interface mutation-containing antibodies were compared to those of their corresponding antibody without CH3 interface mutations, and a fold-change of ≥2 was predefined to be considered a difference.

At pH 6.0, the tested CH3 interface mutation-containing variants of IgG1-VIII (**Figure 12A**) and IgG1-II (**Figure 12C**) showed $k_a$, $k_d$, or $K_D$ values that did not differ more than 2-fold from those of their corresponding antibody without CH3 interface mutations. The exception was IgG1-VIII-DKDKD, which showed increased $k_a$ and decreased $k_a$, values, resulting in more than a 2-fold decrease in $K_D$ values (**Figure 12A**, B). At pH 7.4, none of the tested antibodies showed FcRn binding, as evidenced by the absence of any signals detected during SPR measurements for any of the tested antibodies at the highest tested antibody concentration (**Figure 12D, E**).

These findings show that introduction of CH3 interface mutations in antibody variants of IgG1-VIII or IgG1-II did not affect antibody binding to FcRn at pH 6.0 or 7.4.

**Example 11: Target binding of IgG-VIII antibody variants harboring CH3 interface mutations**

The effect of CH3 interface mutations on target binding of IgG1-VIII antibody variants was assessed by flow cytometry, using target-expressing Raji and Daudi cells (Figure 13A and B respectively).

Raji (ATCC, Cat # CCL-86) and Daudi cells (ATCC, Cat # CCL-213) in FACS buffer (PBS [Lonza, Cat # BE17-517Q, diluted to 1×PBS in distilled water] supplemented with 0.1% [w/v] BSA [Roche, Cat # 10735086001] and 0.02% [w/v] sodium azide [bioWORLD, Cat # 41920044-2]) were plated in polystyrene round-bottom 96-well plates (50,000 cells/well; Greiner Bio-One, Cat # 650261) and incubated with concentration series of antibodies (final concentration of 0.009 to 37.5 μg/mL in 4-fold dilution steps) in a total volume of 50 μL for 45 min at 4°C. After incubation, cells were pelleted by centrifugation and washed three times with FACS buffer. Next, cells were incubated with a 50 μL solution of secondary antibody R-Phycoerythrin (PE) AffiniPure F(ab')$_2$ Fragment Goat Anti-Human IgG, Fcγ fragment specific (Jackson ImmunoResearch, Cat # 109-116-098, diluted 1:400) in FACS buffer for 30 minutes at 4°C, protected from light. Cells were then washed three times with FACS buffer, resuspended in 30 μL FACS buffer supplemented with viability marker TO-PRO™-3 Iodide (Invitrogen, Cat # T3605; diluted 1:20,000), and measured on an iQue flow cytometer (Sartorius). Data were analyzed using FlowJo software, and Kd values were determined by non-linear regression analysis of the binding curves using GraphPad Prism, after subtraction of the background signal observed for cells only incubated with secondary antibody. Kd values were used to initially compare target binding of CH3 interface mutation-containing antibodies with that of their corresponding antibody without CH3 interface mutations, and a Kd fold-change of ≥25% was predefined to be considered a difference. For antibodies with a Kd deviating ≥25%, target binding was additionally compared to that of the corresponding antibody without CH3 interface mutations by visual comparison of binding curves.

None of the tested CH3 interface mutation-containing antibody variants of IgG1-VIII or IgG1-II showed Kd values deviating more than 25% from that of their corresponding antibody without CH3 interface mutations (**Figure 13**), except for the IgG1-VIII antibody variant with the KWDKAD mutation which showed a lower Kd than IgG1-VIII-WT (**Figure 13A,B**). Binding curves for mutant, however, shows that the difference of Kd may be due to its lower maximum binding capacity (**Figure 13A,B**).

These findings show that introduction of CH3 interface mutations in IgG1-VIII and IgG1-II antibody variants did not significantly affect target binding on Raji and Daudi cells.

## Example 12: Pharmacokinetic (PK) analysis of IgG1-VIII antibody variants containing CH3 interface mutations

The effect of introducing selected CH3 interface mutations (i.e. KEKD, KDKD, KKD, REVTW, QKDS, RRDVMAV, VYLWAV) on the clearance of anti-CD20 antibody IgG1-VIII harboring no additional Fc mutations in absence of target binding was studied in severe combined immunodeficient (SCID) mice.

The mice used in this study were housed in the AAALAC- and ISO 9001:2000-accredited Central Laboratory Animal Facility (Gemeenschappelijk Dierenlaboratorium [GDL]; Utrecht, The Netherlands) and handled in accordance with good animal practice as defined by FELASA. All experiments were performed in compliance with the Dutch animal protection law (WoD) translated from the directives (2010/63/EU) and approved by the Dutch animal ethics committees (CCD) and by the local Animal Welfare Body. 11-12 weeks old female SCID (C.B-17/IcrHan®Hsd-*Prkdc*$^{scid}$, Envigo) mice (3 mice per group, unless otherwise mentioned) were injected intravenously with a 100 μL antibody solution containing either 10 μg or 100 μg of an IgG1-VIII antibody variant (corresponding to approximately 0.5 and 5 mg antibody per kg bodyweight, respectively), or containing 100 μg of an IgG-Fc domain-fused VHH antibody variant (corresponding to approximately 5 mg antibody per kg bodyweight). Blood samples (40 μL per sample) were obtained via alternating punctures in the submandibular and saphenous vein at 10 min, 4 or 5 h, 1 day, 2 days, 8 days, 14 days, and 21 days after antibody administration. Blood was collected into heparin-containing vials and centrifuged for 10 min at 14,000×g. Undiluted supernatant (plasma) was then transferred to Eppendorf tubes and stored at <-65 °C until determination of antibody concentrations. Prior to determination of plasma antibody concentrations, mouse plasma samples were diluted 100-fold (to a final mouse plasma concentration of 1%) in assay buffer, consisting of 1% MSD Blocker A (Meso Scale Discovery [MSD], Cat # R93AA-1) in PBS-T (PBS [Lonza, Cat # BEBP17-516Q] supplemented with 0.05% Tween 20 [Sigma, Cat # P1379]). Selected plasma samples were further diluted 1:10 or 1:50 in 1% mouse plasma in assay buffer to accommodate for the expected wide range of antibody concentrations present in the samples.

Total human IgG concentrations in plasma samples were determined using a human IgG ECLIA. 96-well MULTI-ARRAY Standard plates (MSD, Cat # L15XA-3) were coated with 2 μg/mL goat anti-human IgG Fcγ fragment specific capture antibody (Jackson ImmunoResearch, Cat # 109-005-098) in PBS for 16-24 h at 2-8 °C. Plates were subsequently washed with PBS-T, blocked with 3% (w/v) MSD Blocker A in PBS-T for 60 min at RT on a shaker, and washed again. Next, the coated plates were incubated with 50 μL diluted mouse plasma samples for 90 min at RT on a shaker, and were subsequently washed with PBS-T. Plates were then incubated with 1 μg/mL SULFO-TAG (ST)-conjugated anti-idiotypic detection antibody (IgG1mm-Sab1.1-ST for detection of IgG1-VIII antibody variants, and IgG2amm-1015-4A01-ST for detection of IgG-Fc domain-fused VHH antibody variants; Genmab) in assay buffer for 90 min at RT on a shaker. After washing with PBS-T, electrochemiluminescence signal was generated by adding tripropylamine (TPA)-containing Read Buffer (MSD GOLD Read Buffer, Cat # R92TG-2) and measured at 620 nm on a MESO Sector S 600 plate reader (MSD). Data were processed using SoftMax® Pro GxP Software (Molecular Devices) and were visualized using GraphPad Prism.

PK parameters were derived by non-compartmental methods for intravenous drug infusion using the PKNCA package in Rstudio. Terminal elimination half-life ($t_{1/2}$) was determined by linear regression of at least three data points on the terminal phase of the log(concentration) versus time plot. The area under the antibody concentration-time curve from time point zero (t=0) to infinity (AUCinf) was calculated by the linear up - log-linear down trapezoidal method. The AUC from day 21 ($t_{last}$) to infinity was calculated using the elimination rate ($\lambda z$) estimated to determine $t_{1/2}$. Statistical analysis of the PK parameters was performed using GraphPad Prism.

The mean plasma PK profile of IgG1-VIII with a wild-type Fc domain (i.e. IgG1-VIII-WT) was typical for that of an IV administered non-target binding human IgG1 antibody in mice, with maximum exposure at the earliest tested time point after injection, followed by a two-phase decline (i.e. an initial phase and a terminal phase; **Figure 14**). Introduction of the KEKD, KDKD, and KKD mutations in this antibody enhanced the antibody's clearance from plasma, as evidenced by a faster decline of the plasma antibody concentration over time (**Figure 14B**) and by a significantly lower $t_{1/2}$ and $AUC_{inf}$ compared with IgG1-VIII-WT (**Figure 15**), at both antibody doses tested. Introduction of the REVTW mutations did not affect the antibody's clearance at the highest dose tested (**Figure 14A**) but increased the antibody's clearance at the lowest dose tested, as best demonstrated by the significantly lower $t_{1/2}$ and $AUC_{inf}$ compared with IgG1-VIII-WT at this dose (**Figure 15B, D**). Introduction of the QKDS, RRDVMAV, and VYLLWAV mutations either decreased the $t_{1/2}$ of the antibody only at the lowest dose tested or did not significantly affect the $t_{1/2}$, but decreased the $AUC_{inf}$ at both doses tested (**Figure 15**), indicating that these mutations affected the antibody's PK profile mostly in the initial rather than the terminal phase, as also indicated by the antibodies' plasma concentration versus time curves (**Figure 14C**).

Together, these data indicate that the effect of introduction of CH3 interface mutations on antibody clearance from plasma may be dependent on the antibody in which the mutations are introduced. All tested CH3 interface mutations (i.e. KEKD, KDKD, KKD, REVTW, QKDS, RRDVMAV, VYLLWAV) enhanced the clearance of IgG1-VIII either at one or both of the tested antibody doses. The clearance of IgG1-VIII was enhanced most prominently and consistently by introduction of the KEKD, KDKD, or KKD mutations, and least prominently by the REVTW mutations, which enhanced the antibody's clearance only at the highest dose tested and only slightly.

**Claims**

1. A binding agent comprising at least CH3 domain of a human IgG and a target-binding region, wherein said CH3 domain comprises at least one of the following amino acid residues:

   a. K356;
   b. R347 or K347;
   c. V350;
   d. K351, Y351, D351 or E351;
   e. Q357 or K357;
   f. E360 or D360;
   g. K364 or W364;
   h. W366, L366, K366 or V366;
   i. D368;
   j. S370, E370 or A370;
   k. D392, L392 or E392;
   l. W394;
   m. K399, V399 or M399;
   n. A405 or T405;
   o. A407 or V407;
   p. D409, W409 or V409; or
   q. E439,

   wherein the numbering is according to the EU index, optionally wherein the binding agent is an antibody and the target-binding region is an antigen-binding region, optionally wherein the CH3 domain is of the IgG1, IgG2, IgG3 or IgG4 subclass.

2. The binding agent according to claim 1, wherein said CH3 domain comprises any two, three or all four of the following residues: K356, X392, K399, D409, wherein X is E or D.

3. The binding agent according to claim 1, wherein said CH3 domain comprises any two, three, four or all five of the following residues: R347, E360, V399, T405, W409.

4. The binding agent according to claim 1, wherein said CH3 domain comprises any two, three or all four of the following residues: D351, K357, K366, E370.

5. The binding agent according to claim 1 or 4, wherein said CH3 domain comprises K347 and/or E360.

6. The binding agent according to claim 1, wherein said CH3 domain comprises K356 and E439.

7. The binding agent according to claim 1 or 6, wherein said CH3 domain comprises

      a. any two, three or all four of the following residues: K356, V399, W409, E439; or
      b. any two, three or all four of the following residues: D351, K356, K366, E439; or
      c. any two, three, four or all five of the following residues: K356, D392, V399, W409, E439.

8. The binding agent according to claim 1, wherein said CH3 domain comprises K399 and D409.

9. The binding agent according to claim 1 or 8, wherein said CH3 domain comprises

      a. any two or all three of the following residues: K351, K399, D409;
      b. any two, three, four or all five of the following residues: D351, K366, D392, K399, D409; or
      c. any two, three, four, five or all six of the following residues: K356, W366, D392, K399, A407, D409.

10. The binding agent according to claim 1, wherein said CH3 domain comprises K347 and E360.

11. The binding agent according to claim 1, wherein said CH3 domain comprises D351 and K366.

12. The binding agent according to claim 1, 10 or 11, wherein said CH3 domain comprises

      a. any two, three or all four of the following residues: K347, E360, V399, W409; or
      b. any two, three or all four of the following residues: K347, D351, E360, K366.

13. The binding agent according to claim 1, wherein said CH3 domain comprises

      a. any two, three or all four of the following residues: Q357, K364, D368, S370; or
      b. any two, three or all four of the following residues: W364, A370, D392, K399; or
      c. any two, three, four, five or all six of the following residues: R347, D360, V366, M399, A407, V409 and optionally R345; or
      d. any two, three, four, five, six or all seven of the following residues: V350, Y351, L366, L392, W394, A405, V407; or
      e. any two, three, four, five, six or all seven of the following residues: K347, E351, K357, E360, K364, D368, E370.

14. The binding agent according to any of the preceding claims, wherein said CH3 domain comprises the following residues:

      a. K356, E392, K399 and D409
      b. K356, D392, K399 and D409;
      c. R347, E360, V399, T405 and W409;
      d. D351, K357, K366 and E370;
      e. K356, V399, W409 and E439;
      f. D351, K356, K366 and E439;
      g. K356, D392, V399, W409 and E439;
      h. K351, K399 and D409;
      i. D351, K366, D392, K399 and D409;
      j. K356, W366, D392, K399, A407 and D409;
      k. K347, E360, V399 and W409;
      l. K347, D351, E360 and K366;
      m. Q357, K364, D368 and S370;
      n. W364, A370, D392 and K399;
      o. R347, D360, V366, M399, A407 and V409, and optionally R345;

p. V350, Y351, L366, L392, W394, A405 and V407; or

q. K347, E351, K357, E360, K364, D368 and E370.

15. The binding agent according to any one of the preceding claims, wherein said CH3 domain is dimeric and consists of two CH3 monomer polypeptides, wherein both CH3 monomer polypeptides contain the specified amino acid residues at the specified positions.

16. The binding agent according to any one of the preceding claims, wherein the binding agent has an altered capacity to activate Fc-mediated effector functions relative to a comparator binding agent which is identical except that it has the wild-type amino acid residue(s) at the specified position(s), optionally wherein the one or more effector functions are selected from: FcgammaRIIb activation, capacity for FcgammaRIIIa-V activation, capacity for FcgammaRIIa-H activation, capacity for FcRn binding/activation, capacity for complement activation, and/or capacity for CDC activation .

17. The binding agent according to any of the preceding claims, wherein the binding agent has an altered capacity to induce the binding to and/or activation of one or more Fc receptors and/or complement components through allosteric modulation of the effector binding site relative to a comparator binding agent which is identical except that it has the wild-type amino acid residue(s) at the specified position(s), optionally wherein the Fc receptors are selected from FcgammaRIIb, FcgammaRIIIa-V and/or FcgammaRIIa-H and/or FcRN

18. A method for altering one or more effector functions or for allosteric modulation of one or more effector binding sites of a parent binding agent comprising a CH3 domain of a human IgG and a target-binding region, which method comprises introducing one or more of the following amino acids into the parent binding agent:

a. R347 or K347;
b. V350;
c. K351, Y351, D351 or E351;
d. K356;
e. Q357 or K357;
f. E360 or D360;
g. K364 or W364;
h. W366, L366, K366 or V366;
i. D368;
j. S370, E370 or A370;
k. D392, L392 or E392;
l. W394;
m. K399, V399 or M399;
n. A405 or T405;
o. A407 or V407;
p. D409, W409 or V409; or
q. E439,

wherein the numbering is according to the EU index and wherein the binding agent has an altered effector function relative to a comparator binding agent which is identical except that it has the wild-type amino acid residue(s) at the specified position(s), optionally comprising one or more of the further features of claims 2 to 17.

A

CH3

CH3'

CH3:CH3 dimer

B

K392

P395

K409

T394

T366

Y407

K360

E357

D399

L351

K370

D356    P352

K439

Intra- CH3:CH3 interface

Figure 1

EP 4 644 413 A1

**Figure 2**

A — Wild type interface

B — Charged pairs swaped interface

C — Hydrophobic to charged converted interface

D — Charged to hydrophobic converted interface

**Figure 3**

**Figure 4**

Cluster IV

**Figure 5**

# Figure 6

```
                                  350        360        370        380        390        400
G1*03        GQPREPQVYTLPPSREEMTKNQVSLTCLVKGFYPSDIAVEWESNGQPENNYKTTPPVLDS
G2*01        GQPREPQVYTLPPSREEMTKNQVSLTCLVKGFYPSDIAVEWESNGQPENNYKTTPPMLDS
G3*01        GQPREPQVYTLPPSREEMTKNQVSLTCLVKGFYPSDIAVEWESSGQPENNYNTTPPMLDS
G4*01        GQPREPQVYTLPPSQEEMTKNQVSLTCLVKGFYPSDIAVEWESNGQPENNYKTTPPVLDS

G1-KEKD      GQPREPQVYTLPPSRKEMTKNQVSLTCLVKGFYPSDIAVEWESNGQPENNYETTPPVLKS
G1-KDKD      GQPREPQVYTLPPSRKEMTKNQVSLTCLVKGFYPSDIAVEWESNGQPENNYDTTPPVLKS
G1-QKDS      GQPREPQVYTLPPSREQMTKNQVKLTCDVSGFYPSDIAVEWESNGQPENNYKTTPPVLDS
G1-VYLLWAV   GQPREPQVYVYPPSREEMTKNQVSLTCLVKGFYPSDIAVEWESNGQPENNYLTWPPVLDS
G1-REVTW     GQPREPRVYTLPPSREEMTENQVSLTCLVKGFYPSDIAVEWESNGQPENNYKTTPPVLVS
G1-KKD       GQPREPQVYTKPPSREEMTKNQVSLTCLVKGFYPSDIAVEWESNGQPENNYKTTPPVLKS
G1-DKKE      GQPREPQVYTDPPSRKEMTKNQVSLKCLVKGFYPSDIAVEWESNGQPENNYKTTPPVLDS
G1-KDEK      GQPREPKVYTDPPSREEMTENQVSLKCLVKGFYPSDIAVEWESNGQPENNYKTTPPVLDS
G1-WADK      GQPREPQVYTLPPSREEMTKNQVWLTCLVAGFYPSDIAVEWESNGQPENNYDTTPPVLKS
G1-KVWE      GQPREPQVYTLPPSRKEMTKNQVSLTCLVKGFYPSDIAVEWESNGQPENNYKTTPPVLVS
G1-DKDKD     GQPREPQVYTDPPSREEMTKNQVSLKCLVKGFYPSDIAVEWESNGQPENNYDTTPPVLKS
G1-KDEKE     GQPREPKVYTDPPSREKMTENQVSLKCLVEGFYPSDIAVEWESNGQPENNYKTTPPVLDS
G1-KDVWE     GQPREPQVYTLPPSRKEMTKNQVSLTCLVKGFYPSDIAVEWESNGQPENNYDTTPPVLVS
G1-KEKEKDE   GQPREPKVYTEPPSREKMTENQVKLDCLVEGFYPSDIAVEWESNGQPENNYKTTPPVLDS
G1-KEVW      GQPREPKVYTLPPSREEMTENQVSLTCLVKGFYPSDIAVEWESNGQPENNYKTTPPVLVS
G1-L351D-KKE GQPREPQVYTDPPSREKMTKNQVSLKCLVEGFYPSDIAVEWESNGQPENNYKTTPPVLDS
G1-KWDKAD    GQPREPQVYTLPPSRKEMTKNQVSLWCLVKGFYPSDIAVEWESNGQPENNYDTTPPVLKS


                                  410        420        430        440
G1*03        DGSFFLYSKLTVDKSRWQQGNVFSCSVMHEALHNHYTQKSLSLSPGK
G2*01        DGSFFLYSKLTVDKSRWQQGNVFSCSVMHEALHNHYTQKSLSLSPGK
G3*01        DGSFFLYSKLTVDKSRWQQGNIFSCSVMHEALHNRFTQKSLSLSPGK
G4*01        DGSFFLYSRLTVDKSRWQEGNVFSCSVMHEALHNHYTQKSLSLSLGK

G1-KEKD      DGSFFLYSDLTVDKSRWQQGNVFSCSVMHEALHNHYTQKSLSLSPGK
G1-KDKD      DGSFFLYSDLTVDKSRWQQGNVFSCSVMHEALHNHYTQKSLSLSPGK
G1-QKDS      DGSFFLYSKLTVDKSRWQQGNVFSCSVMHEALHNHYTQKSLSLSPGK
G1-VYLLWAV   DGSFAVSKLTVDKSRWQQGNVFSCSVMHEALHNHYTQKSLSLSPGK
G1-REVTW     DGSFTLYSWLTVDKSRWQQGNVFSCSVMHEALHNHYTQKSLSLSPGK
G1-KKD       DGSFFLYSDLTVDKSRWQQGNVFSCSVMHEALHNHYTQKSLSLSPGK
G1-DKKE      DGSFFLYSKLTVDKSRWQQGNVFSCSVMHEALHNHYTQESLSLSPGK
G1-KDEK      DGSFFLYSKLTVDKSRWQQGNVFSCSVMHEALHNHYTQKSLSLSPGK
G1-WADK      DGSFFLYSKLTVDKSRWQQGNVFSCSVMHEALHNHYTQKSLSLSPGK
G1-KVWE      DGSFFLYSWLTVDKSRWQQGNVFSCSVMHEALHNHYTQESLSLSPGK
G1-DKDKD     DGSFFLYSDLTVDKSRWQQGNVFSCSVMHEALHNHYTQKSLSLSPGK
G1-KDEKE     DGSFFLYSKLTVDKSRWQQGNVFSCSVMHEALHNHYTQKSLSLSPGK
G1-KDVWE     DGSFFLYSWLTVDKSRWQQGNVFSCSVMHEALHNHYTQESLSLSPGK
G1-KEKEKDE   DGSFFLYSKLTVDKSRWQQGNVFSCSVMHEALHNHYTQKSLSLSPGK
G1-KEVW      DGSFFLYSWLTVDKSRWQQGNVFSCSVMHEALHNHYTQKSLSLSPGK
G1-L351D-KKE DGSFFLYSKLTVDKSRWQQGNVFSCSVMHEALHNHYTQKSLSLSPGK
G1-KWDKAD    DGSFFLASDLTVDKSRWQQGNVFSCSVMHEALHNHYTQKSLSLSPGK
```

**Figure 7**

**Figure 8**

Figure 8 - *continued*

**B**

IgG galactosylation (%)

Figure 9

A   Normalized CDC          EC50

Normalized AUC (%)
(b12=0% - IgG1-VIII=100%)

EC50 (µg/mL)

**Figure 9 - *continued***

**B**   Normalized CDC

EC$_{50}$

Figure 10

**A** Normalized CDC

Normalized AUC (%)
(b12=0% - E430G=100%)

**Figure 10 – *continued***

## B    Normalized CDC

Normalized AUC (%)
(b12=0% - E430G=100%)

**Figure 11**

**Figure 11 -** *continued*

FcyRIIa-H

Normalized AUC (%)
(b12=0% - IgG1-VIII=100%)

**Figure 11 - *continued***

## FcγRIIb

Normalized AUC (%)
(b12=0% - IgG1-VIII=100%)

**Figure 11 - *continued***

## FcyRIIIa-V

Normalized AUC (%)
(b12=0% - IgG1-VIII=100%)

**Figure 12**

Figure 12 – *continued*

B

# FcRn binding
## (Octet - FAB2G sensor)

**Figure 12 - *continued***

**Figure 12 - _continued_**

**D**  FcRn binding (pH7.4) - IgG1-VIII

**E**  FcRn binding (pH7.4) - IgG1-II

Figure 13

**Figure 13 - *continued***

Figure 13 - *continued*

Figure 14

**Figure 14 - *continued***

**C**

5 mg/kg antibody

0.5 mg/kg antibody

Figure 15

**Figure 15 -** *continued*

## C — AUC~inf~ at 5 mg/kg

## D — AUC~inf~ at 0.5 mg/kg

**EUROPEAN SEARCH REPORT**

Application Number

EP 24 17 4150

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/229915 A1 (IGAWA TOMOYUKI [JP] ET AL) 11 August 2016 (2016-08-11) * whole document, especially Example 1; SEQ ID NOs 11, 13 * | 1,15-18 | INV. C07K16/00 C07K16/28 |
| X | US 2015/344570 A1 (IGAWA TOMOYUKI [JP] ET AL) 3 December 2015 (2015-12-03) * whole document, especially paragraphs [0043, 0388, 0390]; SEQ ID NOs 3, 5; Figure 5 * | 1,15-18 | |
| X | US 2015/274807 A1 (XU TING [CN] ET AL) 1 October 2015 (2015-10-01) * whole document, especially Example 2; Table 5 * | 1,16,17 | |
| X | WO 2019/028316 A1 (AMGEN INC [US]) 7 February 2019 (2019-02-07) * whole document, especially paragraph [00192]; SEQ ID NO: 475 * | 1,16,17 | |
| A,D | LIU RENA ET AL: "Fc-Engineering for Modulated Effector Functions-Improving Antibodies for Cancer Treatment", ANTIBODIES, vol. 9, no. 4, 17 December 2020 (2020-12-17), page 64, XP055918764, CH ISSN: 2073-4468, DOI: 10.3390/antib9040064 * the whole document * | 1,15-18 | |

TECHNICAL FIELDS SEARCHED (IPC)

C07K

The present search report has been drawn up for all claims

| Place of search | Date of completion of the search | Examiner |
|---|---|---|
| The Hague | 17 October 2024 | Luyten, Kattie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 24 17 4150

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1, 15-18(all partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

1. claims: 1, 15-18(all partially)

   A binding agent comprising at least CH3 domain of a human IgG and a target-binding region, wherein said CH3 domain comprises the amino acid residue K356, but does not comprise any of the other amino acid residues listed in claim 1, wherein the numbering is according to the EU index.
   ---

2. claims: 1, 15-18(all partially)

   A binding agent comprising at least CH3 domain of a human IgG and a target-binding region, wherein said CH3 domain comprises the amino acid residue K356 and either R347 or K347, wherein the numbering is according to the EU index.
   ---

3. claims: 1, 15-18(all partially)

   A binding agent comprising at least CH3 domain of a human IgG and a target-binding region, wherein said CH3 domain comprises the amino acid residues K356 and V350, wherein the numbering is according to the EU index.
   ---

4. claims: 1, 7, 14-18(all partially)

   A binding agent comprising at least CH3 domain of a human IgG and a target-binding region, wherein said CH3 domain comprises the amino acid residue K356 and any amino acid residue selected from K351, Y351, D351 or E351, wherein the numbering is according to the EU index.
   ---

5-17. claims: 1-18(partially)

   A binding agent comprising at least CH3 domain of a human IgG and a target-binding region, wherein said CH3 domain comprises the amino acid residue K356 and an amino acid residue according to claim 1e to 1q, respectively, wherein the numbering is according to the EU index.
   ---

18. claims: 1, 3, 5, 10, 12-18(all partially)

   A binding agent comprising at least CH3 domain of a human IgG and a target-binding region, wherein said CH3 domain comprises the amino acid residue R347 or K347, wherein the numbering is according to the EU index.
   ---

page 1 of 2

Europäisches
Patentamt

European
Patent Office

Office européen
des brevets

**LACK OF UNITY OF INVENTION
SHEET B**

**Application Number**

**EP 24 17 4150**

The Search Division considers that the present European patent application does not comply with the requirements of unity of invention and relates to several inventions or groups of inventions, namely:

19-33. claims: 1-8(partially)

A binding agent comprising at least CH3 domain of a human IgG and a target-binding region, wherein said CH3 domain comprises an amino acid residue according to claim 1c to 1q, respectively, wherein the numbering is according to the EU index.

---

page 2 of 2

## ANNEX TO THE EUROPEAN SEARCH REPORT
## ON EUROPEAN PATENT APPLICATION NO.

EP 24 17 4150

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2024

| Patent document cited in search report | | Publication date | Patent family member(s) | | Publication date |
|---|---|---|---|---|---|
| US 2016229915 | A1 | 11-08-2016 | AU | 2014325063 A1 | 07-04-2016 |
| | | | BR | 112016006197 A2 | 26-09-2017 |
| | | | CA | 2925256 A1 | 02-04-2015 |
| | | | CN | 105764922 A | 13-07-2016 |
| | | | DK | 3050896 T3 | 19-07-2021 |
| | | | EP | 3050896 A1 | 03-08-2016 |
| | | | ES | 2881306 T3 | 29-11-2021 |
| | | | HK | 1221963 A1 | 16-06-2017 |
| | | | JP | 6534615 B2 | 26-06-2019 |
| | | | JP | 2019167343 A | 03-10-2019 |
| | | | JP | WO2015046467 A1 | 09-03-2017 |
| | | | KR | 20160056940 A | 20-05-2016 |
| | | | PT | 3050896 T | 24-08-2021 |
| | | | RU | 2758952 C1 | 03-11-2021 |
| | | | RU | 2016116314 A | 01-11-2017 |
| | | | SG | 10201803449V A | 30-05-2018 |
| | | | SG | 11201602261V A | 28-04-2016 |
| | | | TW | 201602132 A | 16-01-2016 |
| | | | TW | 201936635 A | 16-09-2019 |
| | | | US | 2016229915 A1 | 11-08-2016 |
| | | | WO | 2015046467 A1 | 02-04-2015 |
| US 2015344570 | A1 | 03-12-2015 | CN | 105102618 A | 25-11-2015 |
| | | | DK | 2940135 T3 | 09-08-2021 |
| | | | EP | 2940135 A1 | 04-11-2015 |
| | | | ES | 2876009 T3 | 11-11-2021 |
| | | | HK | 1217731 A1 | 20-01-2017 |
| | | | JP | 6433297 B2 | 05-12-2018 |
| | | | JP | 6719507 B2 | 08-07-2020 |
| | | | JP | 2018188445 A | 29-11-2018 |
| | | | JP | WO2014104165 A1 | 12-01-2017 |
| | | | KR | 20150097786 A | 26-08-2015 |
| | | | US | 2015344570 A1 | 03-12-2015 |
| | | | WO | 2014104165 A1 | 03-07-2014 |
| US 2015274807 | A1 | 01-10-2015 | CN | 102851338 A | 02-01-2013 |
| | | | CN | 103388013 A | 13-11-2013 |
| | | | DK | 2889313 T3 | 18-02-2019 |
| | | | EP | 2889313 A1 | 01-07-2015 |
| | | | EP | 3444280 A1 | 20-02-2019 |
| | | | ES | 2710936 T3 | 29-04-2019 |
| | | | HR | P20190272 T1 | 05-04-2019 |
| | | | HU | E041454 T2 | 28-05-2019 |
| | | | JP | 6185584 B2 | 23-08-2017 |
| | | | JP | 2015522295 A | 06-08-2015 |
| | | | US | 2015274807 A1 | 01-10-2015 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 24 17 4150

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2024

| Patent document cited in search report | Publication date | Patent family member(s) | | Publication date |
|---|---|---|---|---|
| | | WO 2014015804 A1 | | 30-01-2014 |
| WO 2019028316 A1 | 07-02-2019 | AU 2018311079 | A1 | 13-02-2020 |
| | | AU 2022228122 | A1 | 29-09-2022 |
| | | BR 112020002013 | A2 | 28-07-2020 |
| | | BR 122021015266 | B1 | 24-01-2023 |
| | | CA 3071376 | A1 | 07-02-2019 |
| | | CL 2020000252 | A1 | 21-08-2020 |
| | | CL 2022000300 | A1 | 23-09-2022 |
| | | CN 111164100 | A | 15-05-2020 |
| | | CN 118126157 | A | 04-06-2024 |
| | | CO 2020001113 | A2 | 15-05-2020 |
| | | CR 20200099 | A | 24-07-2020 |
| | | CY 1125429 | T1 | 16-02-2024 |
| | | DK 3661954 | T3 | 19-04-2022 |
| | | EP 3661954 | A1 | 10-06-2020 |
| | | EP 4029877 | A1 | 20-07-2022 |
| | | ES 2910969 | T3 | 17-05-2022 |
| | | ES 2975221 | T3 | 04-07-2024 |
| | | HR P20220404 | T1 | 27-05-2022 |
| | | HU E058233 | T2 | 28-07-2022 |
| | | IL 272137 | A | 31-03-2020 |
| | | IL 312607 | A | 01-07-2024 |
| | | JO P20200020 | B1 | 17-09-2023 |
| | | JP 7079171 | B2 | 01-06-2022 |
| | | JP 7531545 | B2 | 09-08-2024 |
| | | JP 2019033743 | A | 07-03-2019 |
| | | JP 2022116099 | A | 09-08-2022 |
| | | KR 20200035291 | A | 02-04-2020 |
| | | KR 20220092652 | A | 01-07-2022 |
| | | LT 3661954 | T | 11-04-2022 |
| | | MA 56289 | B1 | 29-04-2022 |
| | | MY 195974 | A | 27-02-2023 |
| | | PH 12020500231 | A1 | 11-01-2021 |
| | | PL 3661954 | T3 | 16-05-2022 |
| | | PT 3661954 | T | 14-04-2022 |
| | | RS 63101 | B1 | 29-04-2022 |
| | | SA 520411230 | B1 | 08-11-2022 |
| | | SA 522432917 | B1 | 05-11-2023 |
| | | SG 11202000821S | A | 27-02-2020 |
| | | SI 3661954 | T1 | 31-05-2022 |
| | | SM T202200162 | T1 | 12-05-2022 |
| | | TN 2020000015 | A1 | 04-10-2021 |
| | | TW 201920239 | A | 01-06-2019 |
| | | TW 202246308 | A | 01-12-2022 |
| | | TW 202342501 | A | 01-11-2023 |

For more details about this annex : see Official Journal of the European Patent Office, No. 12/82

EPO FORM P0459

page 2 of 3

## ANNEX TO THE EUROPEAN SEARCH REPORT
## ON EUROPEAN PATENT APPLICATION NO.

EP 24 17 4150

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2024

| Patent document cited in search report | Publication date | Patent family member(s) | | Publication date |
|---|---|---|---|---|
| | | US | 2019046611 A1 | 14-02-2019 |
| | | US | 2023381276 A1 | 30-11-2023 |
| | | UY | 37829 A | 31-01-2019 |
| | | WO | 2019028316 A1 | 07-02-2019 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013004842 A
- WO 2014108198 A
- WO 2016164480 A
- WO 9951642 A
- WO 2006105062 A
- WO 200042072 A
- US 6737056 B
- US 7083784 B
- WO 2007005612 A1

- WO 2013004842 A2
- US 10590206 B2
- WO 2022189667 A1
- WO 2018083126 A1
- WO 2019211472 A
- US 20120020952 A1
- WO 2015101588 A1
- WO 2009080253 A1


**Non-patent literature cited in the description**

- **HA et al.** *Front. Immunol.*, 2016, vol. 7, 394
- **DIEBOLDER**. *Science*, 2014, vol. 343, 1260
- **NATSUME et al.** *Cancer Res*, 2008, vol. 68 (10), 3863-72
- **DALL'ACQUA et al.** *J Immunol*, 2006, vol. 177, 1129-1138
- **IDUSOGIE et al.** *J Immunol*, 2001, vol. 166, 2571-2575
- **MICHAELSEN et al.** *Scand J Immunol*, 2009, vol. 70, 553-564
- **MOORE et al.** *mAbs*, 2010, vol. 2 (2), 181-189
- **CHOTHIA ; LESK**. *J. Mol. Biol.*, 1987, vol. 196, 901-917
- **LEFRANC MP.** *Nucleic Acids Research*, 1999, vol. 27, 209-212
- **EHRENMANN F. ; KAAS Q. ; LEFRANC M.-P.** *Nucleic Acids Res.*, 2010, vol. 38, D301-307
- **EDELMAN et al.** *Proc Natl Acad Sci U SA.*, May 1969, vol. 63 (1), 78-85
- **KABAT et al.** Sequences of proteins of immunological interest.. NIH, 1991
- **BEURSKENS et al.** *J Immunol*, 01 April 2012, vol. 188 (7), 3532-3541
- **VAN BIJ et al.** *Journal of Hepatology*, October 2010, vol. 53 (4), 677-685
- **LIU et al.** *Antibodies*, 17 November 2020, vol. 9, 29, 64, 457-66
- **SCHLOTHAUER et al.** *Protein Eng. Design and Selection*, 2016, vol. 29 (10), 457-66

- **OOSTINDIE et al.** Logic-gated antibody pairs that selectively act on cells co-expressing two antigens.. *Nat Biotechnol*, 2022, vol. 40 (10), 1509-1519
- **DALL'ACQUA, W. F. et al.** Increasing the affinity of a human IgG1 for the neonatal Fc receptor: biological consequences.. *J. Immunol.*, 2002, vol. 169, 5171-5180
- **PINCETIC et al.** *Nat Immunol.*, August 2014, vol. 15 (8), 707-716
- **NEEDLEMAN ; WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **KABAT et al.** Sequences of Proteins of Immunological Interest. Public Health Service, National Institutes of Health, 1991
- **VIDARSSON et al.** *Front. Immunology*, 2014, vol. 5, 520
- **COSTA et al.** *Crit Rev Biotechnol*, 2014, vol. 34 (4), 281-99
- **FALCONER et al.** *ACS Chem Biol*, 2018, vol. 13, 2179
- **SHINKAWA et al.** *J Biol Chem*, 2003, vol. 278, 3466
- **VAN OSCH et al.** *J Immunoll*, 2021, vol. 207, 1545
- **BRYSON et al.** *BioDrugs*, 2010, vol. 24 (1), 1-8